(19) **Europäisches Patentamt / European Patent Office / Office européen des brevets**

(11) **EP 3 845 247 B1**

(12) **EUROPEAN PATENT SPECIFICATION**

(45) Date of publication and mention of the grant of the patent:
**04.06.2025 Bulletin 2025/23**

(21) Application number: **19855564.1**

(22) Date of filing: **30.08.2019**

(51) International Patent Classification (IPC):
*A61K 45/00* (2006.01)    *A61K 38/00* (2006.01)
*A61K 31/195* (2006.01)    *A61K 31/401* (2006.01)
*A61K 31/405* (2006.01)    *A61K 31/4172* (2006.01)
*A61K 31/198* (2006.01)    *A61K 9/08* (2006.01)
*A61P 35/00* (2006.01)    *A61P 1/16* (2006.01)
*A61P 17/00* (2006.01)    *A61K 9/19* (2006.01)
*A61K 47/10* (2017.01)    *A61K 47/12* (2006.01)
*A61K 47/18* (2017.01)

(52) Cooperative Patent Classification (CPC):
(C-Sets available)
A61K 9/0019; A61K 9/0014; A61K 9/0043;
A61K 9/19; A61K 31/198; A61K 31/513;
A61K 31/675; A61K 31/704; A61K 38/063;
A61K 45/06; A61K 47/10; A61K 47/12;
A61K 47/183; A61K 47/186; A61K 47/26;    (Cont.)

(86) International application number:
**PCT/CN2019/103659**

(87) International publication number:
**WO 2020/043185 (05.03.2020 Gazette 2020/10)**

(54) **USE OF AMINO ACID BASED NUTRIENTS AND PHARMACEUTICAL COMPOSITION CONTAINING THE SAME**

ANWENDUNG EINES AMINOSÄUREBASIERTEN NÄHRSTOFFS UND PHARMAZEUTISCHE ZUSAMMENSETZUNG DAMIT

APPLICATION D'UN NUTRIMENT À BASE D'ACIDES AMINÉS ET COMPOSITION PHARMACEUTIQUE LES COMPRENANT

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **31.08.2018  CN 201811009698**

(43) Date of publication of application:
**07.07.2021  Bulletin 2021/27**

(73) Proprietors:
• **Chengdu Kuachangaopu Medical Technology Co., Ltd.**
  **Chengdu, Sichuan 610000 (CN)**
• **Kuachang Inc.**
  **Anaheim, CA 92804 (US)**

(72) Inventors:
• **ZOU, Fanglin**
  **Chengdu, Sichuan 610041 (CN)**
• **ZOU, Lichang**
  **Chengdu, Sichuan 610041 (CN)**
• **WANG, Jianxia**
  **Chengdu, Sichuan 610041 (CN)**
• **WANG, Yixi**
  **Chengdu, Sichuan 610041 (CN)**

(74) Representative: **ABG Intellectual Property Law, S.L.**
**Avenida de Burgos, 16D**
**Edificio Euromor**
**28036 Madrid (ES)**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

(56) References cited:
EP-A2- 1 570 840    CN-A- 101 223 999
US-A- 5 690 967

- SHI XIAO-LE: "Clinical Effects of L-Ornithine-L-Aspartate Combined with Lactulose in the Treatment of Alcoholic Liver Disease Hyperammonemia", CLINICAL MEDICINE, vol. 34, no. 2, 20 February 2014 (2014-02-20), pages 3 - 5, XP055795970, ISSN: 1003-3548
- LI BIN : "The effect of Arginine and Its Product on Digestive Tract Tumor", JOURNAL OF NEW MEDICINE, vol. 39, no. 8, 31 August 2008 (2008-08-31), pages 557 - 559, XP009527072, ISSN: 0253-9802

(52) Cooperative Patent Classification (CPC): (Cont.)
A61K 47/38; A61P 1/16; A61P 5/00; A61P 17/00; A61P 29/00; A61P 35/00

C-Sets
A61K 31/198, A61K 2300/00;
A61K 31/513, A61K 2300/00;
A61K 31/675, A61K 2300/00;
A61K 31/704, A61K 2300/00;
A61K 38/063, A61K 2300/00

# EP 3 845 247 B1

**Description**

**Technical Field**

The disclosure of the present application relates a high-concentration amino acid based nutrient in manufacturing a topical pharmaceutical composition for use in treating a local lesion disease, a topical pharmaceutical composition for use in treating a local lesion disease containing the amino acid based nutrient and a synergist thereof, and a device containing the composition.

**Background**

Supported by a large amount of research work, solid tumors are often used as research models for local lesion diseases, especially refractory local lesion diseases. Solid tumor is a tumor disease with a tumor symptom. A tumor is a characteristic pathological tissue containing tumor cells. Taking a pancreatic cancer tumor as an example, pancreatic cancer cells account for only about 30% of the volume. It can be seen that in addition to tumor cells, there are often a larger number of other components (sometimes called a microenvironment of tumor cells) in a tumor tissue, including various other cells, various intercellular substances, and various tubes.

One of the main problems in development of anti-tumor drugs is specificity. Cytotoxic drugs can target tumor cells through systemic administration based on the positive results of tumor cell suppression experiments. However, the drugs may induce a great risk of systemic toxicity while generating a systemic effect (effect in inhibiting tumor cells inside and outside a tumor), since they cannot sufficiently recognize the target cells from normal cells, and the difference between their effective dose and safety limit is not large enough. In addition, drug molecules need to effectively penetrate a tumor tissue to act on tumor cells therein, and this is not a barrier-free event. For some tumors with poor blood supply (such as pancreatic cancer), a chance of benefiting the patient is even smaller.

Topical administration has an advantage of physically targeting of drugs. Therefore, it was once believed that topical administration of cytotoxic drugs can increase intratumoral concentration of the drugs, thereby improving their efficacy. However, topical administration of cytotoxic drugs has not shown significant improvement in efficacy. Merely increasing the intratumoral concentration does not seem to be able to significantly improve efficiency of the drugs in targeting cancer cells in intratumoral tissues. In addition to resorting to a delayed release form, cytotoxic drugs are still administered systemically in clinical practice. Chemical ablation agents (high-purity ethanol, high-concentration acid and base) are not characterized by cell destruction but tissue destruction. Compared with cytotoxic drugs, they have almost no systemic curative effect of targeting cancer cells, but often show higher local curative effects. However, they are usually strong destructive agents that cannot sufficiently recognize a target tissue from other tissues. This makes an intervention volume (for example, an acid-base dosage of not greater than 0.2 ml/kg) and an intervention site that can practically apply the drugs are very limited (for example, limitation on an organ in which the tumor is located, limitation on an edge ablation of the tumor, and the like). Therefore, chemical ablation agents have gradually faded out of clinic treatment of malignant solid tumors in the last decade. In fact, there are almost no topical drugs with both high local safety and high local efficacy in clinical practice.

US5690967A discloses topical formulations comprising lactic acid and amino acids such as arginine, lysine, histidine in a concentracion such as 7.6% or 7.8%.

Therefore, there is still a need to develop new drugs, especially topical drugs, for treating a local lesion disease such as a solid tumor, to meet various clinical needs that cannot be met in the prior art. In fact, it is also urgently needed for prevention and treatment of other local lesion diseases, especially refractory local lesion diseases.

**Summary**

The object of the present invention is to provide a topical medicine for preventing and treating a local lesion disease, especially a refractory local lesion disease. More specifically, the object of the present invention is to provide a topical drug that physically targets a local lesion but has higher specificity.

According to an aspect of the present invention, it provides a topical pharmaceutical composition for use in treating a breast cancer selected from the group consisting of:

> a) a topical pharmaceutical composition comprising arginine, sodium hydroxide, sodium bicarbonate, and a pharmaceutically acceptable liquid carrier, and wherein, the concentration (w/v) of arginine is 15-25%, the concentration (w/v) of sodium hydroxide is 2-5%, and the concentration (w/v) of sodium bicarbonate is 3-10%;
> b) a topical pharmaceutical composition comprising arginine, sodium carbonate, sodium bicarbonate, and a pharmaceutically acceptable liquid carrier, and wherein the concentration (w/v) of arginine is 15-25%, the concentration (w/v) of sodium carbonate is 3-10%, and the concentration (w/v) of sodium bicarbonate is 3-10%.

According to an aspect of the present invention, it provides a device for treating a local lesion disease, containing the pharmaceutical composition according to the invention.

According to one embodiment, the concentration (w/v) of the arginine in the topical pharmaceutical composition is 15%-25%, and the topical pharmaceutical composition further comprises a synergist capable of generating a synergistic effect with the amino acid based nutrient in treating the local lesion disease, and a pharmaceutically acceptable liquid carrier.

According to one embodiment, the topical pharmaceutical composition further optionally comprises a synergist capable of generating a synergistic effect with the amino acid based nutrient in treating the local lesion disease.

According to another aspect of the present disclosure, it provides a topical pharmaceutical composition selected from the group consisting of:

a) a topical pharmaceutical composition comprising arginine, sodium hydroxide, sodium bicarbonate, and a pharmaceutically acceptable liquid carrier, wherein the concentration (w/v) of arginine is 15- 25%, the concentration (w/v) of sodium hydroxide is 2-5%, and the concentration (w/v) of sodium bicarbonate is 3-10%;

b) a topical pharmaceutical composition comprising arginine, sodium carbonate, sodium bicarbonate, and a pharmaceutically acceptable liquid carrier, wherein the concentration (w/v) of arginine is 15-25%; the concentration (w/v) of sodium carbonate is 3-10%; and the concentration (w/v) of sodium bicarbonate is 3-10%.

The composition containing an amino acid based nutrient and a conventional ineffective compound as the local active synergistic drug thereof according to the present disclosure has the following advantages compared with a drug containing a single corresponding ingredient: it provides a synergistic effect against the local lesion diseases to improve effectiveness, while providing antagonism against non-specific tissue destruction to improve safety.

The embodiments according to the present invention has the following advantages compared with the prior art for the treatment of a local lesion disease: compared with a cytotoxic drug of prior art, it shows almost non-toxic systemic safety and significantly higher efficacy on a local lesion disease; compared with a molecular targeted drug, it shows less stringent screening of indications, and has great potential for rapidly growing tumors, large tumors, and tumors with poor blood donors; compared with a chemical ablation agent of prior art, it shows higher specificity, that is, while showing effectiveness for a local lesion disease, it also shows significantly lower local irritation to a non-lesion tissue, so that it can have a larger interventional adaptation range and a higher application volume. The use and composition of the present invention are also not troubled by the drug resistance problems encountered by prior art cytotoxic drugs and existing molecular targeted drugs. In addition, the use and composition are easy to prepare and low in cost, which is especially helpful to provide safe and effective treatment for general population who cannot afford high expenses.

## Detailed Description of the Invention

The inventors of the present invention found that when reaching a certain concentration threshold (for example, >5%), although the amino acid based nutrient still cannot be used as a systemic active ingredient, it can be used as a local active ingredient to treat a local lesion disease. The inventors of the present invention further found that a composition containing the high-concentration amino acid based nutrient as a local active ingredient and certain preferred substances (synergists) has a synergistic effect in treating a local lesion disease, especially when the concentration of the high-concentration amino acid based nutrient in the topical pharmaceutical composition for treating a local lesion disease has a ratio as defined below. In the context of the present invention, the term "local active ingredient" as used herein refers to an active ingredient in a topical drug that provides a local effect (usually a destructing effect on a local tissue). The term "systemic active ingredient" as used herein refers to an active ingredient of a drug (for example, an intravenous drug, an oral drug) that reaches a target area by transferring through the blood to provide an effect (usually a destructing effect on a local tissue).

In the context of the present invention, the term "local action" or "local activity" as used herein means that a pharmacological action or pharmacological activity is generated in a target area mainly by the drug itself rather than the drug-carrying blood. The term "topical drug (composition)" refers to a therapeutic drug (composition) that generates a drug effect mainly through a local action. The term "target area" as used herein refers to a target site of administration, such as an adjacent area, interface, interior (preferably the internal part) of a local lesion, and the like.

Within the context of the present invention, the pharmaceutical composition is a local synergistic composition. Within the context of the present invention, the pharmaceutical composition is locally synergistic in the same solution.

In the context of the present invention, the term "local synergy" refers to the synergistic effect of drugs through a local effect achieved by locally co-administration of multiple drug ingredients together. The term "synergistic effect" means a pharmaceutical effect for treatment shown in joint use of drug ingredients (such as amino acid based nutrients and their local synergists) that is more favorable than that when the ingredients are used separately, and includes, for example, synergistic efficacy and synergistic safety. The term "synergistic efficacy" refers to a desired efficacy shown in joint use of active ingredients that is higher than that when anyone of the ingredients is used alone, and/or a desired efficacy (such as tumor suppression rate) shown in the joint use that is not shown when anyone of the ingredients is used alone. The term "synergistic safety" refers to desirable safety (such as irritation, damage to a surrounding normal tissue) shown in joint use of active ingredients that is higher than that when anyone of the ingredients is used alone. Even if the joint use effect is not greater than the maximum effect when a single ingredient is used alone, it is effective in drug effect, and the joint use safety is significantly higher than the single-use safety when a single ingredient achieves the maximum drug effect (such as antagonism against a side effect), and then the joint use also generates a synergistic effect. The term "local synergist" (often abbreviated as synergist in this invention application) refers to an ingredient (for example, an organic nutrient other than an amino acid based nutrient, an acidulant, an alkalizer, a conventional ineffective aromatic compound) that can generate a synergistic effect in combination with a specific drug (for example, an amino acid based nutrient) to generate a local action. Generally, systemic co-administration of a local synergist and a specific drug either does not generate a synergistic effect, or the synergistic effect produced is not a synergistic effect generated by a local action.

Therefore, according to one aspect of the present invention, it provides a topical pharmaceutical composition for use in treating breast cancer comprising arginine , wherein the concentration (w/v) of arginine in the topical pharmaceutical composition is 15%-25%, and wherein the topical pharmaceutical composition further comprises a synergist capable of generating a synergistic effect with the amino acid based nutrient in treating the local lesion disease, and a pharmaceutically acceptable liquid carrier.

According to another aspect according to the present disclosure, it provides a topical pharmaceutical composition for use in treating breast cancer, comprising arginine with a concentration >2.5%, a synergist capable of generating a synergistic effect with the amino acid based nutrient at the concentration in treating the local lesion disease, and a pharmaceutically acceptable liquid carrier, wherein the concentration (w/v) of the amino acid based nutrient in the topical pharmaceutical composition is 15%-25%.

According to an embodiment, the concentration (w/v) of arginine in the topical pharmaceutical composition is 15%-25%.

In the context of the present invention, the term "therapeutically effective amount" refers to an amount of a drug used to treat a disease (such as a tumor) and obtain an effective effect (such as reducing or/and alleviating symptoms of the disease).

In the context of the present invention, unless otherwise specified, the term "concentration" refers to weight/volume percentage concentration% (w/v) of a specified ingredient in the topical pharmaceutical composition. The term "local administration concentration" refers to the concentration of a specified component when the drug is administered locally, and may be the concentration of the specified component where the drug contacts a target area (for example, an injection needle hole or an outlet of a perfusion tube).

In the present disclosure, the term "amino acid based nutrient" refers to an amino acid based compound with a nutritional and healthcare effect, and is preferably selected from an amino acid, an amino acid polymer and an amino acid derivative with a nutritional and healthcare effect, more preferably selected from an amino acid based nutritional drug included in official pharmacopoeia or guidelines of China, United States or Europe, and an amino acid based adjuvant with a nutritional and healthcare effect. **In** the context of the present invention, the term "nutritional and healthcare effect" used refers to an in vivo effect produced by one or more of the following biological actions: providing energy, participating in synthesis of biologically active substances (such as protein), participating in partial metabolism, maintaining animal intestinal microecological balance and participation in other physiological adjustments that are beneficial to the health of an organism (such as regulating protein synthesis, regulating immune response).

In the context of the present invention, the amino acid, amino acid polymer, and amino acid derivative as the amino acid based nutrient are preferably an amino acid selected from the group consisting of a protein amino acid and a non-protein amino acid, or an amino acid polymer containing an amino acids selected from the group consisting of a protein amino acid and a non-protein amino acid, a derivative of an amino acid selected from the group consisting of a protein amino acid and a non-protein amino acid, or a derivative of an amino acid polymer containing an amino acid selected from the group consisting of a protein amino acid and a non-protein amino acid.

In the context of the present invention, the term "protein amino acid" refers to main amino acids that constitute a protein; and the term "non-protein amino acid" refers to an amino acid other than the protein amino acids, which can also be used in nutritional healthcare products, traditional diets and functional diets (such as healthcare diets) as nutritional and healthcare functional components.

Specifically, in the present disclosure, the protein amino acid includes an amino acid selected from the group consisting of a non-polar amino acid (such as alanine, valine, leucine, isoleucine, phenylalanine, proline), a polar neutral amino acid (such as tryptophan, tyrosine, serine, cysteine, methionine, asparagine, glutamine, threonine), a basic amino acid (such as lysine, arginine, histidine), an acidic amino acid (such as aspartic acid, glutamic acid). All of the above except glycine are L-type $\alpha$-amino acids. The non-protein amino acid may include the following amino acids: $\beta$-alanine, taurine, $\gamma$-aminobutyric acid (GABA), tea polyphenols (theanine), pumpkin seed amino acid (3-amino-3-carboxypridefine acid), glutamine, citrulline, ornithine, and the like.

The amino acid polymer may be selected from oligopeptides and polypeptides containing the amino acids as described above.

In the present disclosure, the term "oligopeptide" used herein refers to an amino acid polymer containing 2-10 identical or different amino acids connected by peptide bonds; and the term "polypeptide" refers to an amino acid polymer containing 11-100 identical or different amino acids connected by peptide bonds. The amino acids constituting the oligopeptide or polypeptide may all be one or more of the above amino acids, or may additionally include other amino acids. In one embodiment, the oligopeptide may be one or more selected from the group consisting of glycyl-L-tyrosine, glycylalanine, glycylglycine, lysine-glycine dipeptide, N-(2)-L-alanyl-L-glutamine, carnosine (β-alanine histidine copolymer), glutathione, collagen oligopeptides, casein hydrolyzed peptides, soybean oligopeptides, oligoarginine, oligoglycine, oligolysine. In one embodiment, the polypeptide may be one or more selected from the group consisting of polyaspartic acid, polyglutamic acid, and polylysine.

The amino acid derivative may be for example selected from amino acid salts containing the above amino acids. In the context of the present invention, the term "amino acid salt" used herein refers to a salt formed by the above amino acid with an acid or a base, such as a sodium salt, a calcium salt, a potassium salt, an iron salt, a magnesium salt, a zinc salt, a magnesium salt formed with a base, and a salt formed with an acid, such as hydrochloride, hydrobromide, sulfate, hydrogen sulfate, phosphate, hydrogen phosphate, dihydrogen phosphate, acetate, succinate, citrate, tartrate, lactate, mandelate, methane sulfonate, p-toluenesulfonate, amino acid salt, and the like; more specifically, for example, lysine hydrochloride, histidine hydrochloride, glutamic acid hydrochloride, cysteine hydrochloride, arginine hydrochloride, glycine sulfate, iron glycine sulfate, lysine hydrochloride, aspartic hydrochloride, and the like.

In the present disclosure, the amino acid based nutrient includes one or more of the following amino acid compounds with nutritional and healthcare effects: an amino acid, an amino acid salt, an oligopeptide and a polypeptide. In the pharmaceutical composition of the present invention, the amino acid based nutrient may be one or more, for example, two, three, four, or five or more of an amino acid, an amino acid salt, an oligopeptide, and a polypeptide.

In the present disclosure, the amino acid, amino acid salt, oligopeptide and polypeptide as the amino acid based nutrient are preferably an amino acid selected from the group consisting of alanine, valine, leucine, isoleucine, phenylalanine, proline, tryptophan, tyrosine, serine, cysteine, methionine, threonine, lysine, arginine, histidine, aspartic acid, glutamic acid, β-alanine, taurine, γ-aminobutyric acid (GABA), theanine, citrulline and ornithine, or a salt thereof, an oligopeptide and polypeptide containing or consisting of the above amino acids; and more preferably are an amino acid selected from the group consisting of arginine, lysine, glycine, cysteine, alanine, serine, aspartic acid, and glutamic acid, or salts thereof, an oligopeptide and polypeptide containing or consisting of the above amino acids.

In one embodiment, the amino acid based nutrient is arginine with a nutritional and healthcare effect, and the concentration (w/v) of the amino acid or amino acid salt in the topical pharmaceutical composition is 15%-25%.

In one embodiment, the amino acid based nutrient includes an amino acid with a nutritional and healthcare effect.

In one embodiment, the concentration (w/v) of the amino acid with a nutritional and healthcare effect in the topical pharmaceutical composition is 15%-25%.

In one embodiment, the amino acid based nutrient includes an amino acid salt with a nutritional and healthcare effect.

In one embodiment, the concentration (w/v) of the amino acid salt with a nutritional and healthcare effect in the topical pharmaceutical composition is 15%-25%.

In the present disclosure, a synergist that has a synergistic effect with the amino acid based nutrient in treatment of a local lesion disease includes a conventional ineffective compound and an anti-tumor chemical agent.

In the scope of the present disclosure, the term "conventional ineffective compound" as used herein is different from a conventional effective drug (such as an anti-tumor agent), and refers to an agent which may show an effect for specific cells (such as anti-tumor cell effects) in cell experiments, but does not show more effective inhibition than conventional effective drugs in animal experiments through absorption action, and thus has not been approved by a drug regulatory authority (such as the FDA) as a drug for effective treatment of a specific local lesion disease, such as a non-anti-local lesion disease drug, a nutritious agent, an agent for diagnostic, a pharmaceutical excipient, and the like.

In the present disclosure, the conventional ineffective compound is one or more selected from the group consisting of an organic nutrient other than the amino acid based nutrient, an acidulant, an alkalizer, and a conventional ineffective aromatic compound.

In the present disclosure, the organic nutrient other than the amino acid based nutrient includes a carbohydrate nutrient and/or a lipid nutrient.

In the present disclosure, the conventional ineffective aromatic compound is one or more selected from the group consisting of a vital dye, a salicylic acid compound, and a quinine compound.

In the present disclosure, the conventional ineffective compound is one or more selected from the group consisting of a carbohydrate nutrient, a lipid nutrient, a vital dye, an acidulant, an alkalizer, a salicylic acid compound, and a quinine compound.

In the prior art, the carbohydrate nutrient and the lipid nutrient mainly generate nutrition and healthcare effects through absorption. It is generally believed that while nutritional support can improve nutritional status of a cancer patient, it can also promote growth of tumor cells. In the present invention, the inventors unexpectedly made a carbohydrate nutrient and a lipid nutrient useful as a local action synergistic drug of the amino acid based nutrient.

In the context of the present invention, the term "carbohydrate nutrient" as used herein refers to a carbohydrate compound with a nutritional and healthcare effect, preferably selected from a monosaccharide, a sugar polymer and a sugar derivative with nutritional and health effects, more preferably selected from carbohydrate nutritious drugs and carbohydrate excipients with nutritional and health effects included in official pharmacopoeias or guidelines of China, the United States or Europe.

In a specific embodiment, the topical pharmaceutical composition comprises the amino acid based nutrient and the carbohydrate nutrient.

In a specific embodiment, the topical pharmaceutical composition comprises the amino acid based nutrient, the carbohydrate nutrient, and one or more of the group consisting of a lipid nutrient, a vital dye, an acidulant, an alkalizer, a salicylic acid compound, a quinine compound, and an anti-tumor chemical agent.

In the present disclosure, the monosaccharide, sugar polymer, and sugar derivative as the carbohydrate nutrient are preferably a monosaccharide selected from the group consisting of glucose, ribose, deoxyribose, xylose, fructose, galactose, and fucose, a sugar polymer containing the above monosaccharide, or a derivative thereof.

The sugar polymer may be selected from a disaccharide, an oligosaccharide and a polysaccharide containing the monosaccharide as described above. In the context of the present invention, the term "disaccharide" as used herein refers to a polymer containing 2 monosaccharides connected by a glycosidic bond; the term "oligosaccharide" as used herein refers to a polymer containing 3-10 monosaccharides connected by glycosidic bonds; and the term "polysaccharide" as used herein refers to a polymer containing more than 10 monosaccharides connected by glycosidic bonds. The monosaccharides constituting the disaccharide, oligosaccharide or polysaccharide may all be one or more of the above monosaccharides, or may additionally comprise other monosaccharides. In one embodiment, the disaccharide may be one or more selected from lactulose, maltose, sucrose, lactose, and trehalose. In an embodiment, the oligosaccharide may be one or more selected from chitooligosaccharides, xylo-oligosaccharides, fructooligosaccharides, mannose oligosaccharides, malto-oligosaccharides, and isomalto-oligosaccharides. In one embodiment, the polysaccharide may be one or more selected from starch, cellulose, dextran, and glycosaminoglycan.

The sugar derivative may be, for example, the sugar derivatives of the above monosaccharide or sugar polymer, and selected from the group consisting of sugar acid, sugar acid salt, and sugar alcohol. In the context of the present invention, the term "sugar acid" used herein refers to an acid derivative of a monosaccharide or a sugar polymer; the term "sugar acid salt" as used herein refers to a salt derivative of a monosaccharide or a sugar polymers; the term "sugar alcohol" used herein refers to an alcohol derivative of a monosaccharide or a sugar polymer. In one embodiment, the sugar acid may be one or more selected from gluconic acid, mannonic acid, and arabinoic acid. In one embodiment, the sugar acid salt may be one or more selected from the sodium gluconate, sodium mannate, and sodium arabinate. In an embodiment, the sugar alcohol may be one or more selected from mannitol, maltitol, lactitol, and xylitol.

In the pharmaceutical composition of the present invention, the carbohydrate nutrient may be one or more, for example, 2, 3, 4 or 5 or more of a monosaccharide, an oligosaccharide, a polysaccharide, a sugar acid, a sugar acid salt, and a sugar alcohol.

In one embodiment, the carbohydrate nutrient is selected from glucose, a glucose-containing sugar polymer, or a glucose derivative.

In one embodiment, the carbohydrate nutrient is selected from ribose, a ribose-containing sugar polymer, or a ribose derivative.

In one embodiment, the carbohydrate nutrient is selected from xylose, a xylose-containing sugar polymer, or a xylose derivative.

In one embodiment, the carbohydrate nutrient is preferably one or more selected from glucose, fructose, oligochitosan, glucosamine, lactulose, sorbitol, ribose, sorbose, mannose, galactose, sucrose, lactose, trehalose, xylo-oligosaccharide, fructooligosaccharide, mannose-oligosaccharide, xylitol; more preferably one or more selected from glucose, sodium gluconate, oligochitosan, glucosamine, lactulose, ribose, mannose oligosaccharide, xylitol. In one embodiment, the concentration (w/v) of the carbohydrate nutrient in the pharmaceutical composition is greater than 5%, preferably ≥10%, 10-40%, 15-50% or 25-50%.

In the pharmaceutical composition according to the present disclosure, the lipid nutrient includes any pharmaceutically acceptable lipid nutrient, preferably selected from lipid compounds with nutritional and healthcare effects included in the official pharmacopoeias or guidelines of China, the United States or Europe, and more preferably is one or more selected from a fat, a fatty acid, a fat emulsion and an adipoid.

In an embodiment, the topical pharmaceutical composition comprises the amino acid based nutrient and the lipid nutrient.

In an embodiment, the topical pharmaceutical composition comprises the amino acid based nutrient, the lipid nutrient, and one or more of an anti-tumor chemical agent, a carbohydrate nutrient, a vital dye, an acidulant, and an alkalizer.

In one embodiment, the concentration (w/v) of the lipid nutrient in the pharmaceutical composition is ≥4%, preferably 4-25%.

In one embodiment, the lipid nutrient is one or more selected from a vegetable oil, eicosapentaenoic acid (EPA), docosahexaenoic acid (DHA), long-chain fat emulsion, medium-chain fat emulsion, and phospholipids.

In an embodiment, the topical pharmaceutical composition comprises the amino acid based nutrient and the vital dye.

In one embodiment, the topical pharmaceutical composition comprises the amino acid based nutrient, the vital dyes, and one or more of a carbohydrate nutrient, a lipid nutrient, an acidulant, an alkalizer, a salicylic acid compound, a quinine compound, and an anti-tumor chemical agent.

In the pharmaceutical composition according to the present disclosure, the vital dye includes an aromatic compound dye that can color a structure of a tissue, a cell, a subcellular unit and the like after entering the living tissue of an animal, but has no unacceptable harm to the whole body of the animal. The vital dye may be any suitable one known to a person skilled in the art. For example, it may be one or more organic dyes and hydrates or derivatives thereof selected from the group consisting of methylene blue, patent blue, isosulfide blue, Bengal Red, Toluidine Blue, Trypan Blue, Basic Blue, Eosin, Basic Fuchsin, Crystal Violet, Gentian Violet, Neutral Red, Janus Green B, and Safranin.

In the pharmaceutical composition according to the present disclosure, the methylene blue dye may be selected from the following compounds and hydrates and derivatives thereof: methylene blue, patent blue, isosulfide blue, and neomethylene blue. The methylene blue dye is preferably selected from methylene blue and hydrates and derivatives thereof. In the pharmaceutical composition according to the present disclosure, the concentration (w/v) of the methylene blue dye is ≥0.35%, preferably 0.35-2%, more preferably 0.35-1.5% or 0.5-1%.

In one embodiment, in the pharmaceutical composition according to the present disclosure, the concentration (w/v) of the vital dyes other than the methylene blue dye is 1-10%.

In an embodiment, the topical pharmaceutical composition includes the amino acid based nutrient and the acidulant.

In one embodiment, the topical pharmaceutical composition comprises the amino acid based nutrient, the acidulant, and one or more of a carbohydrate nutrient, a lipid nutrient, a vital dye, an alkalizer, a salicylic acid compound, and a quinine compound.

In the context of the present invention, the term "acidulant" refers to an acid that is mainly used as an adjuvant, more specifically for pH adjustment in preparation of a drug. It usually does not introduce special biological activity in addition to providing acidity. In the composition according to the present disclosure, the acidulant includes any pharmaceutically acceptable acidulant, preferably selected from the acidulants approved by an official competent administrative department of China, the United States, or Europe (such as FDA or China Food and Drug Administration), or an acidulant that has been included in the official pharmacopoeias or guidelines of China, the United States or Europe (for example, the Volume IV "Pharmaceutical Excipients" of the 2015 edition of the Chinese Pharmacopoeia, or the fourth edition of the "Manual of Pharmaceutical Excipients" edited by R.C. Luo et al.).

In the present disclosure, the acidulant includes a strong acid and a weak acid. Acids are divided into strong acids and weak acids according to their degree of ionization in an aqueous solution. The degree of ionization of an acid is represented by an ionization constant (Ka). The smaller Ka is, the weaker the acidity is. For example, the ionization constant (Ka) of a weak acid is less than 0.0001. More generally, the degree of ionization is represented by the negative logarithm of the ionization constant (referred to as acidity coefficient, pKa). The greater pKa is, the weaker the acidity is. The term "strong acid" refers to an acid with pKa of less than 1, such as hydrochloric acid, sulfuric acid, nitric acid, perchloric acid, selenic acid, hydrobromic acid, hydroiodic acid, chloric acid, and the like. The term "weak acid" refers to an acid with pKa of greater than 1, such as carbonic acid, boric acid, acetic acid, phosphoric acid, sulfurous acid, pyruvic acid, oxalic acid, tartaric acid, nitrous acid, and the like.

In one embodiment, the acidulant is one or more selected from the strong acid and the weak acid, and in the topical pharmaceutical composition, the concentration (w/v) of the acidulant is > 0.25%, and preferably 0.75-15%.

In one embodiment, the strong acid includes one or more of hydrochloric acid, sulfuric acid, and nitric acid, and is preferably hydrochloric acid.

In one embodiment, the weak acid includes one or more of phosphoric acid, maleic acid, oxalic acid, and tartaric acid.

In one embodiment, the weak acid includes an acidulant selected from a water-soluble saturated aliphatic carboxylic acid or/and a water-soluble hydroxycarboxylic acid. The weak acid acidulant may be a C1-10 aliphatic carboxylic acid optionally substituted by 1-3 hydroxyl groups. The aliphatic carboxylic acid may be a mono-, di- or tri-aliphatic carboxylic acid. The acidulant selected from a water-soluble saturated aliphatic carboxylic acid includes one or more of acetic acid, propionic acid, butyric acid, malonic acid, succinic acid, and is preferably acetic acid. The acidulant selected from a water-soluble hydroxycarboxylic acid includes one or more of glycolic acid, lactic acid (2-hydroxypropionic acid), citric acid (2-hydroxy-1,2,3-propanetricarboxylic acid), and malic acid (2-hydroxysuccinic acid).

In one embodiment, the acidulant includes a strong acid, preferably hydrochloric acid, and the concentration (w/v) of the strong acid in the pharmaceutical composition is $\geq$ 0.25%, 0.35-2%, preferably 0.5-1.5% or 0.75-1.2%, or preferably 0.75-2% or 1-1.5%.

In one embodiment, the acidulant includes a weak acid, and the concentration (w/v) of the weak acid in the pharmaceutical composition is >0.5%, preferably 1-10%, 1%-5%, or 0.8-15%, and preferably 3.5-15% or 5-15%.

In one embodiment, the acidulant includes a weak acid, preferably acetic acid, and the concentration (w/v) of the weak acid in the pharmaceutical composition is 0.8-20%, 1.5-20%, preferably 2-15 %, 3.5-15%, and more preferably 5-12.5% or 5-15%.

According to one embodiment, the acidulant is one or more selected from acetic acid, propionic acid, butyric acid, malonic acid, succinic acid, glycolic acid, lactic acid (2-hydroxypropionic acid), citric acid (2-hydroxy-1,2,3-propanetricarboxylic acid), malic acid (2-hydroxysuccinic acid) and tartaric acid.

According to one embodiment, the acidulant is a combination of a weak acid and a strong acid, and the concentration of the acidulant in the topical pharmaceutical composition may be 1-10% or 1-15%, where the weight ratio of the weak acid to the strong acid may be in the range of 99:1 to 1:99.

In one embodiment, the composition comprises, arginine/carbonic acid, glycine/arginine/acetic acid.

In an embodiment, the topical pharmaceutical composition comprises the amino acid based nutrient and the alkalizer.

In some embodiments, the pharmaceutical composition comprises one alkalizer.

In one embodiment, the pharmaceutical composition includes multiple alkalizers.

In one embodiment, the topical pharmaceutical composition comprises the amino acid based nutrient, the alkalizer, and one or more of the a carbohydrate nutrient, a lipid nutrient, a vital dye, an acidulant, a salicylic acid compound, a quinine compound, and anti-tumor chemotherapeutic agent.

In the context of the present invention, the term "alkalizer" refers to a basic compound that is mainly used as an adjuvant, more specifically for pH adjustment in preparation of a drug. It usually does not introduce special biological activity in addition to providing basicity. In the composition according to the present disclosure, the alkalizer includes any pharmaceutically acceptable alkalizer, preferably selected from the alkalizers approved by an official competent administrative department of China, the United States, or Europe (such as FDA or China Food and Drug Administration), or an alkalizer that has been included in the official pharmacopoeias or guidelines of China, the United States or Europe (for example, the Volume IV "Pharmaceutical Excipients" of the 2015 edition of the Chinese Pharmacopoeia, or the fourth edition of the "Manual of Pharmaceutical Excipients" edited by R.C. Luo et al.).

In the present disclosure, the alkalizer includes a strong base and a weak base. The strong base includes an alkali metal hydroxide and a strong organic base. In the present disclosure, the alkali metal hydroxide includes, sodium hydroxide; and the organic strong base is, for example, choline.

In one embodiment, the composition comprises, for example, arginine/sodium hydroxide.

In one embodiment, the composition comprises, arginine/sodium bicarbonate/sodium hydroxide.

In the present disclosure, the weak base includes a weak acid-strong base basic inorganic salt, an organic weak acid alkali metal salt, and a nitrogen-containing weak base.

In the context of the present invention, the term "polybasic weak acid acidic inorganic salt" refers to a polybasic weak acid inorganic salt capable of ionizing hydrogen ions in water. As for the polybasic weak acid acidic inorganic salt as a chemical ablation agent, preferably its 0.01M aqueous solution is alkaline, and preferably has a pH>8.0 (for example, disodium hydrogen phosphate, sodium hydrogen carbonate, potassium hydrogen carbonate). The term "polybasic weak acid basic inorganic salt" refers to a polybasic weak acid inorganic salt that cannot ionize hydrogen ions in water, and includes a positive salt of a polybasic weak acid-strong base inorganic salt. The term "nitrogen-containing weak base" refers to a weakly basic compound containing nitrogen, where the weakly basic compound is preferably selected from weakly basic compounds with a water solubility (w/w) of $\geq$2%.

In one embodiment, the alkalizer includes a polybasic weak acid basic inorganic salt, preferably sodium carbonate, and the concentration (w/v) of the polybasic weak acid basic inorganic salt in the pharmaceutical composition is 3-10%.

In one embodiment, the polybasic weak acid inorganic salt includes, sodium bicarbonate.

In one embodiment, the polybasic weak acid acidic inorganic salt is preferably sodium bicarbonate and disodium hydrogen phosphate.

In one embodiment, the alkalizer includes a polybasic weak acid acidic inorganic salt, preferably sodium carbonate, and the concentration (w/v) of the polybasic weak acid acidic inorganic salt in the pharmaceutical composition is 3-10%.

In one embodiment, the nitrogen-containing weak base is selected from, for example, ammonia, ammonium chloride, 2-aminoethanol, tromethamine, triethanolamine, trimethylolaminomethane, 2-aminoethanol, tromethamine, triethanolamine, meglumine, and ethyl-D-glucamine.

In one embodiment, the alkalizer includes a nitrogen-containing weak base, and the concentration (w/v) of the nitrogen-containing weak base in the pharmaceutical composition is ≥2%, and preferably 2-35% or 3 -35%.

In one embodiment, the polybasic weak acid basic inorganic salt includes, sodium carbonate.

In one embodiment, the polybasic weak acid basic inorganic salt is sodium carbonate.

In one embodiment, the composition comprises, arginine/sodium carbonate, In one embodiment, the composition comprises, arginine/sodium bicarbonate, arginine/sodium bicarbonate/sodium carbonate,.

In a specific embodiment, the pharmaceutical composition comprises arginine, sodium hydroxide and sodium bicarbonate, and in the topical pharmaceutical composition, the concentration (w/v) of arginine is 15- 25%, the concentration (w/v) of sodium hydroxide is 2-5%, and the concentration (w/v) of sodium bicarbonate is 3-10%.

In a specific embodiment, the pharmaceutical composition comprises arginine, sodium carbonate and sodium bicarbonate, and in the topical pharmaceutical composition, the concentration (w/v) of arginine is 15-25 %; the concentration (w/v) of sodium carbonate is 3-10%; and the concentration (w/v) of sodium bicarbonate is 3-10%.

In one embodiment, the pharmaceutical composition comprises an acidulant and an alkalizer and has pH buffering capability.

In one embodiment, the pharmaceutical composition comprises an acidulant and an alkalizer, and its buffer capacity is $>0.01 mol \cdot L^{-1} \cdot pH^{-1}$.

In one embodiment, the buffer capacity of the pharmaceutical composition is $0.015\text{-}0.45 mol \cdot L^{-1} \cdot pH^{-1}$.

In one embodiment, the buffer capacity of the pharmaceutical composition is preferably $\geq 0.04 mol \cdot L^{-1} \cdot pH^{-1}$, more preferably $\geq 0.05 mol \cdot L^{-1} \cdot pH^{-1}$.

In one embodiment, the buffer capacity of the pharmaceutical composition is $0.04\text{-}0.45 mol \cdot L^{-1} \cdot pH^{-1}$, more preferably $0.05\text{-}0.45 mol \cdot L^{-1} \cdot pH^{-1}$.

In the context of the present invention, the term "buffering capacity" (also referred to as buffer index) as used herein refers to an amount (for example, xmol) of a strong monobasic acid (such as hydrochloric acid) or a strong monobasic base (for example, sodium hydroxide) when the pH of the pharmaceutical composition per unit volume (for example, 1L) changes by 1 unit, in a unit of $mol \cdot L^{-1} \cdot pH^{-1}$.

In one embodiment, the topical pharmaceutical composition comprises the amino acid based nutrient and the salicylic acid compound.

In one embodiment, the topical pharmaceutical composition comprises the amino acid based nutrient, the salicylic acid compound, and one or more of a carbohydrate nutrient, a lipid nutrient, a vital dye, an alkalizer, an acidulant, and a quinine compound.

In the context of the present disclosure, the term "salicylic acid compound" as used herein refers to a salicylic acid and a derivative thereof. The chemical name of salicylic acid is 2-hydroxybenzoic acid. The salicylic acid derivative may be any suitable one known to a person skilled in the art, which may include, for example, a salicylic acid derivative containing or not containing a metal compound. The former may include, for example, sodium salicylate, magnesium salicylate, zinc salicylate, metal element complexes (such as copper aspirin), and the like, while the latter may include, for example, acetylsalicylic acid (Aspirin), Aspisol, difluorobenzenesalicylic acid, aminosalicylic acid, p-aminosalicylic acid, N-phenylanthranilic acid, salicylanilide, o-ethoxybenzamide, phenyl salicylate, methyl salicylate, methyl p-hydroxybenzoate, ethyl p-hydroxybenzoate, di-salicylate, dicoumarin, and their pharmaceutically acceptable derivatives.

In one embodiment, the salicylic acid compound is one or more selected from the group consisting of a salicylic acid, and acetylsalicylic acid, difluorobenzene salicylic acid, di-salicylate, dicoumarin, and Aspisol, and derivatives thereof.

In one embodiment, the salicylic acid compound is one or more selected from the group consisting of salicylic acid, acetylsalicylic acid, and Aspisol, and in the topical pharmaceutical composition, the concentration (w/v) of the salicylic acid compound is greater than 1%, and preferably 3-10%.

In one embodiment, the topical pharmaceutical composition comprises the amino acid based nutrient and the quinine compound.

In one embodiment, the topical pharmaceutical composition comprises the amino acid based nutrient, the quinine compound, and one or more of a carbohydrate nutrient, a lipid nutrient, a vital dye, an alkalizer, an acidulant, and a salicylic acid compound.

In the context of the present disclosure, the term "quinine compound" as used herein refers to a pharmaceutically acceptable quinine and a structural analog thereof, such as quinine and an isomer and a pharmaceutically acceptable salt thereof. For quinine, its isomer may be, for example, quinidine, cinchonine, and cinchonidine, and its salts may be, for example, quinine hydrochloride, quinine dihydrochloride, quinine sulfate, and the like.

In one embodiment, the quinine compound is selected from a water-soluble quinine compound, preferably one or more selected from quinine hydrochloride, quinine dihydrochloride, quinine sulfate, and in the topical pharmaceutical composition, the concentration (w/v) of the quinine compound is greater than 1%, and preferably 3-10%.

In an embodiment, the topical pharmaceutical composition comprises the amino acid based nutrient and an anti-tumor chemotherapeutic agent.

In one embodiment, the topical pharmaceutical composition comprises the amino acid based nutrient, the anti-tumor chemotherapeutic agent, and one or more of a carbohydrate nutrient, a lipid nutrient, a vital dye, an alkalizer, an acidulant, a quinine compound, and a salicylic acid compound.

**In** the present disclosure, the term "anti-tumor chemotherapeutic agent" as used herein refers to an agent that can effectively inhibit a solid tumor by absorption at a safe dose, and is selected from any pharmaceutically acceptable anti-tumor chemotherapeutic agent, preferably selected from anti-tumor chemotherapeutic agents well known in the art, more preferably selected from anti-tumor chemotherapeutic agents that have been approved or will be approved by the official competent administrative department of China, the United States or Europe (for example, the FDA or China Food and Drug Administration), or have been included or will be included in the official pharmacopoeias of China, the United States or Europe. As used herein, the term "absorption" refers to the pharmacological effect of a drug being absorbed by the blood to form the drug-carrying blood into a target area; and the term "absorbed drug" refers to a therapeutic drug that generates a drug effect mainly through absorption.

In the present disclosure, the anti-tumor chemotherapeutic agent may be one or more selected from the following group: a drug that damages the structure and function of DNA, a drug that is intercalated in DNA and interferes with transcription of RNA, a drug that interferes with DNA synthesis, and a drug that affects protein synthesis. The drug that damages the structure and function of DNA includes, for example, an acidulant (for example, cyclophosphamide, and carmustine), a metal platinum complex (for example, cisplatin, and carboplatin), and a DNA topoisomerase inhibitor (for example, doxorubicin, topotecan, and irinotecan). The drug that is intercalated into DNA and interferes with transcription of RNA includes, for example, anti-tumor antibiotics, such as actinomycins, daunorubicin, doxorubicin, and the like. The drug that interferes with DNA synthesis includes, for example, a pyrimidine antagonist (such as a uracil derivative 5-fluorouracil, ftorafur, tegadifur, cytosine derivative cytarabine, cyclocytidine, 5-azacytidine), a purine antagonist (such as tisupurine, thioguanine), a folic acid antagonist (such as methotrexate). The drug that affects protein synthesis includes, for example, colchicines, vinblastines, taxanes (such as paclitaxel, docetaxel) and the like.

In the pharmaceutical composition according to this disclosure, the pharmaceutically acceptable liquid carrier is water and/or ethanol. The pharmacologically acceptable liquid carrier is mainly selected according to the properties of the anti-tumor chemotherapeutic agent, so that the agent can reach a corresponding concentration.

The anti-tumor chemotherapeutic agent is selected from a water-soluble anti-tumor chemotherapeutic agent and an alcohol-soluble anti-tumor chemotherapeutic agent. In the context of the present invention, the term "alcohol-soluble anti-tumor chemotherapeutic agent" refers to an anti-tumor chemotherapeutic agent that has solubility in ethanol or ethanol aqueous solution at room temperature greater than or equal to a concentration required for effective local action, including, for example, taxanes, vinblastines, and the like. The term "water-soluble anti-tumor chemotherapeutic agent" refers to an anti-tumor chemotherapeutic agent that has solubility in an aqueous solution at room temperature greater than or equal to a concentration required for effective local action, and includes, for example, one or more water-soluble drugs selected from a uracil derivative, cyclophosphamide, gemcitabine (such as gemcitabine hydrochloride), epirubicin (such as epirubicin hydrochloride), anti-tumor antibiotics (such as doxorubicin, actinomycetes), vinblastines (such as vinblastine sulfate), teniposide, a metal platinum complex, and the like.

In the pharmaceutical composition disclosed in the present application, the anti-tumor chemotherapeutic agent may be one or more selected from uracil derivatives, cyclophosphamides, gemcitabine, epirubicin, anti-tumor antibiotics, teniposide, a metal platinum complex, taxanes; preferably one or more selected from : 5-fluorouracil, cyclophosphamide, gemcitabine, epirubicin, anti-tumor antibiotics, teniposide, a metal platinum complex, and paclitaxel, and similar derivatives thereof.

In one embodiment, the pharmaceutically acceptable liquid carrier is water.

In the topical pharmaceutical composition according to the present disclosure, the concentration of the anti-tumor chemotherapeutic agent is greater than 30% of its saturated concentration, preferably 50-100% of its saturated concentration, wherein the saturated concentration refers to the saturation concentration of the anti-tumor chemotherapeutic agent in the liquid carrier.

In a specific embodiment, the topical pharmaceutical composition comprises an amino acid based nutrient and an anti-tumor chemotherapeutic agent, where the amount ratio (w:w) of the amino acid based nutrient to the anti-tumor chemotherapeutic agent is $\geq$ 125%.

In a specific embodiment, the concentration (w/v) of the anti-tumor chemotherapeutic agent selected from the acidulant (such as cyclophosphamide, carmustine) in the topical pharmaceutical composition is 0.5-6 %, preferably 0.75-1.5%.

In a specific embodiment, the concentration (w/v) of the anti-tumor chemotherapeutic agent selected from the metal platinum complex (such as cisplatin, carboplatin) in the topical pharmaceutical composition is 0.03-0.15%, and preferably 0.05-0.15%.

In a specific embodiment, the concentration (w/v) of the antitumor chemotherapeutic agent selected from the DNA topoisomerase inhibitor (such as doxorubicin, topotecan, irinotecan) in the topical pharmaceutical composition is 0.05-0.20%, and preferably 0.075-0.15%.

In a specific embodiment, the concentration (w/v) of the anti-tumor chemotherapeutic agent selected from the anti-tumor antibiotics (such as actinomycins, daunorubicin) in the topical pharmaceutical composition is 1-4%, and preferably 1-2%.

In a specific embodiment, the concentration (w/v) of the anti-tumor chemotherapeutic agent selected from the pyrimidine antagonist (such as a uracil derivative, such as 5-fluorouracil, ftorafur, tegadifur; a cytosine derivative, such as cytarabine, cyclocytidine, and 5-azacytidine) in the topical pharmaceutical composition is 0.5-2%, and preferably is 0.75-1.5%.

In a specific embodiment, the pharmaceutical composition comprises arginine, the uracil derivative (such as 5-fluorouracil), and/or the metal platinum complex, and in the topical pharmaceutical composition, the concentration (w/v) of arginine is 15-25%; the concentration (w/v) of the uracil derivative is 0.5-1.5%; and/or the concentration (w/v) of the metal platinum complex is 0.05-0.15%.

In a specific embodiment, the pharmaceutical composition comprises glutamate hydrochloride and the uracil derivative (such as 5-fluorouracil), and in the topical pharmaceutical composition, the concentration (w/v) of glutamate hydrochloride is 10-25%; and the concentration (w/v) of the uracil derivative is 0.5-1.5%.

In a specific embodiment, the concentration (w/v) of the anti-tumor chemotherapeutic agent selected from the taxanes (such as paclitaxel, docetaxel) in the topical pharmaceutical composition is 0.5-2%, and preferably 0.75-1.5%.

The pharmaceutical composition according to the present disclosure may further optionally comprise an excipient. The excipient may be any suitable one known to a person skilled in the art, which may include, for example, one or more of dispersion medium, preservative, stabilizer, wetting agent and/or emulsifier, solubilizer, tackifier, and the like. The thickener is, for example, sodium carboxymethyl cellulose, carboxymethyl cellulose, polyvinylpyrrolidone or gelatin. The preservative is, for example, an antioxidant (such as ascorbic acid).

The pharmaceutical composition according to the present disclosure may be in any dosage form suitable for topical administration which comprises an active ingredient (the amino acid based nutrient, the conventional ineffective compound, and optionally the other active ingredients as described above) and a liquid carrier (such as water, ethanol, or water/ethanol mixture), preferably the dosage form is an injection (preferably local injection), a liquid for external use, an atomizing preparation, and the like.

In the context of the present invention, the term "injection" as used herein refers to a sterile preparation for in vivo administration which comprises an active ingredient and a liquid carrier. The injections are divided according to the administration mode into local injections, intravenous injections, and the like, and the intravenous injections may be used as local injections only after reaching a given local administration concentration. Injections are divided according to types of commodities into liquid injections, injectable powder preparations, and the like. Injectable powder preparations contain sterile dry powder and a solvent, where the sterile dry powder contains part or all of the active ingredients, and the solvent contains all liquid carriers. The concentration of the active ingredient in the injection is the concentration of the active ingredient in the mixture of the active ingredient with all the liquid carriers, and is usually the concentration of the active ingredient in the liquid medicine at the end point (such as a needle hole, a catheter outlet, and the like) of a local administration device (a syringe, a puncture outfit, an injection catheter, and the like). For injectable powder preparations, the concentration of the active ingredient is the concentration of the active ingredient in the mixture of sterile dry powder and a solvent (for example, a reconstituted solution, or the pharmaceutically acceptable liquid carrier).

In the context of the present invention, the term "liquid for external use" refers to a liquid medicine that contains an active ingredient and a liquid carrier, and is administered to a body surface [such as skin, mucous membrane (such as ocular mucosa, nasal mucosa, and the like) or/and a cavity (such as oral cavity, rectum, vagina, urethra, nasal cavity, ear canal, and the like)], and includes, for example, lotion, liniment, drop, gargle, paint and the like. For local administration, the liquid medicine is generally provided by a local administration device such as a lotion bottle, a drip bottle, a dropping pipette, a gargle bottle, a cotton swab and the like. The concentration of the active ingredient in the liquid for external use is the concentration of the active ingredient in the liquid medicine.

In the scope of the present invention, the term "atomizing preparation" refers to a dosage form that contains an active ingredient and a liquid carrier and that can be used to spray the above liquid medicine through pressure when being used. The atomizing preparation may be administered to a skin and a mucous membrane (such as eye mucosa, nasal mucosa, and the like) or/and a cavity (such as oral cavity, rectum, vagina, urethra, nasal cavity, ear canal, and the like), and includes, for example, an aerosols, a sprays, a nebula and the like. In local administration, atomization of a liquid medicine is often formed by a local administration device such as an atomizer, a nebulizer, and a sprayer. After the medicine is sprayed to a target position, and it accumulates to form a liquid medicine, the composition of which is approximately the same as that of the liquid medicine before atomization. As such, the concentration of the active ingredient in the atomizing preparation can be represented by the concentration of the active ingredient in the liquid medicine before atomization.

According to another aspect of the present disclosure, it provides a topical pharmaceutical composition for use in treating a breast cancer in a lyophilized or semi-lyophilized form, obtainable or obtained through lyophilization or semi-lyophilization of part or all of the pharmaceutical composition including the amino acid based nutrient, the ineffective aromatic compound and the pharmaceutically acceptable carrier according to the present disclosure.

According to yet another aspect of the present invention, it further provides a device for treating a local lesion disease, wherein the device includes the pharmaceutical composition comprising the amino acid based nutrient and the conventional ineffective compounds according to the present disclosure. The device may be, for example, a device known in the art that can spray or atomize the liquid therein, such as an atomizer, a nebulizer, a sprayer, and the like.

A person skilled in the art would understand that, according to the technical solutions of the present invention, the composition of the present invention should be prepared into a dosage form that can be locally administered to a target area, preferably a topical pharmaceutical dosage form.

According to the preparation method of the present invention, the preparation of the pharmaceutical composition of the present invention includes the following steps: preparing a liquid pharmaceutical composition that includes the topical active ingredient(s), a liquid medium and optionally other substances. The liquid drug may be a solution (for example, a solution in a hydrophilic solvent, preferably an aqueous solution), a suspension, or an emulsion containing the topical active ingredient(s). When the liquid pharmaceutical composition is a suspension, the dispersion medium therein can be any suitable one known to a person skilled in the art, such as a micro-material or a nano-material. When the liquid pharmaceutical composition is an emulsion, the dispersion medium therein may be any suitable one known to a person skilled in the art, such as vegetable oil, synthetic oil or semi-synthetic oil that can be used for injection. The vegetable oil may be, for example, cottonseed oil, almond oil, olive oil, castor oil, sesame oil, soybean oil, and peanut oil.

According to the preparation method of the present invention, the concentrations of the amino acid based nutrient and the synergist (such as the conventional ineffective compound) are greater than or equal to their concentrations in the composition of the present invention. When such concentration is greater than the concentration in the pharmaceutical composition of the present invention, it can be further diluted for use.

According to the present disclosure, the liquid injection of the pharmaceutical composition of the present invention can be prepared by a method including the following steps: 1) adding an amino acid based nutrient, optionally a conventional ineffective compound, and optionally other ingredients in a required amount based on a local administration concentration to a solvent (or a pharmaceutically acceptable liquid carrier), mixing them to uniform, and sterilizing to prepare a sterile liquid I; 2) adding optionally other ingredients (such as other nutrients, and the like) in a required amount based on the local administration concentration to a solvent (or a pharmaceutically acceptable liquid carrier), mixing them to uniform, and sterilizing to prepare a sterile liquid II. In use, the sterile liquid I and the sterile liquid II form a mixed liquid before or after entering a local administration device, which can be used directly or diluted to be used as a liquid medicine for local administration.

According to the present disclosure, the injectable powder of the pharmaceutical composition of the present invention can be prepared by a method including the following steps: preparing a sterile dry powder of the amino acid based nutrient and optionally, for example, a conventional ineffective compound in a required amount based on a local administration concentration; and preparing a sterile vehicle containing other ingredients (such as an amino acid based nutrient, an analgesic, and the like) in a required amount of based on the local administration concentration. The sterile dry powder is preferably a sterile lyophilized dry powder, and a preparation method of the sterile dry powder includes: 1) preparing a solution containing an amino acid based nutrient, a conventional ineffective compound and optionally other components; 2) sterilizing, filtering and packaging; 3) lyophilizing; 4) and plugging and capping. Process conditions for lyophilizing include, for example, a pre-freezing condition in which a pre-freezing temperature -45°C is kept for 4 hours; a sublimation drying condition in which the temperature is raised to -15°C at a rate of 0.1°C/min and kept at least for 10 hours; and an adsorption drying condition in which a temperature of 30°C is kept for 6 hours. In use, the sterile dry powder of the injectable powder preparation is reconstituted in a sterile solvent to form a reconstituted drug, which can be used directly or diluted to be used as a liquid drug for local administration.

According to the present disclosure, the liquid for external use of the pharmaceutical composition of the present invention is prepared by a method including the following steps: 1) adding an amino acid based nutrient and a synergist (such as the conventional ineffective compound) and optionally other ingredients in a required amount based on a local administration concentration to a solvent to prepare a liquid; 2) adding other ingredients (such as a water-soluble acid) in a required amount based on the local administration concentration to the liquid prepared in 1), and evenly mixing them to obtain a liquid drug. In use, the liquid drug can be used directly or diluted to be used as the liquid for external use for local administration.

According to the present disclosure, the atomizing preparation of the pharmaceutical composition of the present invention can be prepared by a method including the following steps: 1) adding an amino acid based nutrient and a synergist (such as the conventional ineffective compound) and an atomizing excipient in a required amount based on a local administration concentration to a solvent to prepare a liquid; 2) adding other ingredients (such as a water-soluble acid) in a required amount based on the local administration concentration to the liquid prepared in 1),and mixing them evenly to obtain a liquid drug. atomizing excipients commonly used include, for example, glycerin, polysorbate-80, benzalkonium chloride, microcrystalline cellulose-sodium carboxymethyl cellulose and the like. **In** use, the liquid drug is added to an atomizer (such as a sprayer) and locally administered to a target area in a form of mist under an atomization action, where these mists are accumulated into the liquid drug at the target area.

According to the principles of the above methods, a person skilled in the art can adopt any suitable specific method to prepare a variety of specific dosage forms containing the composition of the present invention. For example, variants made to the composition of the present invention may include: containing different types and concentrations of the amino acid based nutrient, containing different types and concentrations of the synergist, containing different types and concentrations of other additives (such as an analgesic, an activator, and the like).

In the present disclosure, the pharmaceutical composition is for use in treating a breast cancer, through local administration.

In the context of the present invention, the term "local lesion disease" refers to a disease with a local lesion symptom, and the term "local lesion" refers to an original or secondary abnormality in a structure, morphology or function of a local body part of an animal (preferably human), which may include, for example, one or more of the followings: a tumor body, non-neoplastic enlargement, local inflammation, abnormal secretory function of secretory gland, and the like. The local body part may be any suitable one known to a person skilled in the art. For example, it may be a local body part that includes one or more of the following organs: a secretory organ in which the secretory system is located, a cardiovascular organ in which the blood circulation system is located, skin, and the like.

Local administration requires that the pharmaceutical composition (a local active ingredient, ratio and concentration of the ingredient(s)) can be administered to a tissue in which the local lesion is located by an interventional means, and generates a desired therapeutic effect in the tissue. For example, when the lesion is a tumor, the local tissue is the tumor body in which the tumor cells are located. When the lesion is non-neoplastic enlargement, the local tissue is an abnormality such as a swollen mass, such as hyperplasia, cyst, nodules, and the like. When the lesion is a local inflammation, the local tissue is an inflamed area, such as an inflamed swollen body. When the lesion is abnormal secretion, the local tissue is a source of the abnormality or a secretory gland in which such an abnormal secretion is located. For another example, when the disease is abnormal insulin secretion, the source of the abnormality is the pancreatic islet, and the local tissue is the pancreatic islet or the pancreas in which the pancreatic islet is located. When the disease is a skin disease, the local tissue is the lesion skin or an accessory organ of the lesion skin.

Specifically, in the present disclosure, the local lesion includes a tumor, non-neoplastic enlargement, local inflammation, abnormal secretory gland function, and a skin disease.

Within the context of the present invention, the term "tumor" refers to a swollen mass formed due to abnormal proliferation of cells or mutated cells, and includes a solid tumor. The term "solid tumor" refers to a tumor with a tumor body, and can be a tumor formed due to any pathology (malignant and non-malignant) and at any stage, including, for example, the following group classified according to tumor cell types: epithelial cell tumors, sarcomas, lymphoma, germ cell tumor, blastoma; and tumors named after an organ or tissue in which the tumor cell concentration area is located, including, for example, tumors named after the following organs or tissues: skin, bone, muscle, breast, kidney, liver, lung, gallbladder, pancreas, brain, esophagus, bladder, large intestine, small intestine, spleen, stomach, prostate, testis, ovary or uterus.

Specifically, the malignant tumor includes, breast cancer.

The non-malignant tumor includes, for example, breast tumor, pancreatic tumor, thyroid tumor, prostate tumor, liver tumor, lung tumor, intestinal tumor, oral tumor, esophageal tumor, stomach tumor, nasopharyngeal tumor, laryngeal tumor, testicular tumor, vaginal tumor, uterine tumor, fallopian tube tumor, ovarian tumor, and the like.

In an embodiment, the local lesion disease includes non-neoplastic enlargement. The term "non-neoplastic enlargement" refers to enlargement other than tumors, including, for example, hyperplasia (such hyperplasia of breast, pancreas, thyroid, parathyroid, prostate, and the like), cysts (such as cysts of breast, thyroid, parathyroid, and the like), nodules (such as nodules in breast, thyroid, parathyroid, and the like), abnormal venous mass (such as hemorrhoids), local inflammation and swelling, microbial infection and swelling, and the like. The hemorrhoids include internal hemorrhoids, external hemorrhoids, and mixed hemorrhoids.

In an embodiment, the local lesion disease includes local inflammation, especially refractory inflammation. Within the context of the present invention, the term "local inflammation" refers to non-neoplastic inflammation of a local body part, including, for example, alterative inflammation, exudative inflammation, and proliferative inflammation, which may be any suitable one known to a person skilled in the art, for example, including one or more of the followings: arthritis, mastitis, pancreatitis, thyroiditis, prostatitis, hepatitis, pneumonia, enteritis, stomatitis, pharyngitis, periodontitis, esophagitis, gastritis, gastric ulcer, rhinitis, sinusitis, laryngitis, tracheitis, bronchitis, vaginitis, metritis, salpingitis, oophoritis, and the like.

In one embodiment, the local lesion disease includes a skin disease, especially a refractory skin disease. Within the context of the present invention, the term "skin disease" refers to a lesion that is primary or secondary to a skin or a skin accessory organ, which may be any suitable one known to a person skilled in the art, for example, including one or more of the followings: skin cancer, non-malignant skin tumors, viral skin diseases (such as herpes, warts, rubella, hand-foot-and-mouth disease), bacterial skin diseases (such as impetigo, boils, leprosy), fungal skin diseases (such as various ringworms), sexually transmitted diseases (such as syphilis, gonorrhea, and condyloma acuminatum), allergic and autoimmune skin diseases (such as contact dermatitis, eczema, urticaria), physical skin diseases (such as solar skin diseases, frostbite, corns, cracked skin of hand and foot, pressure sores), connective tissue diseases (such as lupus erythematosus), pigmented skin diseases (such as freckles, pigmented moles, various spots), skin appendage diseases (such as acne, rosacea, seborrheic dermatitis, alopecia areata, baldness, hyperhidrosis and bromhidrosis).

In an embodiment, the local lesion disease includes abnormal secretory function of secretory gland. Within the context of the present invention, the term "secretory gland" refers to a structure that is formed by gland cells or gland cell population and performs a secretion function, including exocrine glands and endocrine glands. The abnormal secretory function of secretory gland includes hyperfunction of secretory glands (such as hyperthyroidism) and hypofunction of secretory glands (such as hypothyroidism, hypoinsulinism (a type of diabetes)).

In an embodiment, the local lesion disease includes a cardiovascular disease. Interventional therapy has become an important treatment for a cardiovascular disease. The cardiovascular disease includes, for example, hemangioma, hypertrophic obstructive cardiomyopathy, atrial fibrillation, arrhythmia, arterial embolism and the like.

The local pharmaceutical composition of the present invention is a therapeutic drug. When used to prevent and treat a local lesion disease, the pharmaceutical composition can also be applied in combination with other interventional therapies, systemic chemotherapy, immunotherapy, photodynamic therapy, sonodynamic therapy, surgical intervention or a combination thereof, to further increase the efficacy.

In the present disclosure, the pharmaceutical composition is mainly for used in treating breast cancer through local administration.

In the use and method for local treatment and prevention of a local lesion disease according to the present application, the amino acid based nutrient and the synergist thereof are locally administered at their concentrations or amount ratios in the topical pharmaceutical composition. This administration at the concentrations or amount ratios can provide a synergistic effect of local response.

Based on the studies described in more detail below, although specific mechanism has yet to be further studied, the composition of the present invention has been shown to be effective in promoting damage in a relevant structure of a tissue where a local lesion is located (such as a lesion tissue, lesion cells, and any structure involved in their formation) while minimizing damage to the patient's normal tissues, so as to achieve a safe and effective pharmaceutical effect of treating a local lesion disease.

Examples

The following specific examples are used to further illustrate the present invention, but not as a limitation to the present invention. In the following examples, all experimental animals were conducted in accordance with relevant regulations and industrial self-discipline. Unless otherwise specified, all tests were carried out according to conventional methods.

The materials and reagents used in the following specific examples can be obtained from commercial sources unless otherwise specified. Some amino acid based nutrients, conventional ineffective compounds, and anti-tumor chemotherapeutic agents used in the following examples are listed in Table 1.

Table 1

| Type | Compound | CAS No. | Type | Compound | CAS No. |
|---|---|---|---|---|---|
| Amino acid based nutrien t | L-arginine | 74-79-3 | Lipid nutrient | Eicosapentaenoic acid (EPA) | 10417-94-4 |
| | Glycine | 56-40-6 | | Docosahexaenoic acid (DHA) | 6217-54-5 |
| | L-cysteine hydrochloride | 52-89-1 | | Lecithin | 8002-43-5 |
| | L-arginine hydrochloride | 1119-34-2 | Acid | hydrochloric acid | 7647-01-0 |

(continued)

| Type | Compound | CAS No. | Type | Compound | | CAS No. |
|---|---|---|---|---|---|---|
| | L-lysine hydro-chloride | 657-27-2 | | Sulfuric acid | | 7664-93-9 |
| | L-threonine | 72-19-5 | | Maleic acid | | 110-16-7 |
| | L-proline | 147-85-3 | | acetic acid | | 64-19-7 |
| | L-glutamic hydrochloride | 138-15-8 | | Propanoic acid | | 79-09-4 |
| | L-valine | 72-18-4 | | Malonic acid | | 141-82-2 |
| | L-lysine | 56-87-1 | | Butyric acid | | 107-92-6 |
| | L-alanine | 56-41-7 | | DL-lactic acid | | 50-21-5 |
| | L-serine | 56-45-1 | | Citric acid | | 77-92-9 |
| | L-phenylalanine | 63-91-2 | | Citric acid | | 5949-29-1 |
| | Lysine-glycine dipeptide | 40719-58-2 | Ineffective aromatic compound | Vital dye | Methylene blue | 7220-79-3 |
| | Casein phosphopeptide | 691364-49-5 | | | Patent blue V | 3536-49-0 |
| | Alanyl-glutamine dipeptide | 39537-23-0 | | | Isosulfur blue | 68238-36-8 |
| | Reduced glutathione | 70-18-8 | | | Bengal Red | 632-69-9 |
| | Palmitoyl hexapeptide | 171263-26-6 | | Quinine compound | Quinine mono-hydrochl oride | 6119-47-7 |
| Carboh ydrate nutrien t | Glucose | 50-99-7 | | | Quinine dihydrochlorid e | 60-93-5 |
| | Gluconic acid | 147-85-3 | | | Quinine sulfate | |
| | Sodium gluconate | 71-00-1 | | Salicylic acid compou nd | Acetylsalicylic acid | 50-78-2 |
| | Fructose | 7660-25-5 | | | Salicylic acid | 69-72-7 |
| | Lactulose | 4618-18-2 | | | Aspisol | 62952-06-1 |
| | Trehalose | 6138-23-4 | Anti-tumo r chemother apeutic drug | Cyclophosphamide | | 50-18-0 |
| | Xylitol | 87-99-0 | | Cytarabine | | 147-94-4 |
| | Maltose | 69-79-4 | | Cisplatin | | 15663-27-1 |
| | Ribose | 50-69-1 | | Docetaxel | | 114915-14 -9 |
| | oligochitosan | 148411-57-8 | | Doxorubicin hydrochloride | | 25316-40-9 |
| | Dextran 40 | 9004-54-0 | | 5-fluorouracil | | 51-21-8 |

In the present invention, an L-amino acid is abbreviated as an amino acid (for example, L-arginine is abbreviated as arginine), a reduced glutathione is abbreviated as glutathione, and alanyl-glutamine dipeptide is abbreviated as ALA-GLN.

In the following examples, unless otherwise specified, animal tests of subcutaneous transplantation tumors were performed in accordance with test guidelines issued by a drug administration authority. The test animals were Balb/c nude mice or mice aged 6-8 weeks and weighing 17.5-20.5 g. The subcutaneous transplantation was carried out according to a conventional method for subcutaneous inoculation of tumor cells. Unless otherwise specified, when the tumor grew to a required volume (for example, 75-500 mm$^3$), the PEMS 3.2 software (compiled by West China School of Public Health, Sichuan University) was used to randomly divide them into several groups, each group with 6 animals. Items to be observed, measured and analyzed in the experiments included general status, body weight, food intake, tumor volume, tumor weight, thymus weight, spleen weight, and the like.

The tumor volume is calculated throug the following equation:

$$\text{Tumor volume (V)} = 1/2 \times a \times b^2,$$

wherein "a" represents the length of the tumor and "b" represents the width of the tumor.

The tumor growth inhibition rate (abbreviated as tumor inhibition rate in the present invention) is calculated through the following equation:

$$\text{Tumor inhibition rate Y(\%)} = (CW-TW)/CW \times 100\%,$$

wherein TW is an average tumor weight of a study group; CW is an average tumor weight of a negative control group.

In the following examples, the experimental results (such as the tumor weight) are expressed as mean $\pm$ standard deviation ($x \pm s$), and difference between two experimental animal groups and the group mean value was compared by using statistical software SPSS 13.0 or SPSS 19.0 software to perform a significance test, and the test was carried out by using a statistical quantity t, where the test level is $\alpha = 0.05$, and $P < 0.05$ indicates that the difference is statistically significant, otherwise there is no statistical significance.

Within the context of the present invention, a combination of drug A and drug B is denoted as B/A. In the following examples, unless otherwise specified, drug A and drug B are an amino acid based nutrient and another active ingredient, respectively.

The effects of the co-use of drugs (including efficacy and safety) can theoretically be based on the judgment of q:

$$q = \text{actual combined effect/theoretical expected effect of simple addition.}$$

When $q=1$, the actual combined effect meets the theoretical expectation, showing an additive effect; when $q<1$, the actual combined effect is weaker than the theoretical expectation, showing an antagonistic effect; and when $q>1$, the actual combined effect exceeds the theoretical expectation, showing a synergic effect. Unless otherwise specified, single-use efficacy of A or B (denoted as $E_A$ and $E_B$ respectively) and the actual combined efficacy of A/B (denoted as $E_{A+B}$) are both tumor inhibition rates. There are many methods for calculating the theoretical expected drug effect of simple addition in the above q calculation equation, most of which are for the cell experimental effect.

Improving the efficacy of anti-tumor drugs has always been the world's biggest medical problem. As it is extremely difficult to improve the efficacy by even a few percentage points, the theoretical expectation of co-use of drugs in animal experiments is usually not high, but once such improvement is realized, it is of great significance. A common method for judging the efficacy of co-use of anti-tumor drugs in animal experiment literature is based on an assumption that the actual combined effect significantly exceeding the single-use effect indicates being far beyond theoretical expectations. The specific judgment is as follows: ($E_{A+B} > E_A$ and $E_{A+B} > E_B$), and the difference between the composition group and each ingredient group is statistically significant (both with $p<0.05$).

Another method for judging the effect of co-use of drugs in animal experiments was the Burgi method (Burgi Y. Pharmacology; Drug actions and reactions. Cancer Res. 1978, 38(2), 284-285). Jin Zhengiun improved the Burgi method (Jin Zhengjun, Addition in combined medication, Chinese Journal of Pharmacology, 1980, 1(2), 70-76), and q was calculated through the following equation:

$$q = E_{A+B}/(E_A + E_B - E_A \cdot E_B),$$

($E_A + E_B - E_A \cdot E_B$) is the expected effect of drug A and drug B.

In the following anti-tumor animal experiments, the effects of the co-use of drugs were judged as shown in Table 2.

Table 2

| Drug effect ($E_{A+B}$ judgment or q judgment) | Compare group A/B with group A and B, to see if there is any statistically significant difference in efficacy (judgment of p) | | Judge of joint use of drugs |
|---|---|---|---|
| $E_{A+B} > E_A$ and $E_{A+B} > E_B$ | Both have $p<0.05$ | | Synergistic efficacy |
| $E_{A+B} > E_A$ and | | Not both have | Non synergistic efficacy |
| $E_{A+B} > E_B$ | | $p<0.05$ | |

(continued)

| Drug effect ($E_{A+B}$ judgment or q judgment) | Compare group A/B with group A and B, to see if there is any statistically significant difference in efficacy (judgment of p) | | Judge of joint use of drugs |
|---|---|---|---|
| $E_{A+B} < E_A$ and $E_{A+B} < E_B$ | Both have $p<0.05$ | | Antagonistic effect |
| q>1.00 | Both have $p<0.05$ | | Significant synergistic efficacy |
| q>1.00 | | Not both have $p<0.05$ | Not significant synergistic efficacy |
| q<1.00 | Both have $p<0.05$ | | Significant antagonistic efficacy |
| q<1.00 | | Not both have $p<0.05$ | Not significant antagonistic effect |
| q=1.00 | Both have $p<0.05$ | | Significant addition efficacy |
| q=1.00 | | Not both have $p<0.05$ | Not significant addition |

Example 1: Basic research on use of amino acid based nutrients in the composition of the present invention

In the prior art, it is reported that amino acid based nutrients may facilitate tumor nutrition. Therefore, anti-tumor effect of the amino acid based nutrients is difficult to predict: they may inhibit tumors, or may not inhibit tumors, or may help tumors. Through the following experiments, basic technical solution for application of amino acid based nutrients in the composition of the present invention is optimized.

1. The pharmacologically preferred amino acid based nutrients in the composition of the present invention

In this research experiment, not according to the claimed invention, the experimental animals (nude mice bearing pancreatic cancer cells PANC-1, with an average tumor volume of 105mm$^3$) successfully modeled were randomly divided into 2 negative control groups and 14 study groups. The negative control was normal saline, and 7 study drugs were as shown in the table below. They were injected intraperitoneally and intratumorally. The drugs were all aqueous solutions, which were prepared according to the preparation method of Example 1. Each group was given the drug once every 3 days for a total of 3 times, each time at a dose of 100μl/mouse. The next day after the administration of the drugs was ended, the animals were euthanized, and the tumor weight was measured after anatomy to the animals. The tumor inhibition rate was calculated from the negative control group of each drug administration mode. The results are shown in Table 3.

Table 3

| Study material | Tumor inhibition rate of intraperitoneal injection | Tumor inhibition rate of intratumoral injection |
|---|---|---|
| 1% 5-fluorouracil | 59% | 63% |
| 20% arginine | 5% | 56% |
| 20% lysine | 1% | 53% |
| 20% glycine | 4% | 52% |
| 10% glutathione | 3% | 51% |
| 10% ALA-GLN | 2% | 53% |
| 3% glutamic hydrochloride | 5% | 68% |

In Table 3, the positive control (5-fluorouracil) meets its expectations as an anti-tumor cell drug. The results of the intraperitoneal injection group showed that anti-tumor cell drugs can target tumor cells in the form of blood drugs to inhibit tumor growth. It was once generally believed that the intratumoral administration of anti-tumor cell drugs can increase the local concentration of the drugs, and may greatly improve their efficacy. However, as shown in Table 3, intratumoral injection did not significantly increase the tumor inhibition rate of 5-fluorouracil. It shows that the drug did not substantially change its targeting (tumor cells) and pharmacology (inhibition of tumor cells) when injected intratumorally. It also faced the same intra-tissue targeting barrier, thus showing consistent efficacy. Therefore, unless it is placed in a delayed release system, conventional anti-tumor cell drugs are still mainly administrated through absorption rather than local administration.

In Table 3, the intraperitoneal injection of amino acid based nutrients met its expectations in normal application (absorption through blood), and the tumor inhibition rate was less than 20%, which was deemed as ineffective. Unexpectedly, the tumor volume growth of the intratumoral injection group started to be lower than that of the intraperitoneal injection group on the 3rd day after the first administration. Under the same experimental conditions, it showed obvious effective drug effect, which showed a significant difference in targeting and pharmacology than that of the intraperitoneal injection group.

In another experiment, not according to the claimed invention, the test animals that had been successfully modeled (nude mice bearing pancreatic cancer cells PANC-1, with an average tumor volume of 165mm$^3$) were randomly divided into a blank group (no injection) and two study groups A and B. Groups A and B received intraperitoneal injection and intratumoral injection of 10% glutathione aqueous solution, respectively. The drug was prepared according to the preparation method of Example 1. Both groups A and B were administered the drugs once, with 150µl each time. The next day after the administration of drug was ended, the 3 groups of animals were euthanized, and the tumors were dissected for histological observation. It was found from such observation that there was no obvious difference between group A and the blank group, while group B was significantly different from the blank group, showing obvious intratumoral tissue destruction.

According to the above results, the application of the amino acid based nutrients in the composition of the present invention is preferred synergistic pharmacology for targeting and destroying a tumor tissue through its local action, rather than a conventional pharmacology selection of targeting and inhibiting tumor cells through its absorption.

2. The preferred essential composition of amino acid based nutrients in the composition of the present invention not according to the claimed invention

In this research experiment, the experimental animals that had been successfully modeled (nude mice bearing pancreatic cancer cells PANC-1, with an average tumor volume of 136mm$^3$) were randomly divided into a negative control group and 28 study groups. The negative control was normal saline, and the composition of the study drug is shown in the following table. The drugs were all aqueous solutions and were prepared according to the preparation method of Example 1. All groups were injected intratumorally, once every 3 days for a total of 3 times. The dose of each administration was: arginine $\leq$ 1000 mg/kg, reduced glutathione $\leq$ 1000 mg/kg, glutamic hydrochloride $\leq$ 500mg/kg, glucose $\leq$1500mg/kg, injection volume $\leq$150µl. The next day after the administration of drugs was ended, the animals were euthanized. After anatomy, the tumor weight was measured and the tumor inhibition rate was calculated. The results are shown in Table 4.

Table 4

| X% arginine | | X% glutathione | | X% glutamic hydrochloride | | X% glucose | |
| --- | --- | --- | --- | --- | --- | --- | --- |
| X% (concent ration) | Tumor inhibitio n rate | X% (concent ration) | Tumor inhibiti on rate | X% (concentrati on) | Tumor inhibiti on rate | X% (concentrati on) | Tumor inhibiti on rate |
| 1% | 17% | 1% | 11% | 0.15% | 16% | 1% | 9% |
| 2% | 15% | 2% | 13% | 0.25% | 13% | 2% | 11% |
| 3% | 19% | 3% | 12% | 0.5% | 11% | 3% | 13% |
| 5% | 16% | 5% | 32% | 1 % | 18% | 5% | 8% |
| 7.5% | 21% | 7.5% | 45% | 2% | 49% | 7.5% | 12% |
| 10% | 43% | 10% | 57% | 3% | 69% | 10% | 13% |
| 20% | 61% | 12.5% | 66% | 5% | 76% | 20% | 12% |

In Table 4, in a larger concentration range (<2%, <5% or <10%), amino acid based nutrients and carbohydrate nutrients appear to be the same, and their tumor inhibition rates are not even significantly different from those of the negative control group (tumor inhibition rate <20%), and the doubled increase of local drug concentration did not increase its tumor inhibition rate. After reaching a concentration threshold (arginine concentration $\geq$ 10%, reduced glutathione concentration $\geq$ 5%, or glutamic hydrochloride $\geq$ 2%), the tumor inhibition rate of amino acid based nutrients began to be correlated with the composition of the drug (drug type and local administration concentration), even if the administration dose of this concentration was the same as that of the previous concentration. Glucose did not have this threshold and had not shown a significant increase in tumor inhibition rate. These results show that the composition of topical drugs depended not only on the type of substance (amino acid based nutrients rather than carbohydrate nutrients), but also on the concentration of the active ingredient of the topical drug (greater than the threshold, rather than less than the threshold).

According to the above research and more similar studies, the technical solution for the application of the amino acid based nutrient in the composition of the present invention as a local active ingredient is: local administration, and the local administration concentration of the amino acid based nutrient is ≥2%, preferably 3%-25%, wherein:

when the amino acid based nutrient includes an acidic amino acid salt, the local administration concentration of the amino acid based nutrient is ≥2%, preferably 3%-25%;

when the amino acid based nutrient is one or more selected from an amino acid or/and an amino acid salt other than an acidic amino acid salt, the local administration concentration of the amino acid based nutrient is 10%-25%;

when the amino acid based nutrient is selected from one or more of oligopeptides, the local administration concentration of the amino acid based nutrient is 7.5%-25%; and

when the amino acid based nutrient includes one or more of oligopeptides, amino acids, or/and amino acid salts other than acidic amino acid salts, the local administration concentration of the amino acid based nutrient is 7.5%-25%.

According to the above results and similar studies, comparing the application of the amino acid based nutrient in the composition of the present invention as a local active ingredient (intratumoral injection in the experiment) with the application (intraperitoneal injection in the experiment) of its conventional absorption active ingredient (usually used as a nutrient ingredient in absorbed drugs), there are essential differences:

1) Targeting is different. The amino acid based nutrients in absorbed drugs (such as nutraceuticals) enter the body and disperse in the blood. After the blood carrying the drugs enters the target area passively, they can only perform targeting, such as targeting tumor cells under conditions controlled by the penetration of the blood vessel wall and other tissue barriers. The amino acid based nutrient in the topical drug of the present invention directly target a pathologic tissue (such as an intratumoral tissue), including, for example, blood supply vessels, intercellular substance, and/or other microenvironmental structures in the tumor tissue.

2) The composition of the drug in the target area is different. After the amino acid based nutrient in the pharmaceutical composition is absorbed into the body, their composition in the medicated blood in the target area is quite different from the composition in the original drug (for example, the concentration of free amino acid based nutrient). This composition is no longer the previous one. In the drug liquor in the target area, the composition of the amino acid based nutrients in the topical pharmaceutical composition of the present invention is basically the same as that in the original drug.

3) Different targeting and different composition lead to different pharmacology. Literature has shown that nutrients transported through blood have nutritional effects on both tumor cells and other cells. In the above test, the amino acid based nutrients in the absorbed drug (in the intraperitoneal injection group) did not show a tumor-inhibiting effect that is different from glucose, or even significantly different from normal saline. The amino acid based nutrients in the topical composition (intratumoral injection group) were used as a local active ingredient to destroy the tumor tissue and make the tumor cells lose the basis for survival and proliferation. Under the same dosage, the different pharmacology of the two groups produced completely different effects.

4) Pharmacokinetics effects are different. The absorbed drug (such as a cytotoxic drug) is often based on the results of cell experiment, and its key pharmacokinetic parameter is the blood concentration of the active ingredient (often close to the effective concentration in the cell experiment). The blood drug concentration is usually not guaranteed by the administered concentration (which is often diluted for administration), but by the administered dosage. The amino acid based nutrients used as topical active ingredients show completely different pharmacokinetics. In the above test, there was a local administration concentration threshold and a local administration concentration interval. Only when the concentration threshold was exceeded and within a specific concentration interval, the local activity could be produced.

Example 2: Study on the cell independence of the application of amino acid based nutrients in the composition of the present invention

One of the core characteristics of tumors is heterogeneity. Conventional cytotoxic drugs mostly target tumor cells, and therefore show cell dependence. In order to further confirm the essential difference between its function as a local active ingredient and an absorption active ingredient (for example, showing cytotoxicity), the following experiments studied the cell-independence of amino acid based nutrients as a local active ingredient. In the following experiments, the nude mice bearing human tumor cells and successfully modeled were randomly divided into 1 negative control group and 4 study groups (groups A, B, C, and D). The corresponding negative control was normal saline. The four study drugs were 20% arginine, 3% glutamic hydrochloride, 10% alanyl-glutamine, and 10% glutathione. Each group was injected intratumorally, once every 3 days, for a total of 3 times, each time with 100µl/mouse. On the next day after the administration of drugs was ended, the animals were euthanized, the tumor weight was measured after anatomy, and the tumor inhibition rate was calculated from the respective negative control group.

1) Application in the treatment of breast tumor

In this research experiment, successfully modeled nude mice bearing human breast cancer cells (MDA-MB231) (with an average tumor volume of $71mm^3$) were randomly divided into a negative control group and 4 study groups (groups A, B, C, and D). The tumor inhibition rates of groups A, B, C, and D were 55%, 61%, 57%, and 64%, respectively, which all met the generally considered effective anti-tumor standard (tumor inhibition rate $\geq 40\%$).

2) Application in the treatment of lung tumor not according to the claimed invention

In this research experiment, successfully modeled nude mice bearing human lung cancer cells (A549) (with an average tumor volume of $83mm^3$) were randomly divided into a negative control group and 4 study groups (groups A, B, C, and D). The tumor inhibition rates of groups A, B, C, and D were 53%, 57%, 52%, and 59%, respectively, which all met the generally considered effective anti-tumor standard (tumor inhibition rate $\geq 40\%$).

3) Application in the treatment of thyroid tumor not according to the claimed invention

In this research experiment, successfully modeled nude mice bearing human thyroid cancer cells (SW579) (with an average tumor volume of $103mm^3$) were randomly divided into a negative control group and 4 study groups (groups A, B, C, and D). The tumor inhibition rates of groups A, B, C, and D were 62%, 63%, 59%, and 61%, respectively, which all met the generally considered effective anti-tumor standard (tumor inhibition rate $\geq 40\%$).

4) Application in the treatment of prostate tumor not according to the claimed invention

In this research experiment, successfully modeled nude mice bearing human prostate cancer cells (LNCaP/AR) (with an average tumor volume of $91mm^3$) were randomly divided into a negative control group and 4 study groups (groups A, B, C, and D). The tumor inhibition rates of groups A, B, C, and D were 55%, 59%, 61%, and 65%, respectively, which all met the generally considered effective anti-tumor standard (tumor inhibition rate $\geq 40\%$).

5) Application in the treatment of liver tumor not according to the claimed invention

In this research experiment, successfully modeled nude mice bearing human liver cancer cells (HepG2) (with an average tumor volume of $81mm^3$) were randomly divided into a negative control group and 4 study groups (groups A, B, C, and D). The tumor inhibition rates of groups A, B, C, and D were 63%, 62%, 56%, and 61%, respectively, which all met the generally considered effective anti-tumor standard (tumor inhibition rate $\geq 40\%$).

6) Application in the treatment of head and neck tumors not according to the claimed invention

In this research experiment, successfully modeled nude mice bearing human head and neck cancer cells (F$\mu$da) (with an average tumor volume of $109mm^3$) were randomly divided into a negative control group and 4 study groups (groups A, B, C, and D). The tumor inhibition rates of groups A, B, C, and D were 56%, 58%, 51%, and 53%, respectively, which all met the generally considered effective anti-tumor standard (tumor inhibition rate $\geq 40\%$).

7) Application in the treatment of nasopharynx tumor not according to the claimed invention

In this research experiment, successfully modeled nude mice bearing human nasopharynx cancer cells (CNE1) (with an average tumor volume of $78mm^3$) were randomly divided into a negative control group and 4 study groups (groups A, B, C, and D). The tumor inhibition rates of groups A, B, C, and D were 63%, 68%, 63%, and 61%, respectively, which all met the generally considered effective anti-tumor standard (tumor inhibition rate $\geq 40\%$).

8) Application in the treatment of stomach tumor not according to the claimed invention

In this research experiment, successfully modeled nude mice bearing human stomach cancer cells (BGC823) (with an average tumor volume of $85mm^3$) were randomly divided into a negative control group and 4 study groups (groups A, B, C, and D). The tumor inhibition rates of groups A, B, C, and D were 52%, 57%, 51%, and 59%, respectively, which all met the generally considered effective anti-tumor standard (tumor inhibition rate $\geq 40\%$).

9) Application in the treatment of ovary tumor not according to the claimed invention

In this research experiment, successfully modeled nude mice bearing human ovary cancer cells (PA1) (with an average tumor volume of 104mm$^3$) were randomly divided into a negative control group and 4 study groups (groups A, B, C, and D). The tumor inhibition rates of groups A, B, C, and D were 56%, 61%, 62%, and 67%, respectively, which all met the generally considered effective anti-tumor standard (tumor inhibition rate ≥40%).

The above studies and other similar studies further show that, completely different from its application as a cytotoxic drug, the amino acid based nutrient in the composition of the present invention targets a tumor tissue (thus no obvious tumor cell dependence), rather than targeting tumor cells (therefore dependent on tumor cells).

Example 3: Preparation of the pharmaceutical composition of the present invention not according to the claimed invention

According to the above preparation method of the composition of the present invention, many different compositions of the present invention can be prepared. The components of some of the compositions of the present invention prepared in this example are listed in Table 5.

Table 5

| No. | Composition | | | |
|---|---|---|---|---|
| | amino acid based nutrient | conventional ineffective compound | | anti-tumor chemotherapeutic agent |
| | | other nutrients | ineffective aromatic compound | acidulant and/or alkalizer | |
| 1 | 10% glutathione | 30% sodium gluconate | | | |
| 2 | 10% glutathione | 30% xylitol | | | |
| 3 | 10% ALA-GLN | 30% ribose | | | |
| 4 | 10% glutathione | 30% lactulose | | 5% acetic acid | |
| 5 | 10% glutathione | 15% glucose 15% fructose | | 5% acetic acid | |
| 6 | 10% glutathione | 10% glucose, 15% oligomannose | | 5% acetic acid | |
| 7 | 10% glutathione | 30% maltose | | 5% acetic acid | |
| 8 | 20% arginine | 30% xylitol | | 5% acetic acid | |
| 9 | 10% palmitoyl hexapeptide | 30% glucose | | 5% acetic acid | |
| 10 | 10% soy oligopeptides | 30% glucose | | 5% acetic acid | |
| 11 | 3% glutamic hydrochloride | 5% DHA | | | |
| 12 | 3% glutamic hydrochloride | 5% EPA | | | |
| 13 | 3% glutamic hydrochloride | 5% DHA | | | 1% 5-fluorouracil |
| 14 | 20% arginine | | 0.8% methylene blue | | |
| 15 | 20% glycine | | 0.8% methylene blue | 10% acetic acid | |
| 16 | 20% arginine | | 0.8% patent blue | | |
| 17 | 20% lysine | | 0.8% patent blue | | |

(continued)

| No. | Composition | | | | |
| --- | --- | --- | --- | --- | --- |
| | amino acid based nutrient | conventional ineffective compound | | | anti-tumor chemotherapeu tic agent |
| | | other nutrients | ineffective aromatic compound | acidulant and/or alkalizer | |
| 18 | 7% lysine, 7% arginine, 6% glycine | | 0.8% patent blue | | |
| 19 | 10% Lysine-glycine dipeptide | | 0.8% patent blue | | |
| 20 | 10% ALA-GLN | | 0.8% methylene blue | | |
| 21 | 10% glutathione | | 0.8% methylene blue | | |
| 22 | 10% palmitoyl hexapeptide | | 0.8% methylene blue | | |
| 23 | 20% arginine | | 5% quinine dihydrochlorid e | | |
| 24 | 20% glycine | | 3% quinine dihydrochlorid e | 5% acetic acid | |
| 25 | 20% lysine hydrochloride | | 5% salicylic acid | | |
| 26 | 20% glycine | | | 5% acetic acid, 1% hydrochloric acid | |
| 27 | 5% lysine hydrochloride, 4% proline, 4% arginine, 4% glycine, 3% serine | | | 5% acetic acid | |
| 28 | 20% lysine hydrochloride | | | 5% acetic acid | |
| 29 | 20% lysine hydrochloride | | | 5% acetic acid | 1% cyclophospha mide |
| 30 | 4% glutamic hydrochloride | | | | 1% 5-fluorouracil |
| 31 | 4% glutamic hydrochloride | | | | 1% cyclophospha mide |
| 32 | 4% glutamic hydrochloride | | | | 1% adriamycin |
| 33 | 15% glycine | | | 7% sodium hydroxide | |
| 34 | 15% glycine | | | 5% sodium carbonate | |
| 35 | 15% arginine | 30% xylitol | | 1% sodium hydroxide | |
| 36 | 15% arginine | 30% xylitol | | 5% sodium carbonate | |
| 37 | 20% arginine | 20% glucose | | 2% potassium chloride/1 % so-dium hydroxide | |

(continued)

| No. | Composition | | | | |
| --- | --- | --- | --- | --- | --- |
| | amino acid based nutrient | conventional ineffective compound | | | anti-tumor chemotherapeutic agent |
| | | other nutrients | ineffective aromatic compound | acidulant and/or alkalizer | |
| 38 | 15% arginine | 30% glucose | | 7% sodium bicarbonate/4 % sodium carbonate | |
| 39 | 20% arginine | | | 5% sodium carbonate | |
| 40 | 20% arginine | | | 7% sodium carbonate/1% potassium oxide | |

1. Preparation of liquid injection (1) not according to the claimed invention,

An amino acid based nutrient (for example 10 g glutathione), an anti-tumor chemotherapeutic agent or/and a conventional ineffective compound (for example 30 g glucose), optionally other ingredients, and a liquid carrier (for example, water for injection) that had been adjusted to a constant total volume (for example, 100 ml) were measured and taken according to a required concentration (as shown in Table 5). The mixture was slowly mixed evenly, sterilized and filtered, and then divided into a required amount (for example, 10 ml/bottle) for storage. The preparation (for example, 10% glutathione/30% glucose aqueous solution) can be administered locally as a liquid drug.

2. Preparation of liquid injection (2)

1). An amino acid based nutrient (for example 10 g glutathione), an anti-tumor chemotherapeutic agent or/and a conventional ineffective compound (for example 30 g glucose), optionally other components, and a liquid carrier (for example, water for injection) that had been adjusted to a constant total volume (for example, 85 ml) were measured and taken according to a required concentration (as shown in Table 5). The mixture was slowly mixed evenly, sterilized and filtered, and then divided into a required amount (for example, 8.5ml/bottle) for storage. This was a solution I.

2). An acidulant (for example 5g of acetic acid), optionally other ingredients, and a solvent (for example water for injection) that had been adjusted to a constant total volume (for example 15ml) were measured and taken according to a required concentration (as described in Table 5), and mixed slowly and evenly, sterilized and filtered, and divided into a required amount (for example, 1.5ml/bottle) for storage for later use. This was solution II.

3). Solution I and solution II were mixed evenly according to a required concentration of each component (for example, 8.5 ml solution I and 1.5ml solution II were mixed) into a mixed solution (for example, 10% glutathione/30% glucose/5% acetic acid aqueous solution) to be used as a liquid drug for topical administration.

3. Preparation of an injectable powder preparation

1). An amino acid based nutrient (for example 10g glutathione), an anti-tumor chemotherapeutic agent or/and a conventional ineffective compound (for example 30g glucose), optionally another ingredient and a liquid carrier (for example water for injection) that had been adjusted to a constant total volume (for example 100ml) were measured and taken according to a required concentration (as described in Table 5), mixed slowly and evenly, sterilized and filtered, and then divided into a required amount (e.g. 10ml/bottle) for freeze-drying, stoppering and capping, to be prepared into sterile dry powder for later use.

2). Optionally remaining ingredients (if any, such as 5 g acetic acid), and a liquid carrier (for example water for injection) that had been adjusted to a constant total volume (for example 100ml) were measured and taken according to a required concentration (as described in Table 5), and mixed slowly and evenly, sterilized and filtered, and divided into a required amount (for example, 10 ml/bottle), to be prepared into a sterile liquid for later use.

3). A sterile powder in a required amount (for example, 1 bottle of the above dry powder) were reconstituted in a sterile liquid in a required amount (for example 1 bottle of the above solvent) according to a required concentration of each ingredient into a reconstituted solution (for example, 10% glutathione/30% glucose/5% acetic acid aqueous solution) to be used as a liquid drug for topical administration.

4. Preparation of a liquid for external use

An amino acid based nutrient (10 g glutathione), a conventional ineffective compound (for example 30 g glucose), optionally another ingredient, and a solvent (for example water) that had been adjusted to a constant volume of 80% to 90% of a total volume (for example 80-90ml) were measured and taken according to a required concentration (as described in Table 5), mixed slowly and evenly, and then added with remaining conventional ineffective compound (if any, for example 5 g acetic acid). The optionally remaining other components (if any), and a solvent (such as water for injection) that had been adjusted to a constant total volume (for example 100 ml) were taken, and mixed slowly and evenly. The preparation (for example, 30% glucose/10% glutathione/5% acetic acid aqueous solution) can be directly used as a topical liquid drug for topical administration.

5. Preparation of an atomizing preparation

An amino acid based nutrient (for example, 3 g glutamic hydrochloride), a conventional ineffective compound (for example, 30 g glucose), and the following auxiliary materials for the atomizing preparation: glycerol (2.5 g), polysorbate-80 (1.5 g), benzalkonium chloride (0.02 g), microcrystalline cellulose-sodium carboxymethyl cellulose (1.5 g), and a solvent (such as water for injection) that had been adjusted to a constant total volume (such as 100ml) were measured and taken according to a required concentration (as described in Table 5), and mixed slowly for later use. The preparation (for example, a stock solution of a spray containing 30% glucose/3% glutamic hydrochloride), after being added to the sprayer, may be directly sprayed on a target area to form a liquid drug.

Example 4: Pharmacologically preferred synergistic composition

The studies of Examples 1 and 2 above show that amino acid based nutrients can show local activity under necessary conditions (local administration, concentration threshold). Like all active ingredients, use of the amino acid based nutrients with other substances may generate a synergistic effect, may also generate an additive effect, and may also generate an antagonistic effect. It may be seen from the very few clinical options available that, the probability that (tens of thousands) anti-tumor active ingredients produce a synergistic effect through (hundreds of thousands) co-use options to become (dozens) effective drugs is very low.

1. Amino acid based nutrients/conventional ineffective compound composition not according to the claimed invention,

In an experiment, the experimental animals (nude mice bearing HepG2, with an average tumor volume of 153 mm$^3$) successfully modeled were randomly divided into 2 negative control groups and 22 study groups. The negative control groups were given normal saline, and 11 study drugs were as shown in the table below. They were injected intraperitoneally and intratumorally. The drugs were all aqueous solutions, which were prepared according to the preparation method of Example 1. Each group was administered once every 3 days for a total of 3 times, each time at an injection dose of ≤120μl/mouse. On the 5th day after the administration was ended, the animals were euthanized, and the tumor weight was measured after anatomy to the animals. The tumor inhibition rate was calculated from the negative control group of each drug administration mode. The results are shown in Table 6.

Table 6

| Group | Study drug | Tumor inhibition rate of intraperitoneal injection | Intratumoral injection | |
|---|---|---|---|---|
| | | | Tumor weight (g) | Tumor inhibition rate |
| 0 | Normal saline | (0) | 2.32±0.38g | (0) |
| 1 | 1% 5-fluorouracil | 59% | 0.78±0.21g | 66% |
| 2 | 10% glutathione | 3% | 0.97±0.24g | 58% |
| 3 | 25% glucose | 1% | 1.67±0.29g | 28% |

(continued)

| Group | Study drug | Tumor inhibition rate of intraperitoneal injection | Intratumoral injection | |
|---|---|---|---|---|
| | | | Tumor weight (g) | Tumor inhibition rate |
| 4 | 3.5% acetic acid | 6% | 0.84±0.23g | 64% |
| 5 | 0.7% methylene blue | 2% | 2.02±0.31g | 13% |
| 6 | 5% DHA | 4% | 1.69±0.33g | 27% |
| 7 | 10% glutathione/25% glucose | 5% | 0.42±0.18g | 82% |
| 8 | 10% glutathione/3.5% acetic acid | 1% | 0.35±0.21g | 85% |
| 9 | 10% glutathione/0.7% methylene blue | 3% | 0.26±0.17g | 89% |
| 10 | 10% glutathione/5% DHA | 4% | 0.67±0.26g | 71% |
| 11 | 10% glutathione/25% glucose/ 3.5% acetic acid | 2% | 0.14±0.13g | 94% |

In Table 6, the positive control (5-fluorouracil) meets its expectations as an anti-tumor cell drug. The results of the intraperitoneal injection group showed that anti-tumor cell drugs can target tumor cells in the form of blood drugs to inhibit tumor growth. It was once generally believed that administration in tumors can increase the local concentration of anti-tumor cell drugs, and may greatly improve their efficacy. However, as shown in Table 6, intratumoral injection did not significantly increase the tumor inhibition rate of 5-fluorouracil. It shows that the drug did not substantially change its targeting (tumor cells) and pharmacology (inhibition of tumor cells) when injected intratumorally. It also faced the same intra-tissue targeting barrier, thus showing consistent efficacy. Therefore, unless it is placed in a delayed release system, conventional anti-tumor cell drugs are still mainly administered through absorption rather than locally administration.

In Table 6, the intraperitoneal injection group of the amino acid based nutrients/conventional ineffective compounds compositions met the expectations in normal application (absorption through blood), and the tumor inhibition rate were less than 20%, showing no synergistic efficacy. Unexpectedly, the tumor volume growth of the intratumoral injection group started to be lower than that of the intraperitoneal injection group on the 3rd day after the first administration. In each group of the composition (study groups 7-11), the tumor inhibition rate shown by intratumoral injection was more than 10 times higher than that of intraperitoneal injection, which showed a significantly different targeting and pharmacology from the intraperitoneal injection group of the composition.

In the above table, q of the intratumoral injection groups the following composition was greater than 1.00, and had statistically significant (p<0.05, p<0.05) difference in tumor weight from the ingredient groups, so these composition groups showed significant synergistic efficacy: 10% glutathione/25% glucose (q=1.17>1.00, and the tumor weight difference from the 10% glutathione group and the 25% glucose group was P<0.05), 10% glutathione/0.7% methylene blue (q=1.40>1.00, and the tumor weight difference from the 10% glutathione group and 0.7% methylene blue group was P<0.05), 10% glutathione/5 %DHA (q=1.02>1.00, and the tumor weight difference from the 10% glutathione group and the 5% DHA group was P<0.05), 10% glutathione/25% glucose/3.5% acetic acid (q =1.01>1.00, and the tumor weight difference from the 10% glutathione/3.5% acetic acid group and the 25% glucose group was P<0.05).

In the above table, the tumor inhibition rate of study group 8 was higher than that of each of study groups 2 and 4 ($E_{A+B} > E_A$ and $E_{A+B} > E_B$), and the differences in tumor weight between composition groups 8 and 2, and between the composition groups 8 and 4 were statistically significant (P<0.05, P<0.05, respectively), so the composition study groups showed a synergistic effect.

Experiments using other compositions of the present invention (for example, the composition in Table 5) can also obtain similar results.

In another experiment, not according to the claimed invention, the successfully modeled test animals (nude mice bearing HepG2, with an average tumor volume of 161mm$^3$) were randomly divided into a blank control group and 5 study groups (groups A, B, C, D, and E). The negative control group was given normal saline, and the compositions and injection methods of the 5 study drugs are shown in the table below. The drugs were all aqueous solutions, which were prepared according to the preparation method of Example 1. Each group was administered once, with an injection dose of ≤130μl/mouse. On the next day after the administration was ended, the animals were euthanized, and the tumor was dissected for histological observation. The intratumoral tissue destruction was distinguished by denoting 0 for the blank control group and denoting 5 for the 5% acetic acid group. The results are shown in Table 7.

Table 7

| Experimental group | Study drug | Injection manner | Intratumoral tissue destruction |
|---|---|---|---|
| A | 10% glutathione/30% glucose | Intraperitoneal injection | 0 |
| B | 10% glutathione/30% glucose | Intratumoral injection | 3 |
| C | 10% glutathione | Intratumoral injection | 1 |
| D | 30% glucose | Intratumoral injection | 1 |
| E | 5% acetic acid | Intratumoral injection | 5 |
| Blank control group | - | - | 0 |

The results in the above table further confirmed the synergistic destructive effect of the composition group during intratumoral injection (group B).

2. Amino acid based nutrients/anti-tumor cell drug composition not according to the claimed invention

In this experiment, the experimental animals (nude mice bearing HepG2, with an average tumor volume of 175 mm$^3$) successfully modeled were randomly divided into 2 negative control groups and 14 study groups. The negative control groups were given normal saline, and 7 study drugs were as shown in the table below. They were injected intraperitoneally and intratumorally. The drugs were all aqueous solutions, which were prepared according to the preparation method of Example 1. Each group was administered once every 3 days for a total of 3 times, each time at an injection dose of $\leq 150\mu$l/mouse. On the 5th day after the administration was ended, the animals were euthanized, and the tumor weight was measured after anatomy to the animals. The tumor inhibition rate was calculated from the negative control group of each administration mode. The results are shown in Table 8.

Table 8

| Group | Study drug | Tumor inhibition rate of intraperitoneal injection | Intratumoral injection | |
|---|---|---|---|---|
| | | | Tumor weight (g) | Tumor inhibition rate |
| 0 | Normal saline | (0) | 2.54±0.36g | (0) |
| 1 | 1% 5-fluorouracil | 58% | 0.84±0.25g | 67% |
| 2 | 20% arginine | 5% | 0.99±0.21g | 61% |
| 3 | 20% glycine | 2% | 1.17±0.28g | 54% |
| 4 | 10% glutathione | 1% | 1.04±0.23g | 59% |
| 5 | 1% 5-fluorouracil/20% arginine | 57% | 0.36±0.16g | 86% |
| 6 | 1% 5-fluorouracil/20% glycine | 55% | 0.48±0.17g | 81% |
| 7 | 1% 5-fluorouracil/10% glutathione | 59% | 0.43±0.13g | 83% |

In the above table, the tumor inhibition rate of the intratumoral injection group of each composition was greater than any of its ingredient groups ($E_{A+B} > E_A$ and $E_{A+B} > E_B$), and had the statistically significant difference in tumor weight from the ingredient group ($p < 0.05$, $p < 0.05$), so these composition groups all showed synergistic efficacy: 1% 5-fluorouracil/20% arginine (greater tumor inhibition rate, and tumor weight difference from the 1% 5-fluorouracil group and 20% arginine group was $P < 0.05$), 1% 5-fluorouracil/20% glycine (greater tumor inhibition rate, and tumor weight difference from the 1% 5-fluorouracil group and 20% glycine group was $P < 0.05$), 1% 5-fluorouracil/10% glutathione (relatively high tumor inhibition rate, and tumor weight difference from the 1% 5-fluorouracil group and 10% glutathione was $P < 0.05$).

In another experiment, not according to the claimed invention, the successfully modeled test animals (nude mice bearing HepG2, with an average tumor volume of 171mm$^3$) were randomly divided into a blank control group and 5 study groups (groups A, B, C, D, and E). The negative control groups were given normal saline, and the compositions and injection methods of the 5 study drugs are shown in the table below. The drugs were all aqueous solutions, which were prepared according to the preparation method of Example 1. Each group was administered once, with an injection dose of $\leq 130\mu$l/mouse. On the next day after the administration was ended, the animals were euthanized, and the tumor was dissected for histological observation. The intratumoral tissue destruction was distinguished by denoting 0 for the blank control group and denoting 5 for the 5% acetic acid group. The results are shown in Table 9.

Table 9

| Experimental group | Study drug | Injection manner | Intratumoral tissue destruction |
|---|---|---|---|
| A | 1% 5-fluorouracil/20% arginine | Intraperitoneal injection | 0 |
| B | 1% 5-fluorouracil/20% arginine | Intratumoral injection | 3 |
| C | 20% arginine | Intratumoral injection | 1 |
| D | 1% 5-fluorouracil | Intratumoral injection | 1 |
| E | 5% acetic acid | Intratumoral injection | 5 |
| Blank control group | - | - | 0 |

The results in the above table further confirmed the synergistic destructive effect of the composition group during intratumoral injection (group B).

3. Mouse test of the synergistic pharmacology of the composition

The following experiment employed mice as experimental animals instead of nude mice. The former has a normal immune system and is therefore closer to clinical patients.

In one experiment, the test animal was a BALB/c mouse, the modeling cells were breast cancer 4T1 cells, and the transplantation tumor was modeled at $1\times10^6$ cells/mouse under the skin of the animal's right axilla. The experimental animals (with an average tumor volume of 336mm$^3$) successfully modeled were randomly divided into 14 experimental groups (as shown in the table below). The experimental groups were divided into 1 negative control group (group 0) and 13 study groups (groups 1-13). The negative control group was given normal saline, and the study drugs were as shown in the table below. They were all injected intratumorally. The drugs were all aqueous solutions, which were prepared according to the preparation method of Example 1. Each group was administered once every 3 days for a total of 3 times, each time at an injection dose of 150µl//mouse. During the administration, general state of the animals and the degree of necrosis of normal tissues around the tumor after the administration were observed. On the 5th day after the administration was ended, the animals were euthanized, and the tumor weight was measured after anatomy to the animals. The tumor inhibition rate was calculated from the negative control group of each administration mode. The results are shown in Table 10.

Table 10

| Group | Study drug | Intratumoral injection | |
|---|---|---|---|
| | | Tumor weight (g) | Tumor inhibition rate |
| 0 | Normal saline | 1.28±0.31g | (0) |
| 1 | 1% 5-fluorouracil | 0.63±0.21g | 51% |
| 2 | 20% arginine | 0.86±0.23g | 33% |
| 3 | 20% arginine/1% 5-fluorouracil | 0.19±0.18g | 85% |
| 4 | 15% arginine | 0.92±0.26g | 28% |
| 5 | 4% sodium bicarbonate/7% sodium carbonate | 0.32±0.20g | 75% |
| 6 | 7% sodium bicarbonate/3% NaOH | 0.41±0.21g | 68% |
| 7 | 15% arginine/4% sodium bicarbonate/7% sodium carbonate | 0.14±0.13g | 89% |
| 8 | 15% arginine/7% sodium bicarbonate/3% NaOH | 0.17±0.12g | 87% |
| 9 | 10% acetic acid | 0.60±0.21g | 53% |
| 10 | 20% glycine | 1.05±0.32g | 18% |
| 11 | 20% glycine/10% acetic acid | 0.32±0.18g | 75% |
| 12 | 1% NaOH | 0.76±0.24g | 41% |

(continued)

| Group | Study drug | Intratumoral injection | |
|---|---|---|---|
| | | Tumor weight (g) | Tumor inhibition rate |
| 13 | 20% arginine/1% NaOH | 0.28±0.16g | 78% |

In the above table, the q of the intratumoral injection group of each composition showed a synergistic effect (q>1.00), and the tumor weight difference between the composition intratumoral injection group and the ingredient group was statistically significant (p<0.05, p<0.05), Therefore, these composition groups all showed significant synergistic effects: 20% arginine/1% 5-fluorouracil group (q=1.26>1.00, and the tumor weight difference from the 20% arginine group and 1% 5-fluorouracil group was P<0.05), 15% arginine /4% sodium bicarbonate/7% sodium carbonate (q=1.09>1.00, and the tumor weight difference from the 15% arginine group and 4% sodium bicarbonate/7% sodium carbonate group was P<0.05), 15 %Arginine/7% sodium bicarbonate/3%NaOH (q=1.13>1.00, and the tumor weight difference from the 15% arginine group and 7% sodium bicarbonate/3% NaOH group was P<0.05), 20% glycine/10% acetic acid (q=1.22>1.00, and the tumor weight difference from the 20% glycine group and 10% acetic acid group was P<0.05), 20% arginine/1% NaOH (q=1.29>1.00, and the tumor weight difference from the 20% arginine group and 1% NaOH group was P<0.05).

According to the above studies and more similar studies, the following conclusions can be drawn: under necessary conditions for amino acid based nutrients to show local activity (local administration + local concentration threshold), the pharmacology of the amino acid based nutrients to target and destroy tumor tissues may produce a local synergistic effect with other active ingredients (conventional ineffective compounds and/or anti-tumor chemotherapy agents). The following examples further study the technical solution of the synergy.

Example 5: Further research on synergistic technical solutions

In fact, a probability of synergy between amino acid based nutrients and other active ingredients does not mean necessity of such synergy. The following experiments study different effects (synergism, addition, antagonism) generated byco-use under different conditions.

1. Amino acid based nutrients/conventional ineffective compound composition

In this experiment, the experimental animals (nude mice bearing HepG2, with an average tumor volume of 164 mm$^3$) successfully modeled were randomly divided into a negative control group and 46 study groups. The negative control group was given normal saline. The compositions of the study drug are as shown in the table below: 4 types and concentrations of amino acid based nutrient single drugs (X% amino acid based nutrients), 18 types and concentrations of ineffective absorption drugs (Y% ineffective absorption drugs), 24 compositions comprising various types and concentrations of amino acid based nutrients and ineffectively absorption drugs (X% amino acid based nutrients/Y% ineffectively absorption drugs). The drugs were all aqueous solutions, which were prepared according to the preparation method of Example 1. All groups were injected intratumorally, once every 3 days for a total of 3 times. The dosage of each administration was: glutathione ≤1000 mg/kg, arginine ≤1500 mg/kg, hydrochloric acid ≤50 mg/kg, acetic acid ≤375 mg/kg, methylene blue ≤100 mg/kg, quinine dihydrochloride ≤250 mg/kg, glucose ≤2250 mg/kg, injection volume≤150μl. On the fifth day after the administration was ended, the animals were euthanized, the tumor weight was measured after anatomy, and the tumor inhibition rate was calculated from the negative control group. The tumor inhibition rate of each study group is shown in Table 11.

Table 11

| Tumor inhibition rate of the composition of X% amino acid based nutrient/ Y% ineffective absorption drug | | | | | Tumor inhibition rate of Y% conventional ineffective compound |
|---|---|---|---|---|---|
| Tumor inhibition rate of X% amino acid based nutrient | | | | Y% convention ineffective compound | |
| 2.5% glutathione | 5% glutathione | 10% arginine | 20% arginine | | |
| (12%)* | (31%) | (43%) | (60%) | Y=0 | - |
| 93%*** | | | | 20% acetic acid | (92%)** |
| | 85% | 83% | | 5% acetic acid | (71%) |

(continued)

| Tumor inhibition rate of the composition of X% amino acid based nutrient/ Y% ineffective absorption drug | | | | | Tumor inhibition rate of Y% conventional ineffective compound |
|---|---|---|---|---|---|
| Tumor inhibition rate of X% amino acid based nutrient | | | | Y% convention ineffective compound | |
| 2.5% glutathione | 5% glutathione | 10% arginine | 20% arginine | | |
| | | | 72% | 2% acetic acid | (46%) |
| | | | 61% | 1% acetic acid | (26%) |
| 85% | | | | 2% hydrochloric acid | (83%) |
| | 79% | 77% | | 1% hydrochloric acid | (68%) |
| | | | 71% | 0.5% hydrochloric acid | (53%) |
| | | | 62% | 0.25%hydrochlori c acid | (31%) |
| 64% | 76% | | | 10% Bengal Red | (63%) |
| | | 73% | | 5% Bengal Red | (46%) |
| | | | 68% | 0.35% methylene blue | (18%) |
| | | | 61% | 0.25% methylene blue | (11%) |
| 37% | 79% | | | 40% glucose | (36%) |
| | | 71% | | 30% glucose | (34%) |
| | | | 78% | 20% glucose | (18%) |
| | | | 60% | 5% glucose | (21%) |
| 29% | 52% | | 74% | 5% DHA | (28%) |
| | | | 71% | 3% DHA | (21%) |
| *: The data in the brackets in the rows refers to the average tumor inhibition rate of the X% amino acid based nutrient group. For example, the average tumor inhibition rate of the 2.5% glutathione group was 12% <br> **: The data in the brackets in the columns refers to the average tumor inhibition rate of the Y% conventional ineffective compound group. For example, the average tumor inhibition rate of the 20% acetic acid group was 92% <br> ***: The data without brackets refers to the average tumor inhibition rate of the X% amino acid based nutrient/Y% conventional ineffective compound group. For example, the average tumor inhibition rate of the 2.5% glutathione/20% acetic acid group was 93%. | | | | | |

In the above table, there is no statistically significant difference in tumor weight between the following composition groups and at least one ingredient group (p>0.05), so the efficacy shown by these composition groups is at most addition: 2.5% glutathione/20% acetic acid, 20% arginine/1% acetic acid, 2.5% glutathione/2% hydrochloric acid, 20% arginine/0.25% hydrochloric acid, 2.5% glutathione/10 % Bengal Red, 2.5% glutathione/40% glucose, 20% arginine/5% glucose, 2.5% glutathione/5% DHA.

In the above table, the tumor inhibition rate of each composition intratumoral injection group was greater than any of its ingredient groups ($E_{A+B} > E_A$ and $E_{A+B} > E_B$), and had statistically significant difference in tumor weight from the ingredient group (p<0.05, p<0.05), so these composition groups all showed synergistic efficacy: 20% arginine/2% acetic acid, 10% arginine/1% hydrochloric acid, 20% arginine/0.5% hydrochloric acid.

In the above table, q of the following composition groups was >1.00, and the tumor weight difference from the ingredient group was statistically significant (p<0.05, p<0.05), so these composition groups showed significant synergistic efficacy: 5% glutathione/5% acetic acid (q=1.06), 5% glutathione/1% hydrochloric acid (q=1.01), 5% glutathione/10% Bengal Red (q=1.02), 5% glutathione/40% glucose (q=1.42), 5% glutathione/5% DHA (q=1.03), 10% arginine/5% Bengal Red (q=1.06), 20% arginine/0.35% methylene blue (q=1.041, 10% arginine/30% glucose (q=1.14), 20% arginine/20% glucose (q=1.15), 20% arginine/ 5% DHA (q=1.04), 20% arginine/3% DHA (q=1.04).

In another experiment, the test animal was a BALB/c mouse, the modeling cells were breast cancer 4T1 cells, and the transplantation tumor was modeled at $1\times10^6$ cells/mouse under the skin of the animal's right axilla. Successfully modeled experimental animals (with an average tumor volume of 317 mm$^3$) were randomly divided into 1 negative control group (group 0) and 9 drug study groups (1-9). The negative control was normal saline, and the study drugs are shown in the table below. The drugs were all aqueous solutions, which were prepared according to the preparation method of Example 1. Each group was administered by intratumoral injection once every 3 days for a total of 3 times, each time at an injection dose of 150 $\mu$l//mouse. On the 5th day after the administration was ended, the animals were euthanized, and the tumor weight was measured after anatomy to the animals. The tumor inhibition rate was calculated from the negative control group. The results are shown in Table 12.

Table 12

| Group | Study drug | Tumor weight ($x\pm s$) (g) | Tumor inhibition rate |
|---|---|---|---|
| 0 | Normal saline | 2.36$\pm$0.35 | 0 |
| 1 | 7% NaOH | 0.59 | 75% |
| 2 | 1% NaOH | 1.39 | 41% |
| 3 | 0.5% NaOH | 1.54 | 31% |
| 4 | 20% arginine | 1.72 | 27% |
| 5 | 10% arginine | 1.91 | 19% |
| 6 | 4% arginine | 2.31 | 2% |
| 7 | 4% arginine/7% NaOH | 0.57 | 76% |
| 8 | 20% arginine/1% NaOH | 0.64 | 73% |
| 9 | 10% arginine/0.5% NaOH | 0.99 | 58% |

In the above table, among study groups 7, 1 and 6, the tumor inhibition rate of the composition group 7 and the study group 1 was not much different, and there was no significant difference in the remaining tumor weight from the two groups ($p>0.05$), showing no synergistic effect. Among the study groups 8, 2 and 4, q of the composition group 8 was 1.14>1.00, and the difference in the remaining tumor weight respectively from groups 2 and 4 was statistically significant (all $p<0.05$), showing obvious synergistic efficacy. Among the study groups 9, 3 and 5, q of the composition group 9 was 1.31>1.00, and the difference in the remaining tumor weight respectively from groups 3 and 5 was statistically significant (all $p<0.05$), showing obvious synergistic efficacy.

In another experiment, the test animal was BALB/c mice, the modeling cells were breast cancer 4T1 cells, and the transplantation tumor was modeled at $1\times10^6$ cells/mouse under the skin of the animal's right axilla. Successfully modeled experimental animals (average tumor volume 307mm$^3$) were randomly divided into 1 negative control group (group 0) and 13 drug study groups (1-13). The negative control was normal saline, and the study drugs are shown in the table below. The drugs were all aqueous solutions, which were prepared according to the preparation method of Example 1. Each group was administered by intratumoral injection once every 3 days for a total of 3 times, each time at an injection dose of 150 $\mu$l//mouse. On the 5th day after the administration was ended, the animals were euthanized, and the tumor weight was measured after anatomy to the animals. The tumor inhibition rate was calculated from the negative control group. The results are shown in Table 13.

Table 13

| Group | Drug | | Tumor weight | Tumor |
|---|---|---|---|---|
| | Composition | pH | ($x\pm s$) (g) | inhibition rate |
| 0 | Normal saline | - | 2.26$\pm$0.37 | 0 |
| 1 | 14% sodium carbonate | 10.8 | 1.47$\pm$0.24 | 35% |
| 2 | 7% sodium carbonate | 10.8 | 1.81$\pm$0.21 | 20% |
| 3 | 2.8% sodium carbonate | 10.9 | 2.10$\pm$0.27 | 7% |
| 4 | 10% sodium bicarbonate | 6.8 | 1.58$\pm$0.22 | 30% |
| 5 | 5% sodium bicarbonate | 6.9 | 1.79$\pm$0.24 | 21% |
| 6 | 2% sodium bicarbonate | 6.9 | 2.10$\pm$0.25 | 7% |
| 7 | 2% sodium bicarbonate/ 14% sodium carbonate | 9.9 | 1.45$\pm$0.24 | 36% |
| 8 | 10% sodium bicarbonate/ 14% sodium carbonate | 9.2 | 0.50$\pm$0.13 | 78% |

(continued)

| Group | Drug | | pH | Tumor weight (x±s) (g) | Tumor inhibition rate |
|---|---|---|---|---|---|
| | Composition | | | | |
| 9 | 5% sodium bicarbonate/ 7% sodium carbonate | | 9.2 | 0.63±0.12 | 72% |
| 10 | 2% sodium bicarbonate/ 2.8% sodium carbonate | | 9.3 | 1.29±0.21 | 43% |
| 11 | 5% sodium bicarbonate/ 2.8% sodium carbonate | | 8.6 | 1.76±0.23 | 22% |
| 12 | 15% arginine | | 11.0 | 1.74±0.28 | 23% |
| 13 | 15% arginine/5% sodium bicarbonate/7% sodium carbonate | | 9.8 | 0.20±0.11 | 91% |

In the above table, among study groups 7, 1 and 6, the tumor inhibition rate of the composition group 7 and the single drug group 1 was not much different, and there was no significant difference in the remaining tumor weight between the two groups ($p>0.05$), showing no synergistic effect. Among the study groups 8, 1 and 4, q of the composition group 8 was 1.43>1.00, and the difference in the remaining tumor weight respectively from groups 1 and 4 was statistically significant (all $p<0.05$), showing obvious synergistic efficacy. Among the study groups 9, 2 and 5, q of the composition group 9 was 1.43>1.00, and the difference in the remaining tumor weight respectively from groups 2 and 5 was statistically significant (all $p<0.05$), showing obvious synergistic efficacy. Similarly, the composition group 10 also showed obvious synergistic efficacy, but its tumor inhibition rate was only 60% of the composition group 9. Among study groups 11, 3 and 5, the tumor inhibition rate of the composition group 11 and the single drug group 5 was not much different, and there was no significant difference in the remaining tumor weight between the two groups ($p>0.05$), showing no synergistic effect.

In addition, among the study groups 13, 12 and 9, q of the composition group 13 was 1.16>1.00, and the difference in the remaining tumor weight respectively from groups 12 and 9 was statistically significant (all $p<0.05$), showing obvious synergistic efficacy. This result once again shows that under the condition of little change in pH, the addition of synergists can change the properties of the composition (maybe, for example, increase the buffer capacity) to make the composition show synergistic efficacy.

In another experiment, the test animal was a BALB/c mouse, the modeling cells were breast cancer 4T1 cells, and the transplantation tumor was modeled at $1\times10^6$ cells/mouse under the skin of the animal's right axilla. Successfully modeled experimental animals (average tumor volume 325mm$^3$) were randomly divided into 1 negative control group (group 0) and 5 drug study groups (1-5). The negative control was normal saline, and the study drugs are shown in the table below. The drugs were all aqueous solutions, which were prepared according to the preparation method of Example 1. Each group was administered by intratumoral injection once every 3 days for a total of 3 times, each time at an injection dose of 150 $\mu$l//mouse. On the 5th day after the administration was ended, the animals were euthanized, and the tumor weight was measured after anatomy to the animals. The tumor inhibition rate was calculated from the negative control group. The results are shown in Table 14.

Table 14

| Gro up | Drug | | Tumor weight (x±s) (g) | Tumor inhibition rate |
|---|---|---|---|---|
| | Composition | pH | | |
| 0 | Normal saline | - | 2.43±0.37 | 0 |
| 1 | 1% NaOH | 11.8 | 1.41±0.23 | 42% |
| 2 | 2% KCl | - | 2.19±0.31 | 11% |
| 3 | 2% KCl/1% NaOH | 11.0 | 0.85±0.16 | 65% |
| 4 | 20% arginine | 10.8 | 1.75±0.29 | 28% |
| 5 | 20% arginine/2% KCl/1% NaOH | 10.9 | 0.46±0.11 | 81% |

In the above table, among the study groups 3 1, and 2, q of the composition group 3 was 1.34>1.00, and the difference in the remaining tumor weight respectively from groups 1 and 2 was statistically significant (all $p<0.05$), showing obvious synergistic efficacy. Among the study groups 5, 3 and 4, q of the composition group 5 was 1.08>1.00, and the difference in the remaining tumor weight respectively from groups 3 and 4 was statistically significant (all $p<0.05$), showing obvious synergistic efficacy.

In another experiment, the test animal was a BALB/c mouse, the modeling cells were breast cancer 4T1 cells, and the transplantation tumor was modeled at $1\times10^6$ cells/mouse under the skin of the animal's right axilla. Successfully modeled experimental animals (with an average tumor volume of 316 mm$^3$) were divided into 1 negative control group (group 0) and 5 drug study groups (1-5). The negative control was normal saline, and the study drugs are shown in the table below. The drugs were all aqueous solutions, which were prepared according to the preparation method of Example 1. Each group was administered by intratumoral injection once every 3 days for a total of 3 times, each time at an injection dose of 150 µl/mouse. On the 5th day after the administration was ended, the animals were euthanized, and the tumor weight was measured after anatomy to the animals. The tumor inhibition rate was calculated from the negative control group. The results are shown in Table 15.

Table 15

| Group | Drug | | Tumor weight (x±s) (g) | Tumor inhibition rate |
|---|---|---|---|---|
| | Composition | pH | | |
| 0 | Normal saline | - | 2.18±0.32 | 0 |
| 1 | 15% acetic acid | 2.5 | 0.70±0.18 | 68% |
| 2 | 4% sodium bicarbonate | 6.9 | 1.68±0.27 | 23% |
| 3 | 4% sodium bicarbonate/15% acetic acid | 3.6 | 0.48±0.14 | 78% |
| 4 | 15% glycine | 6.8 | 1.81±0.26 | 17% |
| 5 | 15% glycine/4% sodium bicarbonate/ 15% acetic acid | 4.0 | 0.31±0.12 | 86% |

In the above table, among the study groups 3, 1 and 2, q of the composition group 3 was 1.04>1.00, and the difference in the remaining tumor weight respectively from groups 2 and 1 was statistically significant (all $p<0.05$), showing obvious synergistic efficacy. In addition, among the study groups 5, 3 and 4, q of the composition group 5 was 1.05>1.00, and the difference in the remaining tumor weight respectively from groups 3 and 4 was statistically significant (all $p<0.05$), showing obvious synergistic efficacy. This result once again shows that under the condition of little change in pH, the addition of synergists can change the properties of the composition (maybe, for example, increase the buffer capacity) to make the composition show synergistic efficacy.

In addition, according to the above studies and other similar studies, most of the pharmaceutical composition of the present invention containing an amino acid based nutrient, an acidulant or/and alkalizer had pH buffering capacity, with a buffering capacity of usually >0.01mol·L$^{-1}$ ·pH$^{-1}$, preferably 0.04-0.45mol·L$^{-1}$ ·pH$^{-1}$, more preferably 0.05-0.45mol·L$^{-1}$ ·pH$^{-1}$.

2. Amino acid based nutrients/anti-tumor cell pharmaceutical composition

In this experiment, not according to the claimed invention, the experimental animals (nude mice bearing HepG2, with an average tumor volume of 169mm$^3$) successfully modeled were randomly divided into a negative control group and 16 study groups. The negative control was normal saline. The compositions of 16 study drugs, as shown in the table below, comprise: a 5-fluorouracil single drug of 4 varying concentrations (X% 5-fluorouracil), and an amino acid based nutrient with 5 varying concentrations (Y% amino acid based nutrient), 7 compositions consisting of 5-fluorouracil of varying concentrations and an amino acid based nutrient of varying concentrations (X% 5-fluorouracil/Y% amino acid based nutrient). The drugs were all aqueous solutions, which were prepared according to the preparation method of Example 1. The saturated concentration (C$_{anti}$) of 5-fluorouracil used was about 1.5%. Each group was injected intratumorally, once every 3 days for a total of 3 times. The dose at each time was: 5-fluorouracil 50 mg/kg, glutathione ≤1 g/kg, and an injection volume was ≤ 150 µl/mouse. On the 5th day after the administration was ended, the animals were euthanized, and the tumor weight was measured after anatomy to the animals. The tumor inhibition rate was calculated from the negative control group. The results are shown in Table 16.

Table 16

| Tumor inhibition rate of X% 5-fluorouracil/Y% amino acid based nutrient composition | | | | | Tumor inhibition rate of Y% amino acid based nutrient |
|---|---|---|---|---|---|
| Tumor inhibition rate of X% 5-fluorouracil | | | | Y% amino acid based nutrient | |
| 0.5% 5-fluorouracil | 0.75% 5-fluorouracil | 1% 5-fluorouracil | 1.5% 5-fluorouracil | | |
| (59%)* | (63%) | (66%) | (69%) | Y=0 | - |

(continued)

| Tumor inhibition rate of X% 5-fluorouracil/Y% amino acid based nutrient composition | | | | | Tumor inhibition rate of Y% amino acid based nutrient |
| --- | --- | --- | --- | --- | --- |
| Tumor inhibition rate of X% 5-fluorouracil | | | | Y% amino acid based nutrient | |
| 0.5% 5-fluorourac il | 0.75% 5-fluorourac il | 1% 5-fluorourac il | 1.5% 5-fluorourac il | | |
| 63%*** | 81% | | | 20% arginine | (61%)** |
| | 76% | 82% | | 10% glutathione | (62%) |
| | | | 79% | 5% glutathione | (32%) |
| | | 92% | | 3% glutamic hydrochlorid e | (70%) |
| | | | 70% | 2.5% glutathione | (12%) |
| *: The data in the brackets in the rows refers to the average tumor inhibition rate of the X% 5-fluorouracil group. For example, the average tumor inhibition rate of the 0.5% 5-fluorouracil group was 59%. **: The data in the brackets in the columns refers to the average tumor inhibition rate of the Y% amino acid based nutrient group. For example, the average tumor inhibition rate of the 20% arginine group was 61%. ***: The data without brackets refers to the average tumor inhibition rate of the X%5-fluorouracil/Y% amino acid based nutrient group. For example, the average tumor inhibition rate of the 0.5%5-fluorouracil/20% arginine group was 63%. | | | | | |

In the above table, the tumor inhibition rate of the following composition groups was greater than any of their component groups ($E_{A+B} > E_A$ and $E_{A+B} > E_B$), and had the statistically significant difference in tumor weight from the component group ($p<0.05$, $p<0.05$), so these composition groups all showed synergistic efficacy: 0.75% 5-fluorouracil/20% arginine, 0.75% 5-fluorouracil/10% glutathione, 1% 5-fluorouracil/10% glutathione, 1% 5-fluorouracil/3% glutamate.

In the above table, there is nearly no difference in tumor weight between the following composition groups and at least one ingredient group, so the efficacy shown by these composition groups is at most addition: 0.5% 5-fluorouracil/20% arginine, 1.5% 5-fluorouracil/2.5% glutathione.

In another experiment, the test animal was a BALB/c mouse, the modeling cells were breast cancer 4T1 cells, and the transplantation tumor under the skin of the animal's right axilla was modeled at $1\times10^6$ cells/mouse. Successfully modeled experimental animals (with an average tumor volume of 317 mm$^3$) were randomly divided into a negative control group and 16 study groups. The negative control was normal saline, and the compositions of the study drugs are shown in the table below. The drugs were all aqueous solutions, which were prepared according to the preparation method of Example 1. Each group was injected intratumorally, once every 3 days, for a total of 3 times, each time with a dosage of 150µl/mouse. On the fifth day after the administration was ended, the animals were euthanized, the tumor weight was measured after anatomy, and the tumor inhibition rate was calculated from the negative control group. The tumor inhibition rate of each study drug group is shown in Table 17.

Table 17

| Group | Study drug | Tumor weight | Tumor inhibitio n rate | Group | Study drug | Tumor weight | Tumor inhibition rate |
| --- | --- | --- | --- | --- | --- | --- | --- |
| 0 | Normal saline | 1.56±0.3 4g | (0) | 1 | 1%5-Fu | 0.73±0.21 g | 53% |
| 2 | 20% arginine | 1.03±0.2 9g | 34% | 9 | 20% glycine/ 2.5% acetic acid | 0.98±0.23 g | 37% |
| 3 | 10% arginine | 1.23±0.31g | 21% | 10 | 20% glycine/ 5% acetic acid | 0.45±0.13 g | 71% |
| 4 | 5% arginine | 1.40±0.2 6g | 10% | 11 | 20% glycine/ 15% acetic acid | 0.33±0.10 g | 79% |
| 5 | 2.5% arginine | 1.48±0.3 2g | 5% | 12 | 20% glycine/ 25% acetic acid | 0.31±0.15 g | 80% |

(continued)

| Group | Study drug | Tumor weight | Tumor inhibitio n rate | Group | Study drug | Tumor weight | Tumor inhibition rate |
|---|---|---|---|---|---|---|---|
| 6 | 20% argi-nine/1% 5-Fu | 0.20±0.1 2g | 87% | 13 | 20% glycine/ 2.5% acetic acid/1% 5-Fu | 0.67±0.21 g | 57% |
| 7 | 10% arginine/ 1% 5-Fu | 0.48±0.1 4g | 69% | 14 | 20% glycine/ 5% acetic acid/1% 5-Fu | 0.19±0.14 g | 88% |
| 8 | 5% arginine/ 1% 5-Fu | 0.64±0.1 8g | 59% | 15 | 20% glycine/ 15% acetic acid/1% 5-Fu | 0.11±0.09 g | 93% |
| 9 | 2.5% arginine/ 1% 5-Fu | 0.72±0.2 1g | 54% | 16 | 20% glycine/ 25% acetic acid/1% 5-Fu | 0.27±0.27 g | 83% |

In the above table, q of each composition group was >1.00, showing a synergistic effect, and the tumor weight difference between the composition group and the single ingredient group was statistically significant (p<0.05, p<0.05), Therefore, these composition groups all showed obvious synergistic effects: 20% arginine/1% 5-Fu (q=1.26>1.00), 10% arginine/1% 5-Fu (q=1.10>1.00), 20% glycine/5% acetic acid/1%5- Fu (q=1.02>1.00, and the tumor weight difference from the 1% 5-Fu group and the 20% glycine/5% acetic acid group was p<0.05), 20% glycine/15% acetic acid/1% 5-Fu (q=1.03>1.00, and the tumor weight difference from the 1% 5-Fu group and the 20% glycine/15% acetic acid group was p<0.05). In the above table, q of the 5% arginine/1% 5-Fu group was 1.02>1.00, showing a synergistic effect, but the tumor weight difference between this group and the 1% 5-Fu group was not statistically significant (P >0.05), so that the study group of the composition showed a synergistic effect but the synergy was not obvious.

In the above table, q of the following composition groups was <1.00, showing an antagonistic efficacy, and there was no statistically significant difference in tumor weight from a certain component group (p>0.05), so these composition groups showed antagonism efficacy but the antagonism was not obvious: 2.5% arginine/1% 5-Fu (q=0.98<1.00, and the tumor weight difference from the 1% 5-Fu group was p>0.05), 20% glycine/2.5% acetic acid/1% 5- Fu (q=0.81<1.00, and the tumor weight difference from the 1%5-Fu group was p>0.05), 20% glycine/25% acetic acid/1%5-Fu (q=0.99<1.00, and the tumor weight difference from the 20% glycine/25% acetic acid group was p>0.05).

According to the above study and more similar studies, the synergistic technical solution of the composition, not according to the claimed invention, is:

1. Local administration at a lesion tissue;
2. Local administration with a synergistic composition, wherein:

1). the concentration of the amino acid based nutrient is >2.5%, and preferably is 5-25% or 3-25%, where: when the amino acid based nutrient includes an acidic amino acid salt, the local administration concentration of the amino acid based nutrient is 3%-25%; when the amino acid based nutrient is one or more selected from an amino acid or/and an amino acid salt other than the amino acid salt, the local administration concentration of the amino acid based nutrient is 10%-25%, preferably 15%-25% or 20%-25%; when the amino acid based nutrient is selected from one or more oligopeptides, the local administration concentration of the amino acid based nutrient is 5%-25%, preferably 7.5%-25%; and when the amino acid based nutrient includes one or more of the oligopeptide, the amino acids or/and the amino acid salts other than the acidic amino acid salts, the local administration concentration of the amino acid based nutrients is 5%-25%, preferably 7.5-25% or 10-25%;
2). the concentration of the anti-tumor chemotherapeutic agent is greater than 30% of its saturated concentration, preferably 50%-100% of its saturated concentration; or/and
3). the concentration of the conventional ineffective compound is > 0.25%, preferably 0.35-50%, where: in the conventional ineffective compound, the local administration concentration of the other nutrients is greater than 2%, preferably 3%-40%; the local administration concentration of the ineffective aromatic compound is greater than 0.25%, preferably 0.35%-10%; or/and the local administration concentration of the acidulant is greater than 0.25%, preferably 0.75%-15%.

In the other nutrients, the local administration concentration of the lipid nutrient is greater than 2%, preferably 3%-25%; or/and the local administration concentration of the carbohydrate nutrient is greater than 10%, preferably 15%-40%.

In the ineffective aromatic compound, the local administration concentration of the methylene blue dye is greater than 0.25% and less than or equal to 2%, preferably 0.35%-2%; the local administration concentration of the other ineffective aromatic compound (such as another vital dye, a salicylic acid compound and a quinine compound) is greater than 1%, preferably 3%-10%.

In the acidulant, the local administration concentration of the strong acid is greater than 0.25% and less than 2%, preferably 0.75%-1.5%; the local administration concentration of the weak acid is greater than 1.5% and less than 20%, preferably 3.5%-15%; or/and the local administration concentration of the medium strong acid is greater than 0.5% and less than 5%, preferably 1%-5%.

In the alkalizer, the local administration concentration of the strong base is greater than 0.5% and less than 10%, preferably 0.75%-7%; and the local administration concentration of the weak base is greater than 1.5% and less than 35 %, preferably 2.5%-15%.

By using any of the above synergy technical solutions, the synergistic effect of the composition of the present invention comprising the amino acid based nutrient and the conventional ineffective compound or/and the anti-tumor chemotherapeutic agent indicates neither the inhibitory effect on cancer cells, nor the indiscriminate tissue destruction effect, but the effect of individual components in synergistically increasing the efficacy and reducing the local damage. In other words, the composition of the present invention significantly improves the specificity of a local action.

Example 6: Preferred amino acid based nutrients

In this experiment, not according to the claimed invention, the experimental animals (nude mice bearing HepG2, with an average tumor volume of $186mm^3$) successfully modeled were randomly divided into a negative control group, a positive control group and 10 study groups. The negative control was normal saline, the positive control was 1% 5-fluorouracil, and the study drug was a 10% amino acid based nutrient/5% acetic acid composition (the type of the amino acid based nutrient is variable). The amino acid based nutrients in the compositions used in the study groups 1-9 were: glycine, cysteine hydrochloride, lysine hydrochloride, proline, valine, alanine, glutathione, serine, ALA-GLN, and the study drug used in the study group 10 was 10% arginine/10% glycine/5% acetic acid. The drugs were all aqueous solutions, which were prepared according to the preparation method of Example 1. Each group was injected intratumorally, once every 3 days, for a total of 3 times, with an injection volume of ≤150μl. On the fifth day after the administration was ended, the animals were euthanized, the tumor weight was measured after anatomy, and the tumor inhibition rate was calculated from the negative control group. The tumor inhibition rate of the positive control group was 61%, and the results of the study groups are shown in Table 18.

Table 18

| Group | 1 | 2 | 3 | 4 | 5 | 6 | 7 | 8 | 9 | 10 |
|---|---|---|---|---|---|---|---|---|---|---|
| Y(%) | 92 | 86 | 85 | 49 | 57 | 68 | 88 | 79 | 83 | 86 |

Based on these results and more similar studies, a super-effective and synergistic technical solution of the amino acid based nutrient/acidulant composition of the present invention is: the amino acid based nutrient is preferably selected from the following amino acids and one or more of amino acid derivatives containing the following amino acids: arginine, lysine, cysteine, alanine, serine, glycine, glutamic acid.

In another experiment, not according to the claimed invention, the experimental animals (nude mice bearing HepG2, with an average tumor volume of $172mm^3$) successfully modeled were randomly divided into a negative control group, a positive control group and 14 study groups. The negative control was normal saline, the positive control was 1% 5-fluorouracil, and the study drug was 1% methylene blue/10% amino acid based nutrient composition (the type of the amino acid based nutrient is variable). The amino acid based nutrients in the compositions used in study groups 1-13 were: arginine, glycine, cysteine hydrochloride, valine, threonine, proline, histidine hydrochloride, phenylalanine, lysine, leucine, alanine, glutathione, serine, and the study drug used in the study group 14 was 1% methylene blue/5% arginine/5% glycine. The drugs were all aqueous solutions, which were prepared according to the preparation method of Example 1. Each group was injected intratumorally, once every 3 days, for a total of 3 times, with an injection volume of ≤150μl. On the fifth day after the administration was ended, the animals were euthanized, the tumor weight was measured after anatomy, and the tumor inhibition rate was calculated from the negative control group. The tumor inhibition rate of the positive control group was 63%, and the results of the study groups are shown in Table 19.

Table 19

| Group | 1 | 2 | 3 | 4 | 5 | 6 | 7 | 8 | 9 | 10 | 11 | 12 | 13 | 14 |
|---|---|---|---|---|---|---|---|---|---|---|---|---|---|---|
| Y(%) | 96 | 81 | 94 | 53 | 59 | 48 | 24 | 58 | 95 | 86 | 88 | 89 | 90 | 82 |

Based on these results and more similar studies, a super-effective and synergistic technical solution of the amino acid based nutrient/methylene blue dye composition of the present invention is: the amino acid based nutrient is preferably selected from the following amino acids and one or more of amino acid derivatives containing the following amino acids: arginine, glycine, cysteine, threonine, proline, lysine, leucine, alanine, serine, glutamic acid, more preferably selected from one or more of amino acid compounds containing the following amino acid units: arginine, glycine, cysteine hydrochloride, lysine, alanine, serine, and glutamic acid.

Example 7: Anti-tumor application of the composition

1. Series test 1

In this series of experiments, successfully modeled nude mice bearing human cancer cells were randomly divided into 1 negative control group and 5 study groups (groups A, B, C, D and E). The corresponding negative control was normal saline, and the 5 study drugs were: 20% glycine/10% acetic acid, 20% lysine/1% methylene blue, 10% glutathione/30% glucose, 20% arginine/30% glucose/5% acetic acid, 20% glycine/5% DHA/10% acetic acid. The drugs were all aqueous solutions, which were prepared according to the preparation method of Example 1. Each group was injected intratumorally, once every 3 days, for a total of 3 times, each time with $150\mu$l/mouse. On the next day after the administration was ended, the animals were euthanized, the tumor weight was measured after anatomy, and the tumor inhibition rate was calculated from the respective negative control group.

1) Application in the treatment of breast tumor

In this research experiment, successfully modeled nude mice bearing human breast cancer cells (MDA-MB231) (with an average tumor volume of $153mm^3$) were randomly divided into a negative control group and 5 study groups (groups A, B, C, D and E). The tumor inhibition rates of groups A, B, C, D and E were 85%, 91%, 81%, 88%, and 83%, respectively, which all met the generally considered effective anti-tumor standard (tumor inhibition rate $\geq 40\%$).

2) Application in the treatment of lung tumor not according to the claimed invention,

In this research experiment, successfully modeled nude mice bearing human lung cancer cells (A549) (with an average tumor volume of $183mm^3$) were randomly divided into a negative control group and 5 study groups (groups A, B, C, D and E). The tumor inhibition rates of groups A, B, C, D and E were 81%, 83%, 76%, 86%, and 82%, respectively, which all met the generally considered effective anti-tumor standard (tumor inhibition rate $\geq 40\%$).

3) Application in the treatment of thyroid tumor not according to the claimed invention,

In this research experiment, successfully modeled nude mice bearing human thyroid cancer cells (SW579) (with an average tumor volume of $174mm^3$) were randomly divided into a negative control group and 5 study groups (groups A, B, C, D and E). The tumor inhibition rates of groups A, B, C, D and E were 79%, 81%, 76%, 85%, and 83%, respectively, which all met the generally considered effective anti-tumor standard (tumor inhibition rate $\geq 40\%$).

4) Application in the treatment of prostate tumor not according to the claimed invention,

In this research experiment, successfully modeled nude mice bearing human prostate cancer cells (LNCaP/AR) (with an average tumor volume of $168mm^3$) were randomly divided into a negative control group and 5 study groups (groups A, B, C, D and E). The tumor inhibition rates of groups A, B, C, D and E were 75%, 82%, 79%, 81%, and 78%, respectively, which all met the generally considered effective anti-tumor standard (tumor inhibition rate $\geq 40\%$).

5) Application in the treatment of liver tumor not according to the claimed invention,

In this research experiment, successfully modeled nude mice bearing human liver cancer cells (HepG2) (with an average tumor volume of $183mm^3$) were randomly divided into a negative control group and 5 study groups (groups A, B, C, D and E). The tumor inhibition rates of groups A, B, C, D and E were 73%, 86%, 75%, 81%, and 83%, respectively, which all met the generally considered effective anti-tumor standard (tumor inhibition rate $\geq 40\%$).

6) Application in the treatment of head and neck tumors not according to the claimed invention,

In this research experiment, successfully modeled nude mice bearing human head and neck cancer cells (F$\mu$da)

(with an average tumor volume of 169mm$^3$) were randomly divided into a negative control group and 5 study groups (groups A, B, C, D and E). The tumor inhibition rates of groups A, B, C, D and E were 86%, 81%, 79%, 91%, and 87%, respectively, which all met the generally considered effective anti-tumor standard (tumor inhibition rate ≥40%).

7) Application in the treatment of nasopharynx tumor not according to the claimed invention,

In this research experiment, successfully modeled nude mice bearing human nasopharynx cancer cells (CNE1) (with an average tumor volume of 196mm$^3$) were randomly divided into a negative control group and 5 study groups (groups A, B, C, D and E). The tumor inhibition rates of groups A, B, C, D and E were 91%, 81%, 79%, 86%, and 82%, respectively, which all met the generally considered effective anti-tumor standard (tumor inhibition rate ≥40%).

8) Application in the treatment of stomach tumor not according to the claimed invention,

In this research experiment, successfully modeled nude mice bearing human stomach cancer cells (BGC823) (with an average tumor volume of 164mm$^3$) were randomly divided into a negative control group and 5 study groups (groups A, B, C, D and E). The tumor inhibition rates of groups A, B, C, D and E were 71%, 76%, 82%, 87%, and 83%, respectively, which all met the generally considered effective anti-tumor standard (tumor inhibition rate ≥40%).

9) Application in the treatment of ovary tumor not according to the claimed invention,

In this research experiment, successfully modeled nude mice bearing human ovary cancer cells (PA1) (with an average tumor volume of 191mm$^3$) were randomly divided into a negative control group and 5 study groups (groups A, B, C, D and E). The tumor inhibition rates of groups A, B, C, D and E were 86%, 92%, 81%, 91%, and 86%, respectively, which all met the generally considered effective anti-tumor standard (tumor inhibition rate ≥40%).

2. Series test 2 not according to the claimed invention,

In this series of experiments, successfully modeled nude mice bearing human cancer cells were randomly divided into 1 negative control group and 6 study groups (groups A, B, C, D, E and F). The corresponding negative control was normal saline, and the 6 study drugs were: 1% 5-fluorouracil/20% arginine, 1% 5-fluorouracil/10% ALA-GLN, 1% 5-fluorouracil/10% glutathione, 1% 5-fluorouracil/10% glycine/10% acetic acid, 1% 5-fluorouracil/10% glutathione/10% glucose/5% acetic acid, and 1% 5-fluorouracil/20% lysine/1% methylene blue. The drugs were all aqueous solutions, which were prepared according to the preparation method of Example 1. Each group was injected intratumorally, once every 3 days, for a total of 3 times, each time with 100μl/mouse. On the next day after the administration was ended, the animals were euthanized, the tumor weight was measured after anatomy, and the tumor inhibition rate was calculated from the respective negative control group.

1) Application in the treatment of breast tumor not according to the claimed invention,

In this research experiment, successfully modeled nude mice bearing human breast cancer cells (MDA-MB231) (with an average tumor volume of 187mm$^3$) were randomly divided into a negative control group and 6 study groups (groups A, B, C, D, E and F). The tumor inhibition rates of groups A, B, C, D, E and F were 77%, 73%, 71%, 82%, 86%, and 89%, respectively, which all met the generally considered effective anti-tumor standard (tumor inhibition rate ≥40%).

2) Application in the treatment of lung tumor not according to the claimed invention,

In this research experiment, successfully modeled nude mice bearing human lung cancer cells (A549) (with an average tumor volume of 203mm$^3$) were randomly divided into a negative control group and 6 study groups (groups A, B, C, D, E and F). The tumor inhibition rates of groups A, B, C, D, E and F were 71%, 75%, 72%, 81%, 85%, and 87%, respectively, which all met the generally considered effective anti-tumor standard (tumor inhibition rate ≥40%).

3) Application in the treatment of thyroid tumor not according to the claimed invention,

In this research experiment, successfully modeled nude mice bearing human thyroid cancer cells (SW579) (with an average tumor volume of 211mm$^3$) were randomly divided into a negative control group and 6 study groups (groups A, B, C, D, E and F). The tumor inhibition rates of groups A, B, C, D, E and F were 75%, 77%, 73%, 85%, 89%, and 93%, respectively, which all met the generally considered effective anti-tumor standard (tumor inhibition rate ≥40%).

4) Application in the treatment of prostate tumor not according to the claimed invention,

In this research experiment, successfully modeled nude mice bearing human prostate cancer cells (LNCaP/AR) (with an average tumor volume of 194mm$^3$) were randomly divided into a negative control group and 6 study groups (groups A, B, C, D, E and F). The tumor inhibition rates of groups A, B, C, D, E and F were 73%, 71%, 75%, 87%, 85%, and 91%, respectively, which all met the generally considered effective anti-tumor standard (tumor inhibition rate ≥40%).

5) Application in the treatment of liver tumor not according to the claimed invention,

In this research experiment, successfully modeled nude mice bearing human liver cancer cells (HepG2) (with an average tumor volume of 214mm$^3$) were randomly divided into a negative control group and 6 study groups (groups A, B, C, D, E and F). The tumor inhibition rates of groups A, B, C, D, E and F were 73%, 75%, 72%, 81%, 87%, and 93%, respectively, which all met the generally considered effective anti-tumor standard (tumor inhibition rate ≥40%).

6) Application in the treatment of head and neck tumors not according to the claimed invention,

In this research experiment, successfully modeled nude mice bearing human head and neck cancer cells (FμdA) (with an average tumor volume of 172mm$^3$) were randomly divided into a negative control group and 6 study groups (groups A, B, C, D, E and F). The tumor inhibition rates of groups A, B, C, D, E and F were 76%, 72%, 71%, 81%, 89%, and 95%, respectively, which all met the generally considered effective anti-tumor standard (tumor inhibition rate ≥40%).

7) Application in the treatment of nasopharynx tumor not according to the claimed invention,

In this research experiment, successfully modeled nude mice bearing human nasopharynx cancer cells (CNE1) (with an average tumor volume of 203mm$^3$) were randomly divided into a negative control group and 6 study groups (groups A, B, C, D, E and F). The tumor inhibition rates of groups A, B, C, D, E and F were 73%, 71%, 75%, 83%, 87%, and 85%, respectively, which all met the generally considered effective anti-tumor standard (tumor inhibition rate ≥40%).

8) Application in the treatment of stomach tumor not according to the claimed invention,

In this research experiment, successfully modeled nude mice bearing human stomach cancer cells (BGC823) (with an average tumor volume of 218mm$^3$) were randomly divided into a negative control group and 6 study groups (groups A, B, C, E and F). The tumor inhibition rates of groups A, B, C, D, E and F were 71%, 72%, 75%, 83%, 87%, and 91%, respectively, which all met the generally considered effective anti-tumor standard (tumor inhibition rate ≥40%).

9) Application in the treatment of ovary tumor not according to the claimed invention,

In this research experiment, successfully modeled nude mice bearing human ovary cancer cells (PA1) (with an average tumor volume of 186mm$^3$) were randomly divided into a negative control group and 6 study groups (groups A, B, C, D, E and F). The tumor inhibition rates of groups A, B, C, D, E and F were 72%, 71%, 73%, 81%, 87%, and 92%, respectively, which all met the generally considered effective anti-tumor standard (tumor inhibition rate ≥40%).

3. Series test 3

In this series of experiments, successfully modeled nude mice bearing human cancer cells were randomly divided into 1 negative control group and 3 study groups. The corresponding negative control was normal saline, and the three study drugs were: 15% arginine/7% sodium bicarbonate/3% sodium hydroxide, 2% KCl/1% NaOH/20% arginine/20% xylitol, and 20% glycine/3% sodium bicarbonate/10% acetic acid. The drugs were all aqueous solutions, which were prepared according to the preparation method of Example 1. Each group was injected intratumorally, once every 3 days, for a total of 3 times, each time with 150μl/mouse. On the fifth day after the administration was ended, the animals were euthanized, the tumor weight was measured after anatomy, and the tumor inhibition rate was calculated from the respective negative control group.

1) Application in the treatment of breast tumor

In this research experiment, successfully modeled nude mice bearing human breast cancer cells (MDA-MB231) (with an average tumor volume of 303mm$^3$) were randomly divided into a negative control group and 3 study groups (groups D, E and F). The tumor inhibition rates of groups D, E, and F were 92%, 97%, and 91%, respectively, which all met the generally considered effective anti-tumor standard (tumor inhibition rate $\geq$40%).

2) Application in the treatment of lung tumor not according to the claimed invention,

In this research experiment, successfully modeled nude mice bearing human lung cancer cells (A549) (with an average tumor volume of 326mm$^3$) were randomly divided into a negative control group and 3 study groups (groups D, E and F). The tumor inhibition rates of groups D, E, and F were 92%, 95%, and 89%, respectively, which all met the generally considered effective anti-tumor standard (tumor inhibition rate $\geq$40%).

3) Application in the treatment of thyroid tumor not according to the claimed invention,

In this research experiment, successfully modeled nude mice bearing human thyroid cancer cells (SW579) (with an average tumor volume of 341mm$^3$) were randomly divided into a negative control group and 3 study groups (groups D, E and F). The tumor inhibition rates of groups D, E, and F were 92%, 97%, and 88%, respectively, which all met the generally considered effective anti-tumor standard (tumor inhibition rate $\geq$40%).

4) Application in the treatment of prostate tumor not according to the claimed invention,

In this research experiment, successfully modeled nude mice bearing human prostate cancer cells (LNCaP/AR) (with an average tumor volume of 348mm$^3$) were randomly divided into a negative control group and 3 study groups (groups D, E and F). The tumor inhibition rates of groups D, E, and F were 93%, 97%, and 92%, respectively, which all met the generally considered effective anti-tumor standard (tumor inhibition rate $\geq$40%).

5) Application in the treatment of liver tumor not according to the claimed invention,

In this research experiment, successfully modeled nude mice bearing human liver cancer cells (HepG2) (with an average tumor volume of 309mm$^3$) were randomly divided into a negative control group and 3 study groups (groups D, E and F). The tumor inhibition rates of groups D, E, and F were 92%, 95%, and 89%, respectively, which all met the generally considered effective anti-tumor standard (tumor inhibition rate $\geq$40%).

6) Application in the treatment of head and neck tumors not according to the claimed invention,

In this research experiment, successfully modeled nude mice bearing human head and neck cancer cells (F$\mu$da) (with an average tumor volume of 305mm$^3$) were randomly divided into a negative control group and 3 study groups (groups D, E and F). The tumor inhibition rates of groups D, E, and F were 93%, 97%, and 86%, respectively, which all met the generally considered effective anti-tumor standard (tumor inhibition rate $\geq$40%).

7) Application in the treatment of nasopharynx tumor not according to the claimed invention,

In this research experiment, successfully modeled nude mice bearing human nasopharynx cancer cells (CNE1) (with an average tumor volume of 327mm$^3$) were randomly divided into a negative control group and 3 study groups (groups D, E and F). The tumor inhibition rates of groups D, E, and F were 91%, 95%, and 88%, respectively, which all met the generally considered effective anti-tumor standard (tumor inhibition rate $\geq$40%).

8) Application in the treatment of stomach tumor not according to the claimed invention,

In this research experiment, successfully modeled nude mice bearing human stomach cancer cells (BGC823) (with an average tumor volume of 314mm$^3$) were randomly divided into a negative control group and 3 study groups (groups D, E and F). The tumor inhibition rates of groups D, E, and F were 88%, 91%, and 93%, respectively, which all met the generally considered effective anti-tumor standard (tumor inhibition rate $\geq$40%).

9) Application in the treatment of ovary tumor not according to the claimed invention,

In this research experiment, successfully modeled nude mice bearing human ovarian cancer cells (PA1) (with an average tumor volume of 311mm$^3$) were randomly divided into a negative control group and 3 study groups (groups D, E and F). The tumor inhibition rates of groups D, E, and F were 91%, 93%, and 85%, respectively, which all met the generally considered effective anti-tumor standard (tumor inhibition rate $\geq$40%).
Some other compositions of the present invention prepared by the method of Example 3 (such as the synergistic pharmaceutical composition in each example) can also be used in treatment of the above tumors, and they can also obtain similar results.

The interventional treatment for a disease with local lesion symptoms, especially a refractory disease, usually takes a tumor as a model. Among diseases related to local lesions, the mechanism of tumors is extremely complex and the most difficult to treat. The local drug administration technical solution obtained by using a tumor as a model is usually also applicable to other local lesion-related diseases. The following experiments study more applications of the composition of the present invention.

Example 8: Application (1) in anti-non-neoplastic and non-inflammatory swelling not according to the claimed invention,

In one experiment, non-pregnant female rats (with a weight of 150-180g) were randomly divided into a blank control group and a model group. Taking breast hyperplasia as a model, the model group was intramuscularly injected estradiol benzoate (0.5 mg/kg, once/day for 20 consecutive days), followed by intramuscular injection of progesterone (5 mg/kg, once/day for 5 consecutive days). The experimental animals that had been successfully modeled were randomly divided into a negative control group, a positive control group and 4 study groups (groups A, B, C, and D), with 4 animals in each group. Administration was started on the day of grouping. The negative control was normal saline, and the positive control was Ruzengning tablets. The 4 study drugs were: 20% glycine/10% acetic acid, 20% lysine/1% methylene blue, 10% glutathione/ 30% glucose, and 20% arginine/30% glucose/5% acetic acid. The study drugs were all aqueous solutions, which were prepared according to the preparation method of Example 1. Both the negative control group and the study group were injected once every other day in the swollen area, with 100μl/mouse each time, for a total of 5 times. The positive control group was given the positive control by perfusion once a day at 1 g/kg each time for 27 times. The observed, measured and analyzed items in the experiment included relative nipple enlargement rate (T/C%) and lesions in a breast tissue in addition to conventional parameters such as food intake, body weight, and general state.

The equation for calculating the relative nipple diameter is:

$RTD = Dt/D0$, wherein $D0$ is the nipple diameter measured in the blank control group on the 30th day after the first administration, and $Dt$ is the nipple diameter measured in the positive control group or study group on the same day.

The equation for calculating the relative nipple enlargement rate is:

$T/C\ (\%) = TRTD/CRTD \times 100$, wherein TRTD is the RTD of the positive control group or the study group, and CRTD is the RTD of the negative control group.

The evaluation criteria of the relative nipple enlargement rate in the study group were:

$T/C(\%) > 50$, indicating inactive, and $T/C(\%) \leq 50$ as well as $P < 0.05$ by comparing the nipple diameter with the negative control group by analysis of variance, indicating effective.

The histopathological analysis of the breast tissue was performed as follows: the animals were euthanized on the 4th day after the last administration, the second pair of mammary glands of the rats was taken, paraffin sections were taken, and HE staining was performed to observe the lesions of the mammary gland tissues under a light microscope. By observing morphology of the lobules and acini, pathology of the mammary glands of each group of rats was scored. Among them, if the lobules of the mammary glands had no hyperplasia, a quantity of glands was very small, and the acini had no expansion, score 0 was recorded. If the lobules had no obvious hyperplasia, and some acini had mild hyperplasia but no expansion, score 1 was recorded. If most of the breast lobules had hyperplasia, some acini had obvious expansion, score 2 was recorded. If the breast lobules had obvious hyperplasia, the acini were in an extremely expanded state, the glandular epithelial cells were flat, and there were a lot of secretions in the acini and ducts, score 3 was recorded. If the pathological hyperplasia of breast acini, ducts, and lobules was obvious, score 4 was recorded. Pathological analysis and drug efficacy evaluation criteria were: pathology score $\geq 3$, indicating inactive, $1 < $pathology score$ < 3$ and $P < 0.05$ through comparing with the negative control group by analysis of variance, indicating effective.

From the 7th day after the first administration, the nipple diameters of groups A, B, C, and D were significantly reduced compared with the negative control group. On the 30th day after the first administration, the nipple diameters of groups A, B, C, D and of the positive control group (respectively $0.81 \pm 0.18$, $0.79 \pm 0.13$, $0.85 \pm 0.15$, $0.77 \pm 0.12$, $0.87 \pm 0.1$) were much smaller than those of the negative control group ($2.11 \pm 0.23$), and the difference was statistically significant ($P=0.0027$, $P=0.0021$, $P=0.0018$, $p=0.0013$, $p=0.0029$, all indicating $P<0.05$). In this case, the relative nipple enlargement rates of groups A, B, C, D and the positive control group were 38%, 37%, 40%, 37%, and 46%, respectively.

On the 31st day after the administration, the pathology scores of groups A, B, C, and D were $0.81 \pm 0.23$, $0.92 \pm 0.27$, $0.96 \pm 0.25$, $0.87 \pm 0.21$, respectively, all of which were $\leq 1$, and close to the blank control group ($0.37 \pm 0.18$), and was significantly different from the pathology score ($3.73 \pm 0.52$) of the negative control group ($P=0.0002$, $P=0.0002$, $P=0.0002$, $P=0.0001$, $p=0.0001$, all indicating $p<0.05$). The pathology score of the positive control group was greater than 2 ($2.19 \pm 0.41$). Observation results of the safety of the drugs were basically the same.

In another experiment, the method for modeling the experimental animals was the same as in the previous experiment. The experimental animals successfully modeled were randomly divided into a negative control group, a positive control group and 3 study groups (groups 1, 2, and 3), with 6 animals in each group. Administration was started on the day of grouping. The negative control was normal saline, and the positive control was tamoxifen (Rottendorf Phama GmbH). The 3 study drugs were 15% arginine/7% sodium bicarbonate/3% sodium hydroxide, 2% KCl/1% NaOH/20% arginine/20% xylitol, and 20% glycine/3% sodium bicarbonate/10% acetic acid. The study drugs were all aqueous solutions, which were prepared according to the preparation method of Example 1. Both the negative control group and the study group were injected once every 3 days in the swollen area, with 100μl/mouse each time, for a total of 5 times. The positive control group was given tamoxifen twice a day by perfusion, with 0.1 mg/kg each time, for 30 days. The observed, measured and analyzed items in the experiment included nipple enlargement inhibition rate and lesions in a breast tissue in addition to conventional parameters such as food intake, body weight, and general state. The calculation of lesions in the breast tissue and the evaluation criteria of drug efficacy were the same as those in the previous experiment. The calculation formula of nipple enlargement inhibition rate (R%) was as follows:

$$R\% = [(\triangle D01 - \triangle Dn)/\triangle D01]\% = 1 - \triangle Dn/\triangle D01$$

In the formula, $\triangle D01$ is the difference (D01-D0) between the nipple diameter (D01) of the negative control group and the blank control group (D0) on the 33rd day after the first administration, and $\triangle Dn$ is the difference (Dn-D0) between the nipple diameter (Dn) of the study group and the nipple diameter (D0) of the blank control group.

Drug efficacy evaluation criteria for nipple enlargement inhibition rate (R%) were (R%)<40% indicating invalid, and (R%)≥40% indicating effective.

The results of the nipple enlargement inhibition rate are listed in Table 20 below.

Table 20

| Group | Drug | Nipple diameter (mm) | Nipple enlargement inhibition rate |
|---|---|---|---|
| 0 | Blank control group | 0.76±0.17 | (100%) |
| 01 | Normal saline | 1.71±0.32 | (0) |
| 02 | Tamoxifen | 1.16±0.25 | 58% |
| 1 | 15% arginine/7% sodium bicarbonate/ 3% sodium hydroxide | 0.92±0.21 | 83% |
| 2 | 2% KCl/1% NaOH/20% arginine/ 20% xylitol | 0.96±0.24 | 79% |
| 3 | 20% glycine/3% sodium bicarbonate/ 10% acetic acid | 1.04±0.18 | 71% |

In the above table, on the 33rd day after the first administration, the nipple diameters of groups 1, 2, and 3 and the positive control group were all much smaller than those of the negative control group, and the difference was statistically significant (all P<0.05). In this case, the nipple enlargement inhibition rates of groups 02, 1, 2, and 3 were 58%, 83%, 79%, and 71%, respectively, indicating that they were all effective drugs.

On the 33rd day after the first administration, the pathology scores of groups 1, 2, and 3 were 0.71±0.25, 0.73±0.21, 0.82±0.21, respectively, all of which were ≤1, and close to the blank control group (0.32±0.15), and had statistically significant different (all <0.05) pathology score from the pathology score (3.76±0.36) of the negative control group. The pathology score of the positive control group was greater than 1 (1.74±0.28). Observation results of the safety of the drugs were basically the same.

Some other compositions of the present invention prepared by the method of Example 3 (such as the synergistic composition in each example) can also obtain similar results.

Mammary gland hyperplasia (MGH), also known as malformation of the breast, is characterized by breast pain and swelling accompanied by masses. Considering that the composition of the present invention targets a lesion tissue over pathogenic factors (such as pathogens) themselves, mammary gland hyperplasia can be used as a local lesion model for a proliferative lesion that is neither inflammatory nor malignant. Such diseases include: non-malignant tumors, hyperplasia (such as in breast, pancreas, thyroid, parathyroid, and prostate), cysts (such as in breast, thyroid, and parathyroid), abnormal venous clusters (such as hemorrhoids), and other nodules (such as nodules of breast, thyroid, and parathyroid). The hemorrhoids include internal hemorrhoids, external hemorrhoids, and mixed hemorrhoids.

Example 9: Application (2) in anti-non-neoplastic non-inflammatory local lesion diseases not according to the claimed invention,

In one experiment, non-pregnant female rats were randomly divided into a blank control group and a model group. Taking non-inflammatory goiter as a model, the blank control group was fed under normal conditions (drinking water was deionized water with an iodine content of 0.26 mg/L), and the model group was fed with low-iodine feed (drinking water was deionized without iodine) for more than 3 months, the model was successfully set up with a significant decrease in urine iodine and a significant thyroid enlargement. The experimental animals that had been successfully modeled were randomly divided into a negative control group, a positive control group and 4 study groups (groups A, B, C, and D), with 4 animals in each group. Administration was started on the day of grouping. The negative control was normal saline, and the positive control was potassium iodate (KIO$_3$). The 4 study drugs were: 20% glycine/10% acetic acid, 20% lysine/1% methylene blue, 10% glutathione/ 30% glucose, and 20% arginine/30% glucose/5% acetic acid, which were all aqueous solutions, prepared according to the preparation method of Example 1.

Both the negative control group and the study group were injected once every 1 days in the swollen area, with 100μl/mouse each time, for a total of 5 times. The positive control group was given the positive control by perfusion for 27 times, with an administration frequency being once a day, and the dose being 0.4 μg/kg each time. The observed, measured, and analyzed items in the experiment included goiter, 24-hour iodine excretion, and thyroid pathological examination, in addition to regular food intake, weight, and general state.

From the 10th day of the experiment, the study group had significantly improved goiter compared with the negative control group, and consistent iodine excretion with that of the blank control group (difference <25%), with an effect equivalent to that of the positive control group. In a pathological examination, in the negative control group, it was observed that the thyroid gland was enlarged obviously, the follicles were dense, the epithelial cells had tall column shaped proliferation and hypertrophy, the proliferative cell clusters were visible, the blood vessels between the follicles increased significantly, the lumen was dilated and congested, and the interlobular fibrous tissue increased.

On the 25th day after drug administration, the difference in follicular morphology, epithelial cells, and interlobular fibrous tissue, and the like between the study group and the blank control group was less than 15%, which was close to the positive control group. Observation results of the safety of the drugs in each group were basically the same.

In another experiment, the method for modeling the experimental animals was the same as in the previous experiment. The experimental animals successfully modeled were randomly divided into a negative control group, a positive control group and 3 study groups, with 6 animals in each group. Administration was started on the day of grouping. The negative control was normal saline, the positive control was potassium iodate, and the three study drugs were: 15% arginine/7% sodium bicarbonate/3% sodium hydroxide, 2% KCl/1% NaOH/20% arginine/20% xylitol, and 20% glycine/3% sodium bicarbonate/10% acetic acid. The study drugs were all aqueous solutions, which were prepared according to the preparation method of Example 1.

Both the negative control group (group 01) and the study groups (groups 1, 2, and 3) were injected once every 3 days in the swollen area, with 100μl/mouse each time, for a total of 5 times. The positive control group (group 02) was given potassium iodate (KIO$_3$) by perfusion for 27 times with an administration frequency being once a day, and the dose being 0.4 μg/kg each time. The observed, measured, and analyzed items in the experiment included relative mass of the thyroid, and thyroid pathologic examination, in addition to regular food intake, weight, and general state. The observation of thyroid lesions and the evaluation criteria of drug efficacy are the same as the previous experiment. On the 30th day after the first administration, the rats were euthanized and dissected, the thyroid was stripped, the wet weight was weighed, and the relative mass of the thyroid was calculated (relative mass of the thyroid w = mass of the thyroid / mass of the rat).

The calculation formula for the inhibition rate (R%) of thyroid enlargement was as follows:

$$R\% = [(\triangle W01 - \triangle Wn)/\triangle W01]\% = 1 - \triangle Wn/\triangle W01$$

In the formula, △WO1 is the difference (WO1-WO) in relative mass of thyroid between the negative control group (group 01) and the blank control group (group 0), and △Wn is the difference (Wn-WO) in relative mass of thyroid between the study group (n) and the blank control group (0).

Drug efficacy evaluation standard for the inhibition rate of thyroid enlargement (R%) was: (R%)<40% indicating invalid, and (R%)≥40% indicating effective. The inhibitory rate (R%) of thyroid enlargement obtained in the experiment is shown in Table 21 below.

Table 21

| Group | Drug | Relative mass of thyroid (mg/100g) | Inhibition rate of thyroid enlargement |
|---|---|---|---|
| 0 | Blank control group | 6.75±0.38 | (100%) |
| 01 | Normal saline | 31.6±0.45 | (0) |
| 02 | Potassium iodate | 16.2±0.27 | 62% |

(continued)

| Group | Drug | Relative mass of thyroid (mg/100g) | Inhibition rate of thyroid enlargement |
|---|---|---|---|
| 1 | 15% arginine/7% sodium bicarbonate/ 3% sodium hydroxide | 10.4±0.21 | 85% |
| 2 | 2% KCl/1% NaOH/20% arginine/20% xylitol | 11.4±0.23 | 81% |
| 3 | 20% glycine/3% sodium bicarbonate/ 10% acetic acid | 13.9±0.26 | 71% |

In the above table, on the 30th day after the first administration, the relative mass of the thyroid in the groups 1, 2, 3, and 02 was much smaller than that of the negative control group, and the difference between each of these groups and the negative control group was statistically significant (all $P < 0.05$). In this case, the thyroid enlargement inhibition rates of groups 1, 2, 3, and 02 were 85%, 81%, 71%, and 62%, respectively, indicating that they were all effective drugs.

In addition, on the 30th day after drug administration, the difference in follicular morphology, epithelial cells, and interlobular fibrous tissue, and the like between each of the groups 1, 2, and 3 and the blank control group was less than 15%, which was close to the positive control group. Observation results of the safety of the drugs in each group were basically the same.

Some other compositions of the present invention prepared by the method of Example 3 (such as the synergistic composition in each example) can also obtain similar results. Goiter is one of the most common non-neoplastic non-inflammatory local lesion diseases. Considering that the composition of the present invention targets a lesion tissue over pathogenic factors (such as pathogens) themselves, goiter can be used as a local lesion model for a proliferative lesion that is neither inflammatory nor malignant. Such diseases include: non-malignant tumors, hyperplasia (such as in breast, pancreas, thyroid, parathyroid, and prostate), cysts (such as in breast, thyroid, and parathyroid), abnormal venous clusters (such as hemorrhoids), and other nodules (such as nodules of breast, thyroid, and parathyroid). The hemorrhoids include internal hemorrhoids, external hemorrhoids, and mixed hemorrhoids.

Example 10: Application in anti-local inflammations not according to the claimed invention,

In one experiment, adult male rats (with a weight of 150-180g) were randomly divided into a blank control group and a model group. Taking allergic rhinitis as a model, the model used ovalbumin (OVA) sensitizer (containing 0.5mg OVA and 30mg Al(OH)3 per milliliter) as the allergen and induced sensitization by intraperitoneal injection (once a day, for a total of 7 times), and then used nose drops of ovalbumin modeling solution (3% OVA) (once a day, for a total of 7 times) for modeling. After the model was successfully built, the PEMS 3.2 software was used to randomly divide the rats into a positive control group, a negative control group, and study groups (groups A, B, C, and D), with 4 in each group. Administration was started on the day of grouping. The negative control was a normal saline spray, and the positive control was a commercially available mometasone furoate nasal spray (Schering-Plough, Belgium). The 4 study drugs were sprays containing the following ingredients: 20% glycine/10% acetic acid, 20 % lysine/1% methylene blue, 10% glutathione/30% glucose, and 20% arginine/30% glucose/5% acetic acid. The preparation method of the spray is shown in Example 1. The liquid stock solution contained water and the following auxiliary materials: glycerin (2.5%), polysorbate-80 (1.5%), benzalkonium chloride (0.02%), and microcrystalline cellulose-sodium carboxymethyl cellulose (1.5%).

Each group was given via nasal spraying once a day for 7 times. The number of scratches and sneezes within 3 minutes after 30 minutes of administration was taken as an observation index. The efficacy was judged by comparison with the blank control group. If the number of scratches and sneezes in the model group was more than 7 times and more than 1 time of the blank control group, the modeling was successful. If the number of scratches and sneezes in the drug group reached levels similar to those of the positive control group, the drug was effective.

The number of scratches and sneezes on the 15th day after the administration, the differences in numbers between each of the groups A, B, C, D and the positive control group and the blank control group were 13%, 12%, 16%, 9% and 27%, respectively. During the experiment, no obvious mucosal irritation or allergic reaction was observed in each group.

In another experiment, the method for modeling the experimental animals was the same as in the previous experiment. The experimental animals successfully modeled were randomly divided into a negative control group, a positive control group and 3 study groups, with 6 animals in each group. Administration was started on the day of grouping. The negative control was normal saline, the positive control was nasal drops of budesonide (AstraZeneca Pty Ltd), and the three study drugs were sprays containing the following components: 15% arginine/7% sodium bicarbonate/3% sodium hydroxide, 2% KCl/1% NaOH/20% arginine/20% xylitol, and 20% glycine/3% sodium bicarbonate/10% acetic acid. The liquid stock solution contained water and the following auxiliary materials: glycerin (2.5%), polysorbate-80 (1.5%), benzalkonium chloride (0.02%), and microcrystalline cellulose-sodium carboxymethyl cellulose (1.5%). The study drugs were all aqueous solutions, prepared according to the preparation method of Example 1.

Each model group was given via nasal spraying once a day, with 300µl/mouse each time, for a total of 7 times. The observed, measured, and analyzed items in the experiment included nasal symptom scores, in addition to regular food intake, weight, and general state. The nasal symptom score was evaluated on the next day after the administration was ended. The nasal symptoms (nasal scratching, sneezing, running nose) observed within 30 minutes after intranasal administration of ovalbumin nasal drops were scored by the integral superposition method. Nose scratching: score 1 for lightly nose scratching for several times, score 2 for repeated scratching of the nose and face, and score 3 for rubbing the nose and face everywhere. Sneezing: score 1 for 1-3 sneezes, score 2 for 4-10 sneezes, and score 3 for 11 or more sneezes. Running nose: score 1 for running nose to the anterior nasal hole, score 2 for running nose beyond the anterior nasal hole, score 3 for running nose all over the face. The nasal symptom scores obtained in the experiment are shown in Table 22 below.

Table 22

| Group | Drug | Nasal symptom score before administration | Nasal symptom score after administration |
|---|---|---|---|
| 0 | Blank control group | $0.00\pm0.00$ | $0.00\pm0.00$ |
| 01 | Normal saline | $0.57\pm0.53$ | $7.14\pm0.81$ |
| 02 | Budesonide nasal drops | $0.57\pm0.53$ | $1.64\pm0.41$ |
| 1 | 15% arginine/7% sodium bicarbonate/ 3% sodium hydroxide | $0.57\pm0.53$ | $0.69\pm0.51$ |
| 2 | 2% KCl/1% NaOH/20% arginine/ 20% xylitol | $0.57\pm0.53$ | $0.71\pm0.53$ |
| 3 | 20% glycine/3% sodium bicarbonate/ 10% acetic acid | $0.57\pm0.53$ | $0.83\pm0.63$ |

In the above table, on the 8th day after the first administration, with the exception of the blank control group, all the other groups had symptoms such as scratching, sneezing, and running nose after challenge with nasal drops of the ovalbumin solution. Compared with the negative control group, the nasal symptom scores of groups 1, 2, 3, and 02 were all significantly decreased, and the difference between each of these groups and the negative control group was statistically significant (all $P < 0.05$).

Some other compositions of the present invention prepared by the method of Example 3 (such as the synergistic composition in each example) can also obtain similar results. Allergic rhinitis is one of the most common inflammatory local lesion diseases. Considering that the composition of the present invention targets lesion tissues over pathogenic factors (such as pathogens) themselves, allergic rhinitis can be used as a model of an inflammatory local lesion disease. Such diseases include arthritis, mastitis, pancreatitis, thyroiditis, prostatitis, hepatitis, pneumonia, enteritis, stomatitis, pharyngitis, periodontitis, esophagitis, gastritis, gastric ulcer, rhinitis, sinusitis, laryngitis, bronchitis, bronchitis, vaginitis, metritis, salpingitis, oophoritis, and the like.

Example 11: Application in anti-abnormal secretory gland secretion not according to the claimed invention,

In this experiment, adult male rats (with a weight of 150-180g) were randomly divided into a blank control group and a model group. Taking hyperthyroidism as a model, the model was built with levothyroxine (intraperitoneal injection, with a dose of 50µg/100g body weight, for consecutive 10 days). After serological examination and pathological examination confirmed the successful modeling, PEMS 3.2 software was used to randomly divide them into a positive control group, a negative control group, and 4 study groups (groups A, B, C, and D), with 4 animals in each group. Administration was started on the day of grouping. The negative control was a normal saline spray, and the positive control was methimazole. The 4 study drugs were: 20% glycine/10% acetic acid, 20% lysine/1% methylene blue, 10% glutathione/ 30% glucose, and 20% arginine/30% glucose/5% acetic acid. The study drugs were all aqueous solutions, prepared according to the preparation method of Example 1. Both the negative control group and the study group were injected once every 1 days in the swollen area, with 150µl/mouse each time, for a total of 8 times. The positive control group was given the positive control by perfusion for 27 times, with the administration frequency being once a day, and the dose being 2 mg/kg each time.

The observed, measured and analyzed items in the experiment included serological examination and thyroid pathological examination on the day of administration and the 16th day after the administration, in addition to the regular food intake, body weight, and general state. The serological examination employed radioimmunoassay (RIA) to determine concentrations of serum T3, T4 and thyroid-stimulating hormone (TSH). The pathological examination observed thyroid morphology, follicular morphology, epithelial cell morphology, and interlobular fibrous tissue morphology. The examination results are as follows.

In the serological examination on the 25th day after administration, compared with the negative control group (T3 was 1.9±0.1, and T4 was 171.2±9.3), increase in concentrations of serum T3, T4 in group A (T3 was 1.0±0.1, and T4 was 64.3±3.2), group B (T3 was 0.9±0.1, and T4 was 61.7±3.3), group C (T3 was 0.9±0.1, and T4 was 59.7±3.5), group D (T3 was 0.9±0.1, and T4 was 56.3±3.4) slowed down, and the decrease in TSH tended to improve. In terms of the results of serological examination, the difference in serum values between each of groups A, B, C, D and the positive control group (T3 was 1.1±0.1, and T4 was 66.3±3.6) and the blank control group (T3 was 0.8±0.1, and T4 was 39.6±3.1) was much smaller than the difference between the negative control group and the blank control group.

In the pathological examination, the negative control group showed diffuse enlargement in the thyroid gland, high column shaped follicular epithelial hyperplasia and formation of small follicles, many epithelial cell absorbed vacuoles of different sizes around the follicles, and abundant and congested interstitial blood vessels, and hyperplasia of lymphoid tissue. Compared with the negative control group, the diffuse thyroid gland of groups A, B, C, and D decreased, and the follicular epithelial hyperplasia and small follicle formation were significantly reduced. The quantity of epithelial cell absorbed vacuoles around the follicle, the quantity and congestion of interstitial blood vessels, and lymphoid tissue hyperplasia were all decreased. The pathological examination results of groups A, B, C, and D were similar to those of the blank control group and were significantly better than the positive control group. No irreversible local damage and significant weight loss were observed in each group.

In another experiment, the modeling method of experimental animals was the same as that in the previous experiment. Except for the unmodeled blank control group (group 0), the experimental animals that were successfully modeled were randomly divided into a negative control group (group 01), a positive control group (group 02) and 3 study groups, with 6 animals in each group. Administration was started on the day of grouping. The negative control was normal saline, the positive control was methimazole, and the three study drugs were: 20% glycine/3% sodium bicarbonate/10% acetic acid, 2% KCl/1% NaOH/20% arginine/20% xylitol, and 15% arginine/7% sodium bicarbonate/3% hydroxide sodium. The study drugs were all aqueous solutions, prepared according to the preparation method of Example 1.

Both the negative control group and the study groups were injected once every 3 days in the swollen area, with 100μl/mouse each time, for a total of 7 times. The positive control group was given thiamazole once a day, with 10 mg/kg each time, for 21 days. The observed, measured, and analyzed items in the experiment included serological examination (thyroid hormone level) in addition to regular food intake, weight, and general state. The thyroid hormone levels were determined as follows: on the 5th day after the administration was ended, the rats were fasted but still fed with water. The next day, blood was collected from the abdominal aorta and was centrifuged to obtain the serum. T3 and T4 were determined by enzyme-linked immunosorbent assay according to the kit instructions (Beijing North Institute of Biotechnology Co., Ltd.). Table 23 below shows the measurement results.

Table 23

| Group | Study drug | T3 | T4 |
|---|---|---|---|
| 0 | (Blank control) | 0.9±0.1 | 38.5±4.7 |
| 01 | Normal saline | 2.1±0.1 | 131.2±10.1 |
| 02 | Thiamazole | 1.3±0.1 | 75.3±14.2 |
| 1 | 20% glycine/3% sodium bicarbonate/10% acetic acid | 1.0±0.1 | 68.1±12.5 |
| 2 | 2% KCl/1% NaOH/20% arginine/20% xylitol | 0.9±0.1 | 61.2±10.4 |
| 3 | 15% arginine/7% sodium bicarbonate/3% sodium hydroxide | 0.9±0.1 | 57.5±13.1 |

In the above table, on the 26th day after the first administration, compared with the negative control group, the thyroid hormone levels of the groups 1, 2, 3, and 02 decreased significantly, and the difference between each of these groups and the negative control group was statistically significant (both $P<0.05$).

Some other compositions of the present invention prepared by the method of Example 3 (such as the synergistic composition in each example) can also obtain similar results. Hyperthyroidism is one of the most common local lesion diseases with abnormal secretory gland secretion. Considering that the composition of the present invention targets lesion tissues over pathogenic factors (such as pathogens) themselves, hyperthyroidism can be used as a model of this type of local lesion disease. Such diseases include, for example, hyperthyroidism, hypothyroidism, and hypoinsulinism.

Example 12: Anti-dermatological study not according to the claimed invention,

In this study experiment, 20 volunteers with athlete's foot participated in the experiment and were divided into 4 groups, including a positive control group and 3 study groups (groups A, B, and C), with 5 persons in each group. The positive control was Dermonistat (Xian Janssen Pharmaceutical Ltd), and the 3 study drugs were liquid stocks with the following compositions as active ingredients: 20% glycine/3% sodium bicarbonate/10% acetic acid, 15% arginine/7 % sodium bicarbonate/3% sodium hydroxide, and 2% KCl/1% NaOH/20% arginine/20% xylitol. The preparation method of the spray is shown in Example 1. In addition to the above active ingredients, the liquid stock solution contained the following auxiliary materials: glycerin (2.5%), polysorbate-80 (1.5%), benzalkonium chloride (0.02%), and microcrystalline cellulose-sodium carboxymethyl cellulose (1.5%). The liquid stock spray solution was stored in the container of the sprayer before use. In the positive control group, Dermonistat was applied to the affected area once a day for 7 consecutive times. In the negative control group and the 3 study groups, corresponding sprays were sprayed on the affected area once a day for 7 consecutive times. Efficacy criteria were: healing: skin lesions regression> 90%, effective: skin lesions regression> 50%, ineffective: skin lesions regression <50%.

On the seventh day after the administration, the positive control group and the 3 study groups had obvious skin lesions regression. The results on the 10th day after administration are shown in Table 24 below.

Table 24

| Group | Number of cases | Number of heals | Number of effective cases | Number of ineffective cases | Ineffective rate (%) |
|---|---|---|---|---|---|
| Positive control | 5 | 0 | 3 | 2 | 40 |
| Group A | 5 | 1 | 3 | 1 | 20 |
| Group B | 5 | 2 | 3 | 0 | 0 |
| Group C | 5 | 2 | 3 | 0 | 0 |

Similar results were also obtained using sprays of other compositions of the present invention prepared in Example 3 (for example, the synergistic composition in each example).

Athlete's foot is a fungal skin disease, and one of the most common skin diseases in southern China. Considering that the composition of the present invention targets lesion tissues over the pathogens themselves, athlete's foot can be used as a skin disease model. This type of disease include one or more of the following: skin cancer, non-malignant skin tumors, viral skin diseases (such as herpes, warts, rubella, hand, foot and mouth disease), bacterial skin diseases (such as impetigo, boils, leprosy), fungal skin diseases (such as various ringworms), sexually transmitted diseases (such as syphilis, gonorrhea, and condyloma acuminatum), allergic and autoimmune skin diseases (such as contact dermatitis, eczema, urticaria), physical skin diseases (such as solar skin diseases, frostbite, corns, cracked skin of hand and foot, pressure sores), connective tissue diseases (such as lupus erythematosus), pigmented skin diseases (such as freckles, pigmented moles, various spots), skin appendage diseases (such as acne, rosacea, seborrheic dermatitis, alopecia areata, baldness, hyperhidrosis and bromhidrosis).

**Claims**

1.   A topical pharmaceutical composition for use in treating a breast cancer selected from the group consisting of:

   a) a topical pharmaceutical composition comprising arginine, sodium hydroxide, sodium bicarbonate, and a pharmaceutically acceptable liquid carrier, and wherein, the concentration (w/v) of arginine is 15-25%, the concentration (w/v) of sodium hydroxide is 2-5%, and the concentration (w/v) of sodium bicarbonate is 3-10%;
   b) a topical pharmaceutical composition comprising arginine, sodium carbonate, sodium bicarbonate, and a pharmaceutically acceptable liquid carrier, and wherein the concentration (w/v) of arginine is 15-25%, the concentration (w/v) of sodium carbonate is 3-10%, and the concentration (w/v) of sodium bicarbonate is 3-10%.

2.   The pharmaceutical composition for use according to claim 1, wherein the concentration (w/v) of arginine is 15%, the concentration (w/v) of sodium hydroxide is 3%, and the concentration (w/v) of sodium bicarbonate is 7%.

3.   The pharmaceutical composition for use according to claim 1, wherein the concentration (w/v) of arginine is 15 %; the concentration (w/v) of sodium carbonate is 7%; and the concentration (w/v) of sodium bicarbonate is 4%.

4. The pharmaceutical composition for use according to claim 1, wherein the concentration (w/v) of arginine is 15 %; the concentration (w/v) of sodium carbonate is 7%; and the concentration (w/v) of sodium bicarbonate is 5%.

5. A device for treating a local lesion disease, containing the pharmaceutical composition according to any one of claims 1-4.

6. A topical pharmaceutical composition selected from the group consisting of:

a) a topical pharmaceutical composition comprising arginine, sodium hydroxide, sodium bicarbonate, and a pharmaceutically acceptable liquid carrier, wherein the concentration (w/v) of arginine is 15- 25%, the concentration (w/v) of sodium hydroxide is 2-5%, and the concentration (w/v) of sodium bicarbonate is 3-10%;
b) a topical pharmaceutical composition comprising arginine, sodium carbonate, sodium bicarbonate, and a pharmaceutically acceptable liquid carrier, wherein the concentration (w/v) of arginine is 15-25%; the concentration (w/v) of sodium carbonate is 3-10%; and the concentration (w/v) of sodium bicarbonate is 3-10%.

7. The topical pharmaceutical composition according to claim 6, wherein the concentration (w/v) of arginine is 15%, the concentration (w/v) of sodium hydroxide is 3%, and the concentration (w/v) of sodium bicarbonate is 7%.

8. The topical pharmaceutical composition according to claim 6, wherein the concentration (w/v) of arginine is 15 %; the concentration (w/v) of sodium carbonate is 7%; and the concentration (w/v) of sodium bicarbonate is 4%.

9. The topical pharmaceutical composition according to claim 6, wherein the concentration (w/v) of arginine is 15 %; the concentration (w/v) of sodium carbonate is 7%; and the concentration (w/v) of sodium bicarbonate is 5%.

10. The pharmaceutical composition for use according to any one of claims 1-4, or the topical pharmaceutical composition according to any one of claims 6-9, wherein the pharmaceutically acceptable liquid carrier is water.


**Patentansprüche**

1. Topische pharmazeutische Zusammensetzung zur Verwendung bei der Behandlung eines Brustkrebses, ausgewählt aus der Gruppe bestehend aus:

a) einer topischen pharmazeutischen Zusammensetzung, umfassend Arginin, Natriumhydroxid, Natriumhydrogencarbonat und einen pharmazeutisch verträglichen flüssigen Träger, und wobei die Konzentration (w/v) von Arginin 15-25% beträgt, die Konzentration (w/v) von Natriumhydroxid 2-5% beträgt und die Konzentration (w/v) von Natriumhydrogencarbonat 3-10% beträgt;
b) einer topischen pharmazeutischen Zusammensetzung, umfassend Arginin, Natriumcarbonat, Natriumhydrogencarbonat und einen pharmazeutisch verträglichen flüssigen Träger, und wobei die Konzentration (w/v) von Arginin 15-25% beträgt, die Konzentration (w/v) von Natriumcarbonat 3-10% beträgt und die Konzentration (w/v) von Natriumhydrogencarbonat 3-10% beträgt.

2. Pharmazeutische Zusammensetzung zur Verwendung gemäß Anspruch 1, wobei die Konzentration (w/v) von Arginin 15% beträgt, die Konzentration (w/v) von Natriumhydroxid 3% beträgt und die Konzentration (w/v) von Natriumhydrogencarbonat 7% beträgt.

3. Pharmazeutische Zusammensetzung zur Verwendung gemäß Anspruch 1, wobei die Konzentration (w/v) von Arginin 15% beträgt; die Konzentration (w/v) von Natriumcarbonat 7% beträgt; und die Konzentration (w/v) von Natriumhydrogencarbonat 4% beträgt.

4. Pharmazeutische Zusammensetzung zur Verwendung gemäß Anspruch 1, wobei die Konzentration (w/v) von Arginin 15% beträgt; die Konzentration (w/v) von Natriumcarbonat 7% beträgt; und die Konzentration (w/v) von Natriumhydrogencarbonat 5% beträgt.

5. Vorrichtung zur Behandlung einer lokalen Läsionskrankheit, welche die pharmazeutische Zusammensetzung nach einem der Ansprüche 1 bis 4 enthält.

6. Topische pharmazeutische Zusammensetzung, ausgewählt aus der Gruppe bestehend aus:

a) einer topischen pharmazeutischen Zusammensetzung, umfassend Arginin, Natriumhydroxid, Natriumhydrogencarbonat und einen pharmazeutisch verträglichen flüssigen Träger, wobei die Konzentration (w/v) von Arginin 15-25% beträgt, die Konzentration (w/v) von Natriumhydroxid 2-5% beträgt und die Konzentration (w/v) von Natriumhydrogencarbonat 3-10% beträgt;

b) einer topischen pharmazeutischen Zusammensetzung, umfassend Arginin, Natriumcarbonat, Natriumhydrogencarbonat und einen pharmazeutisch verträglichen flüssigen Träger, wobei die Konzentration (w/v) von Arginin 15-25% beträgt; die Konzentration (w/v) von Natriumcarbonat 3-10% beträgt; und die Konzentration (w/v) von Natriumhydrogencarbonat 3-10% beträgt.

7. Topische pharmazeutische Zusammensetzung nach Anspruch 6, wobei die Konzentration (w/v) von Arginin 15% beträgt, die Konzentration (w/v) von Natriumhydroxid 3% beträgt, und die Konzentration (w/v) von Natriumhydrogencarbonat 7% beträgt.

8. Topische pharmazeutische Zusammensetzung nach Anspruch 6, wobei die Konzentration (w/v) von Arginin 15% beträgt; die Konzentration (w/v) von Natriumcarbonat 7% beträgt; und die Konzentration (w/v) von Natriumhydrogencarbonat 4% beträgt.

9. Topische pharmazeutische Zusammensetzung nach Anspruch 6, wobei die Konzentration (w/v) von Arginin 15% beträgt; die Konzentration (w/v) von Natriumcarbonat 7% beträgt; und die Konzentration (w/v) von Natriumhydrogencarbonat 5% beträgt.

10. Pharmazeutische Zusammensetzung zur Verwendung nach einem der Ansprüche 1 bis 4, oder topische pharmazeutische Zusammensetzung nach einem der Ansprüche 6 bis 9, wobei der pharmazeutisch verträgliche flüssige Träger Wasser ist.

## Revendications

1. Composition pharmaceutique topique pour utilisation dans le traitement d'un cancer du sein, sélectionnée dans le groupe consistant en :

   a) une composition pharmaceutique topique comprenant de l'arginine, de l'hydroxyde de sodium, du bicarbonate de sodium et un support liquide pharmaceutiquement acceptable, et dans laquelle la concentration (p/v) en arginine est de 15-25 %, la concentration (p/v) en hydroxyde de sodium est de 2-5 % et la concentration (p/v) en bicarbonate de sodium est de 3-10 % ;

   b) une composition pharmaceutique topique comprenant de l'arginine, du carbonate de sodium, du bicarbonate de sodium et un support liquide pharmaceutiquement acceptable, et dans laquelle la concentration (p/v) en arginine est de 15-25 %, la concentration (p/v) en carbonate de sodium est de 3-10 % et la concentration (p/v) en bicarbonate de sodium est de 3-10 %.

2. Composition pharmaceutique pour utilisation selon la revendication 1, dans laquelle la concentration (p/v) en arginine est de 15 %, la concentration (p/v) en hydroxyde de sodium est de 3 % et la concentration (p/v) en bicarbonate de sodium est de 7 %.

3. Composition pharmaceutique pour utilisation selon la revendication 1, dans laquelle la concentration (p/v) en arginine est de 15 % ; la concentration (p/v) en carbonate de sodium est de 7 % ; et la concentration (p/v) en bicarbonate de sodium est de 4 %.

4. Composition pharmaceutique pour utilisation selon la revendication 1, dans laquelle la concentration (p/v) en arginine est de 15 % ; la concentration (p/v) en carbonate de sodium est de 7 % ; et la concentration (p/v) en bicarbonate de sodium est de 5 %.

5. Dispositif de traitement d'une maladie à lésions locales, contenant la composition pharmaceutique selon l'une quelconque des revendications 1 à 4.

6. Composition pharmaceutique topique sélectionnée dans le groupe consistant en :

   a) une composition pharmaceutique topique comprenant de l'arginine, de l'hydroxyde de sodium, du bicarbonate de sodium et un support liquide pharmaceutiquement acceptable, dans laquelle la concentration (p/v) en arginine est de 15-25 %, la concentration (p/v) en hydroxyde de sodium est de 2-5 % et la concentration (p/v) en bicarbonate de sodium est de 3-10 % ;

b) une composition pharmaceutique topique comprenant de l'arginine, du carbonate de sodium, du bicarbonate de sodium et un support liquide pharmaceutiquement acceptable, dans laquelle la concentration (p/v) en arginine est de 15-25 % ; la concentration (p/v) en carbonate de sodium est de 3-10 % ; et la concentration (p/v) en bicarbonate de sodium est de 3-10 %.

7. Composition pharmaceutique topique selon la revendication 6, dans laquelle la concentration (p/v) en arginine est de 15 %, la concentration (p/v) en hydroxyde de sodium est de 3 % et la concentration (p/v) en bicarbonate de sodium est de 7 %.

8. Composition pharmaceutique topique selon la revendication 6, dans laquelle la concentration (p/v) en arginine est de 15 % ; la concentration (p/v) en carbonate de sodium est de 7 % ; et la concentration (p/v) en bicarbonate de sodium est de 4 %.

9. Composition pharmaceutique topique selon la revendication 6, dans laquelle la concentration (p/v) en arginine est de 15 % ; la concentration (p/v) en carbonate de sodium est de 7 % ; et la concentration (p/v) en bicarbonate de sodium est de 5 %.

10. Composition pharmaceutique pour utilisation selon l'une quelconque des revendications 1 à 4, ou composition pharmaceutique topique selon l'une quelconque des revendications 6 à 9, dans laquelle le support liquide pharmaceutiquement acceptable est l'eau.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 5690967 A

**Non-patent literature cited in the description**

- *CHEMICAL ABSTRACTS, 74-79-3*
- *CHEMICAL ABSTRACTS, 10417-94-4*
- *CHEMICAL ABSTRACTS, 56-40-6*
- *CHEMICAL ABSTRACTS, 6217-54-5*
- *CHEMICAL ABSTRACTS, 52-89-1*
- *CHEMICAL ABSTRACTS, 8002-43-5*
- *CHEMICAL ABSTRACTS, 1119-34-2*
- *CHEMICAL ABSTRACTS, 7647-01-0*
- *CHEMICAL ABSTRACTS, 657-27-2*
- *CHEMICAL ABSTRACTS, 7664-93-9*
- *CHEMICAL ABSTRACTS, 72-19-5*
- *CHEMICAL ABSTRACTS, 110-16-7*
- *CHEMICAL ABSTRACTS, 147-85-3*
- *CHEMICAL ABSTRACTS, 64-19-7*
- *CHEMICAL ABSTRACTS, 138-15-8*
- *CHEMICAL ABSTRACTS, 79-09-4*
- *CHEMICAL ABSTRACTS, 72-18-4*
- *CHEMICAL ABSTRACTS, 141-82-2*
- *CHEMICAL ABSTRACTS, 56-87-1*
- *CHEMICAL ABSTRACTS, 107-92-6*
- *CHEMICAL ABSTRACTS, 56-41-7*
- *CHEMICAL ABSTRACTS, 50-21-5*
- *CHEMICAL ABSTRACTS, 56-45-1*
- *CHEMICAL ABSTRACTS, 63-91-2*
- *CHEMICAL ABSTRACTS, 40719-58-2*
- *CHEMICAL ABSTRACTS, 7220-79-3*
- *CHEMICAL ABSTRACTS, 691364-49-5*
- *CHEMICAL ABSTRACTS, 3536-49-0*
- *CHEMICAL ABSTRACTS, 39537-23-0*
- *CHEMICAL ABSTRACTS, 68238-36-8*
- *CHEMICAL ABSTRACTS, 70-18-8*
- *CHEMICAL ABSTRACTS, 632-69-9*
- *CHEMICAL ABSTRACTS, 171263-26-6*
- *CHEMICAL ABSTRACTS, 6119-47-7*
- *CHEMICAL ABSTRACTS, 50-99-7*
- *CHEMICAL ABSTRACTS, 60-93-5*
- *CHEMICAL ABSTRACTS, 71-00-1*
- *CHEMICAL ABSTRACTS, 50-78-2*
- *CHEMICAL ABSTRACTS, 7660-25-5*
- *CHEMICAL ABSTRACTS, 69-72-7*
- *CHEMICAL ABSTRACTS, 4618-18-2*
- *CHEMICAL ABSTRACTS, 62952-06-1*
- *CHEMICAL ABSTRACTS, 6138-23-4*
- *CHEMICAL ABSTRACTS, 50-18-0*
- *CHEMICAL ABSTRACTS, 87-99-0*
- *CHEMICAL ABSTRACTS, 147-94-4*
- *CHEMICAL ABSTRACTS, 69-79-4*
- *CHEMICAL ABSTRACTS, 15663-27-1*
- *CHEMICAL ABSTRACTS, 50-69-1*
- *CHEMICAL ABSTRACTS, 114915-14 -9*
- *CHEMICAL ABSTRACTS, 148411-57-8*
- *CHEMICAL ABSTRACTS, 25316-40-9*
- *CHEMICAL ABSTRACTS, 9004-54-0*
- *CHEMICAL ABSTRACTS, 51-21-8*
- **BURGI Y.** *Pharmacology; Drug actions and reactions. Cancer Res.*, 1978, vol. 38 (2), 284-285
- **JIN ZHENGJUN**. Addition in combined medication. *Chinese Journal of Pharmacology*, 1980, vol. 1 (2), 70-76